# EUROPÄISCHE PATENTANMELDUNG

(11) **EP 1 176 200 A2**
(43) Veröffentlichungstag der Anmeldung: **30.01.2002**
(21) Anmeldenummer: 01112963.2
(22) Anmeldetag: 07.06.2001
(51) Int. Cl.: C12N 15/12, A61K 38/17, A61K 48/00, A61K 39/395, G01N 33/50, C12Q 1/68, A61P 17/02

(54) **Verwendung von Polypeptiden oder diese kodierende Nukleinsäuren zur Diagnose oder Behandlung von Hauterkrankung oder Wundheilung sowie ihre Verwendung zur Indentifizierung von pharmakologisch aktiven Substanzen**

(30) Priorität: 20.06.2000 DE 10030149; 01.08.2000 US 222081 P
(71) Anmelder: Switch Biotech Aktiengesellschaft, 82152 Martinsried (DE)
(72) Erfinder: Halle, Jörn-Peter, 82377 Penzberg (DE); Goppelt, Andreas, 80636 München (DE); Regenbogen, Johannes, 82152 Martinsried (DE); Werner, Sabine, 8032 Zürich (CH); Wolf, Eckhard, 85764 Oberschleissheim (DE)
(74) Vertreter: Bösl, Raphael, Dr. rer. nat., Dipl.-Chem.

(57) **Zusammenfassung**

Eine Erfindung die sich auf die Verwendung von Polypeptiden oder diese kodierende Nukleinsäuren zur Diagnose und/oder Prävention und/oder Behandlung von Erkrankungen von Hautzellen und/oder bei der Wundheilung und/oder deren krankhaften Störungen sowie ihre Verwendung zur Identifizierung von pharmakologisch aktiven Substanzen bezieht.

SEQUENZ PROTOKOLL

<110> Switch Biotech Aktiengesellschaft

<120> Verwendung von Polypeptiden oder diese kodierende Nukleinsäuren zur Diagnose oder Behandlung von Hauterkrankungen oder Wundheilung sowie ihre Verwendung zur Identifizierung von pharmakologisch aktiven Substanzen

<150> DE 100 30 149.5-41
<151> 2000-06-20

<150> US 60/222,081
<151> 2000-08-01

<160> 84

<170> Word 6.0, PC-DOS/MS-DOS

<210> 1
<211> 391
<212> PRT
<213> Mus musculus

<220>
<223> TUMOR SUSCEPTIBILITY PROTEIN TSG101

<400> 1

Met Ala Val Ser Glu Ser Gln Leu Lys 9
Lys Met Met Ser Lys Tyr Lys Tyr Arg 18
Asp Leu Thr Val Arg Gln Thr Val Asn 27
Val Ile Ala Met Tyr Lys Asp Leu Lys 36
Pro Val Leu Asp Ser Tyr Val Phe Asn 45
Asp Gly Ser Ser Arg Glu Leu Val Asn 54
Leu Thr Gly Thr Ile Pro Val Arg Tyr 63
Arg Gly Asn Ile Tyr Asn Ile Pro Ile 72
Cys Leu Trp Leu Leu Asp Thr Tyr Pro 81
Tyr Asn Pro Pro Ile Cys Phe Val Lys 90
Pro Thr Ser Ser Met Thr Ile Lys Thr 99
Gly Lys His Val Asp Ala Asn Gly Lys 108
Ile Tyr Leu Pro Tyr Leu His Asp Trp 117
Lys His Pro Arg Ser Glu Leu Leu Glu 126
Leu Ile Gln Ile Met Ile Val Ile Phe 135
Gly Glu Glu Pro Pro Val Phe Ser Arg 144
Pro Thr Val Ser Ala Ser Tyr Pro Pro 153
Tyr Thr Ala Thr Gly Pro Pro Asn Thr 162
Ser Tyr Met Pro Gly Met Pro Ser Gly 171
Ile Ser Ala Tyr Pro Ser Gly Tyr Pro 180

Pro Asn Pro Ser Gly Tyr Pro Gly Cys 189
Pro Tyr Pro Pro Ala Gly Pro Tyr Pro 198
Ala Thr Thr Ser Ser Gln Tyr Pro Ser 207
Gln Pro Pro Val Thr Thr Val Gly Pro 216
Ser Arg Asp Gly Thr Ile Ser Glu Asp 225
Thr Ile Arg Ala Ser Leu Ile Ser Ala 234
Val Ser Asp Lys Leu Arg Trp Arg Met 243
Lys Glu Glu Met Asp Gly Ala Gln Ala 252
Glu Leu Asn Ala Leu Lys Arg Thr Glu 261
Glu Asp Leu Lys Lys Gly His Gln Lys 270
Leu Glu Glu Met Val Thr Arg Leu Asp 279
Gln Glu Val Ala Glu Val Asp Lys Asn 288
Ile Glu Leu Leu Lys Lys Lys Asp Glu 297
Glu Leu Ser Ser Ala Leu Glu Lys Met 306
Glu Asn Gln Ser Glu Asn Asn Asp Ile 315
Asp Glu Val Ile Ile Pro Thr Ala Pro 324
Leu Tyr Lys Gln Ile Leu Asn Leu Tyr 333
Ala Glu Glu Asn Ala Ile Glu Asp Thr 342
Ile Phe Tyr Leu Gly Glu Ala Leu Arg 351
Arg Gly Val Ile Asp Leu Asp Val Phe 360
Leu Lys His Val Arg Leu Leu Ser Arg 369
Lys Gln Phe Gln Leu Arg Ala Leu Met 378
Gln Lys Ala Arg Lys Thr Ala Gly Leu 387
Ser Asp Leu Tyr 391

<210> 2
<211> 390
<212> PRT
<213> Homo sapiens

<220>
<223> TUMOR SUSCEPTIBILITY PROTEIN

<400> 2

Met Ala Val Ser Glu Ser Gln Leu Lys 9
Lys Met Val Ser Lys Tyr Lys Tyr Arg 18
Asp Leu Thr Val Arg Glu Thr Val Asn 27
Val Ile Thr Leu Tyr Lys Asp Leu Lys 36
Pro Val Leu Asp Ser Tyr Val Phe Asn 45
Asp Gly Ser Ser Arg Glu Leu Met Asn 54
Leu Thr Gly Thr Ile Pro Val Pro Tyr 63
Arg Gly Asn Thr Tyr Asn Ile Pro Ile 72
Cys Leu Trp Leu Leu Asp Thr Tyr Pro 81
Tyr Asn Pro Pro Ile Cys Phe Val Lys 90
Pro Thr Ser Ser Met Thr Ile Lys Thr 99
Gly Lys His Val Asp Ala Asn Gly Lys 108
Ile Tyr Leu Pro Tyr Leu His Glu Trp 117
Lys His Pro Gln Ser Asp Leu Leu Gly 126
Leu Ile Gln Val Met Ile Val Val Phe 135
Gly Asp Glu Pro Pro Val Phe Ser Arg 144

Pro Ile Ser Ala Ser Tyr Pro Pro Tyr 153
Gln Ala Thr Gly Pro Pro Asn Thr Ser 162
Tyr Met Pro Gly Met Pro Gly Gly Ile 171
Ser Pro Tyr Pro Ser Gly Tyr Pro Pro 180
Asn Pro Ser Gly Tyr Pro Gly Cys Pro 189
Tyr Pro Pro Gly Gly Pro Tyr Pro Ala 198
Thr Thr Ser Ser Gln Tyr Pro Ser Gln 207
Pro Pro Val Thr Thr Val Gly Pro Ser 216
Arg Asp Gly Thr Ile Ser Glu Asp Thr 225
Ile Arg Ala Ser Leu Ile Ser Ala Val 234
Ser Asp Lys Leu Arg Trp Arg Met Lys 243
Glu Glu Met Asp Arg Ala Gln Ala Glu 252
Leu Asn Ala Leu Lys Arg Thr Glu Glu 261
Asp Leu Lys Lys Gly His Gln Lys Leu 270
Glu Glu Met Val Thr Arg Leu Asp Gln 279
Glu Val Ala Glu Val Asp Lys Asn Ile 288
Glu Leu Leu Lys Lys Lys Asp Glu Glu 297
Leu Ser Ser Ala Leu Glu Lys Met Glu 306
Asn Gln Ser Glu Asn Asn Asp Ile Asp 315
Glu Val Ile Ile Pro Thr Ala Pro Leu 324
Tyr Lys Gln Ile Leu Asn Leu Tyr Ala 333
Glu Glu Asn Ala Ile Glu Asp Thr Ile 342
Phe Tyr Leu Gly Glu Ala Leu Arg Arg 351
Gly Val Ile Asp Leu Asp Val Phe Leu 360
Lys His Val Arg Leu Leu Ser Arg Lys 369
Gln Phe Gln Leu Arg Ala Leu Met Gln 378
Lys Ala Arg Lys Thr Ala Gly Leu Ser 387
Asp Leu Tyr 390

<210> 3
<211> 375
<212> PRT
<213> Mus musculus

<220>
<223> MASPIN PRECURSOR

<400> 3

Met Asp Ala Leu Arg Leu Ala Asn Ser 9
Ala Phe Ala Val Asp Leu Phe Lys Gln 18
Leu Cys Glu Arg Asp Pro Ala Gly Asn 27
Ile Leu Phe Ser Pro Ile Cys Leu Ser 36
Thr Ser Leu Ser Leu Ala Gln Val Gly 45
Thr Lys Gly Asp Thr Ala Asn Glu Ile 54
Gly Gln Val Leu His Phe Glu Asn Val 63
Lys Asp Val Pro Phe Gly Phe Gln Thr 72
Val Thr Ser Asp Val Asn Lys Leu Ser 81
Ser Phe Tyr Ser Leu Lys Leu Val Lys 90
Arg Leu Tyr Ile Asp Lys Ser Leu Asn 99

Pro Ser Thr Glu Phe Ile Ser Ser Thr 108
Lys Arg Pro Tyr Ala Lys Glu Leu Glu 117
Thr Val Asp Phe Lys Asp Lys Leu Glu 126
Glu Thr Lys Gly Gln Ile Asn Ser Ser 135
Ile Lys Glu Leu Thr Asp Gly His Phe 144
Glu Asp Ile Leu Ser Glu Asn Ser Ile 153
Ser Asp Gln Thr Lys Ile Leu Val Val 162
Asn Ala Ala Tyr Phe Val Gly Lys Trp 171
Met Lys Lys Phe Pro Glu Ser Glu Thr 180
Lys Glu Cys Pro Phe Arg Ile Ser Lys 189
Thr Asp Thr Lys Pro Val Gln Met Met 198
Asn Leu Glu Ala Thr Phe Cys Leu Gly 207
Asn Ile Asp Asp Ile Ser Cys Lys Ile 216
Ile Glu Leu Pro Phe Gln Asn Lys His 225
Leu Ser Met Leu Ile Val Leu Pro Lys 234
Asp Val Glu Asp Glu Ser Thr Gly Leu 243
Glu Lys Ile Glu Gln Gln Leu Asn Pro 252
Glu Thr Leu Leu Gln Trp Thr Asn Pro 261
Ser Thr Met Ala Asn Ala Lys Val Lys 270
Leu Ser Leu Pro Lys Phe Lys Val Glu 279
Lys Met Ile Asp Pro Lys Ala Ser Leu 288
Glu Ser Leu Gly Leu Lys Ser Leu Phe 297
Asn Glu Ser Thr Ser Asp Phe Ser Gly 306
Met Ser Glu Thr Lys Gly Val Ser Leu 315
Ser Asn Val Ile His Arg Val Cys Leu 324
Glu Ile Thr Glu Asp Gly Gly Glu Ser 333
Ile Glu Val Pro Gly Ser Arg Ile Leu 342
Gln His Lys Asp Glu Phe Asn Ala Asp 351
His Pro Phe Ile Tyr Ile Ile Arg His 360
Asn Lys Thr Arg Asn Ile Ile Phe Phe 369
Gly Lys Phe Cys Ser Pro 375

<210> 4
<211> 375
<212> PRT
<213> Homo sapiens

<220>
<223> MASPIN PRECURSOR

<400> 4

Met Asp Ala Leu Gln Leu Ala Asn Ser 9
Ala Phe Ala Val Asp Leu Phe Lys Gln 18
Leu Cys Glu Lys Glu Pro Leu Gly Asn 27
Val Leu Phe Ser Pro Ile Cys Leu Ser 36
Thr Ser Leu Ser Leu Ala Gln Val Gly 45
Ala Lys Gly Asp Thr Ala Asn Glu Ile 54
Gly Gln Val Leu His Phe Glu Asn Val 63
Lys Asp Ile Pro Phe Gly Phe Gln Thr 72

Val Thr Ser Asp Val Asn Lys Leu Ser 81
Ser Phe Tyr Ser Leu Lys Leu Ile Lys 90
Arg Leu Tyr Val Asp Lys Ser Leu Asn 99
Leu Ser Thr Glu Phe Ile Ser Ser Thr 108
Lys Arg Pro Tyr Ala Lys Glu Leu Glu 117
Thr Val Asp Phe Lys Asp Lys Leu Glu 126
Glu Thr Lys Gly Gln Ile Asn Asn Ser 135
Ile Lys Asp Leu Thr Asp Gly His Phe 144
Glu Asn Ile Leu Ala Asp Asn Ser Val 153
Asn Asp Gln Thr Lys Ile Leu Val Val 162
Asn Ala Ala Tyr Phe Val Gly Lys Trp 171
Met Lys Lys Phe Pro Glu Ser Glu Thr 180
Lys Glu Cys Pro Phe Arg Leu Asn Lys 189
Thr Asp Thr Lys Pro Val Gln Met Met 198
Asn Met Glu Ala Thr Phe Cys Met Gly 207
Asn Ile Asp Ser Ile Asn Cys Lys Ile 216
Ile Glu Leu Pro Phe Gln Asn Lys His 225
Leu Ser Met Phe Ile Leu Leu Pro Lys 234
Asp Val Glu Asp Glu Ser Thr Gly Leu 243
Glu Lys Ile Glu Lys Gln Leu Asn Ser 252
Glu Ser Leu Ser Gln Trp Thr Asn Pro 261
Ser Thr Met Ala Asn Ala Lys Val Lys 270
Leu Ser Ile Pro Lys Phe Lys Val Glu 279
Lys Met Ile Asp Pro Lys Ala Cys Leu 288
Glu Asn Leu Gly Leu Lys His Ile Phe 297
Ser Glu Asp Thr Ser Asp Phe Ser Gly 306
Met Ser Glu Thr Lys Gly Val Ala Leu 315
Ser Asn Val Ile His Lys Val Cys Leu 324
Glu Ile Thr Glu Asp Gly Gly Asp Ser 333
Ile Glu Val Pro Gly Ala Arg Ile Leu 342
Gln His Lys Asp Glu Leu Asn Ala Asp 351
His Pro Phe Ile Tyr Ile Ile Arg His 360
Asn Lys Thr Arg Asn Ile Ile Phe Phe 369
Gly Lys Phe Cys Ser Pro 375

<210> 5
<211> 833
<212> PRT
<213> Mus musculus

<220>
<223> TRANSCRIPTION TERMINATION FACTOR 1

<400> 5

Met Lys Gly Gly Thr Ser Lys Phe Lys 9
Thr His Thr Glu Thr Leu Tyr Lys Lys 18
Lys Lys Trp Ser Ser Val Ser Glu Lys 27
Arg Pro Gln Lys Cys Pro Ser Gln Cys 36
Leu Glu Ser Lys Gln Pro Gln Val Ser 45
Val Leu Gly Lys Arg Arg Arg Ala Ser 54

Gln Thr Pro Ala Gln Glu Thr Leu Glu 63
Ser Glu Trp Pro Gln Lys Ala Lys Lys 72
Lys Lys Arg Arg Gly Glu Pro Gln Thr 81
Pro Thr Gln Glu Ser Leu Glu Ser Glu 90
Gln Pro Pro Val Ser Leu Leu Gly Lys 99
Arg Arg Arg Glu Ser Gln Thr Pro Ala 108
Gln Glu Asn Ser Glu Ser Glu Gln Pro 117
Arg Lys Ala Lys Arg Arg Arg Lys Lys 126
Arg Lys Gly Ser Gln Gln Pro Thr Ser 135
Ser Leu Leu Lys Thr Pro Glu Thr Phe 144
Leu Lys Ala Lys Lys Thr Thr Ser Ala 153
His Lys Lys Lys Lys Asn Ser Val Leu 162
Glu Val Asp Met Glu Thr Gly Ile Ile 171
Leu Val Asp Lys Glu Asn Met Glu Asn 180
Leu Leu Glu Thr Ser Arg Lys Asp Val 189
Asp Ile Val Tyr Val Asp Met Ser Lys 198
Gly Gln Arg Ser Ala Lys Val Arg Glu 207
Thr Gly Glu Leu Pro Ala Ala Lys Pro 216
Gln Glu His Gly Cys Arg Glu Leu Leu 225
Gly Asp Val Arg Ser Arg Lys Lys Gln 234
Lys His Leu Gln Lys Val Ala Pro Trp 243
Asp Val Val Gln Gly Ser Gln Pro Glu 252
Ser Ile Ser Leu Pro Pro Ser Glu Pro 261
Leu Ser Ser Glu Asp Leu Glu Gly Lys 270
Ser Thr Glu Ala Ala Val Phe Cys Lys 279
Arg Ser Leu Lys Lys Asn Val Phe Arg 288
Ser Gln Glu Leu Glu Pro Ile Pro Asp 297
Ser Leu Asp Asp Ser Glu Thr Ile Ser 306
Glu Arg Leu Asp Ser Thr His His Gly 315
Gly Ala Val Gly Ala Gly Glu Cys Glu 324
Ser Thr Lys Glu Ser His Ser Ile Lys 333
Lys Lys Ser Lys Lys Lys Lys His Lys 342
Ser Val Ala Leu Ala Thr Ser Ser Asp 351
Ser Ala Ser Val Thr Asp Ser Lys Ala 360
Lys Asn Ala Leu Val Asp Ser Ser Glu 369
Gly Ser Gly Ala Val Arg Glu Glu Asp 378
Val Asp His Arg Pro Ala Glu Ala Glu 387
Ala Gln Ala Cys Ser Thr Glu Lys His 396
Arg Glu Ala Met Gln Arg Leu Glu Pro 405
Thr His Glu Glu Glu Ser Asn Ser Glu 414
Ser Ala Ser Asn Ser Ala Ala Arg His 423
Ile Ser Glu Asp Arg Arg Glu Ser Asp 432
Asp Ser Asp Val Asp Leu Gly Ser Ala 441
Val Arg Gln Leu Arg Glu Phe Ile Pro 450
Asp Ile Gln Glu Arg Ala Ala Thr Thr 459
Ile Arg Arg Met Tyr Arg Asp Asp Leu 468
Gly Leu Phe Lys Glu Phe Lys Ala Gln 477
Gly Val Ala Ile Arg Phe Gly Lys Phe 486
Ser Ala Lys Glu Asn Lys Gln Ile Glu 495
Lys Asn Val Gln Asp Phe Leu Ser Leu 504
Thr Gly Ile Glu Ser Ala Asp Lys Leu 513
Leu Tyr Thr Asp Arg Tyr Pro Glu Glu 522

Lys Thr Leu Ile Thr Asn Leu Lys Arg 531
Lys His Ala Phe Arg Leu His Ile Gly 540
Lys Gly Ile Ala Arg Pro Trp Lys Leu 549
Val Tyr Tyr Arg Ala Lys Lys Ile Phe 558
Asp Val Asn Asn Tyr Lys Gly Arg Tyr 567
Asn Glu Glu Asp Thr Lys Lys Leu Lys 576
Ala Tyr His Ser Leu His Gly Asn Asp 585
Trp Lys Lys Ile Gly Ala Met Val Ala 594
Arg Ser Ser Leu Ser Val Ala Leu Lys 603
Phe Ser Gln Ile Gly Gly Thr Arg Asn 612
Gln Gly Ala Trp Ser Lys Ala Glu Thr 621
Gln Arg Leu Ile Lys Ala Val Glu Asp 630
Val Ile Leu Lys Lys Met Ser Pro Gln 639
Glu Leu Arg Glu Leu Asp Ser Lys Leu 648
Gln Glu Asp Pro Glu Gly Arg Leu Ser 657
Ile Val Arg Glu Lys Leu Tyr Lys Gly 666
Ile Ser Trp Val Glu Val Glu Ala Arg 675
Val Glu Thr Arg Asn Trp Met Gln Cys 684
Lys Ser Lys Trp Thr Glu Ile Leu Thr 693
Lys Arg Met Thr His Gly Gly Phe Val 702
Tyr Arg Gly Val Asn Ala Leu Gln Ala 711
Lys Ile Thr Leu Ile Glu Arg Leu Tyr 720
Glu Leu Asn Val Asn Asp Ala Asn Glu 729
Ile Asp Trp Glu Asp Leu Cys Ser Ala 738
Ile Gly Asp Val Pro Pro Pro Phe Val 747
Gln Ala Lys Phe Tyr Lys Leu Lys Ala 756
Ala Cys Val Pro Phe Trp Gln Lys Lys 765
Thr Phe Pro Glu Ile Ile Asp Tyr Leu 774
Tyr Lys Asn Ser Leu Pro Leu Leu Lys 783
Glu Lys Leu Asp Lys Lys Met Lys Lys 792
Lys Asp Gly Gln Ile Gln Thr Pro Ala 801
Ala Pro Lys Gln Asp Phe Leu Phe Lys 810
Asp Ile Phe His Cys Asp Asp Asp Ser 819
Asp Glu Gly Ser Pro Glu Glu Pro Ser 828
Ala Ser Asp Val Gln 833

<210> 6
<211> 886
<212> PRT
<213> Homo sapiens

<220>
<223> TRANSCRIPTION TERMINATION FACTOR 1

<400> 6

<210> 7
<211> 659
<212> PRT
<213> Mus musculus

<220>
<223> b-raf

<400> 7

Met Glu Gly Gly Cys Gly Glu Gly Gly 9
Gly Gly Thr Gly Ser Gly Arg Ser Ala 18
Ala Ala Ala Arg Arg Ala Gly Arg Met 27
Arg Pro Arg Ala Gln Gly Pro Asp Ser 36
Glu Ser Gly Gly Glu Ala Ser Arg Leu 45
Asn Leu Leu Asp Thr Cys Ala Val Cys 54
His Gln Asn Ile Gln Ser Arg Val Pro 63
Lys Leu Leu Pro Cys Leu His Ser Phe 72
Cys Gln Arg Cys Leu Pro Ala Pro Gln 81
Arg Tyr Leu Met Leu Thr Ala Pro Ala 90
Leu Gly Ser Ala Glu Thr Pro Pro Pro 99
Ala Pro Ala Pro Ala Pro Ala Pro Gly 108
Ser Pro Ala Gly Gly Pro Ser Pro Phe 117
Ala Thr Gln Val Gly Val Ile Arg Cys 126
Pro Val Cys Ser Gln Glu Cys Ala Glu 135
Arg His Ile Ile Asp Asn Phe Phe Val 144
Lys Asp Thr Thr Glu Val Pro Ser Ser 153
Thr Val Glu Lys Ser Asn Gln Val Cys 162
Thr Ser Cys Glu Asp Asn Ala Glu Ala 171
Asn Gly Phe Cys Val Glu Cys Val Glu 180
Trp Leu Cys Lys Thr Cys Ile Arg Ala 189
His Gln Arg Val Lys Phe Thr Lys Asp 198
His Thr Val Arg Gln Lys Glu Glu Val 207
Ser Pro Glu Ala Val Gly Val Thr Ser 216
Gln Arg Pro Val Phe Cys Pro Phe His 225
Lys Lys Glu Gln Leu Lys Leu Tyr Cys 234
Glu Thr Cys Asp Lys Leu Thr Cys Arg 243
Asp Cys Gln Leu Leu Glu His Lys Glu 252
His Arg Tyr Gln Phe Ile Glu Glu Ala 261
Phe Gln Asn Gln Lys Val Ile Ile Asp 270
Thr Leu Ile Thr Lys Leu Met Glu Lys 279
Thr Lys Tyr Ile Lys Tyr Thr Gly Asn 288
Gln Ile Gln Asn Arg Ile Ile Glu Ile 297
Asn Gln Asn Gln Lys Gln Val Glu Gln 306
Asp Ile Lys Val Ala Ile Phe Thr Leu 315
Met Val Glu Ile Asn Lys Lys Gly Lys 324
Ala Leu Leu His Gln Leu Glu Lys Thr 333
Leu Gly Arg Arg Asp Ser Ser Asp Asp 342
Trp Glu Ile Pro Asp Gly Gln Ile Thr 351
Val Gly Gln Arg Ile Gly Ser Gly Ser 360
Phe Gly Thr Val Tyr Lys Gly Lys Trp 369
His Gly Asp Val Ala Val Lys Met Leu 378
Asn Val Thr Ala Pro Thr Pro Gln Gln 387
Leu Gln Ala Phe Lys Asn Glu Val Gly 396
Val Leu Arg Lys Thr Arg His Val Asn 405
Ile Leu Leu Phe Met Gly Tyr Ser Thr 414
Lys Pro Gln Leu Ala Ile Val Thr Gln 423
Trp Cys Glu Gly Ser Ser Leu Tyr His 432
His Leu His Ile Ile Glu Thr Lys Phe 441
Glu Met Ile Lys Leu Ile Asp Ile Ala 450

Arg Gln Thr Ala Gln Gly Met Asp Tyr 459
Leu His Ala Lys Ser Ile Ile His Arg 468
Asp Leu Lys Ser Asn Asn Ile Phe Leu 477
His Glu Asp Leu Thr Val Lys Ile Gly 486
Asp Phe Gly Leu Ala Thr Val Lys Ser 495
Arg Trp Ser Gly Ser His Gln Phe Glu 504
Gln Leu Ser Gly Ser Ile Leu Trp Met 513
Ala Pro Glu Val Ile Arg Met Gln Asp 522
Lys Asn Pro Tyr Ser Phe Gln Ser Asp 531
Val Tyr Ala Phe Gly Ile Val Leu Tyr 540
Glu Leu Met Thr Gly Gln Leu Pro Tyr 549
Ser Asn Ile Asn Asn Arg Asp Gln Ile 558
Ile Phe Met Val Gly Arg Gly Tyr Leu 567
Ser Pro Asp Leu Ser Lys Val Arg Ser 576
Asn Cys Pro Lys Ala Met Lys Arg Leu 585
Met Ala Glu Cys Leu Lys Lys Lys Arg 594
Asp Glu Arg Pro Leu Phe Pro Gln Ile 603
Leu Ala Ser Ile Glu Leu Leu Ala Arg 612
Ser Leu Pro Lys Ile His Arg Ser Ala 621
Ser Glu Pro Ser Leu Asn Arg Ala Gly 630
Phe Gln Thr Glu Asp Phe Ser Leu Tyr 639
Ala Cys Ala Ser Pro Lys Thr Pro Ile 648
Gln Ala Gly Gly Tyr Gly Gly Phe Pro 657
Val His 659

<210> 8
<211> 765
<212> PRT
<213> Homo sapiens

<220>
<223> b-raf

<400> 8

Met Ala Ala Leu Ser Gly Gly Gly Gly 9
Gly Gly Ala Glu Pro Gly Gln Ala Leu 18
Phe Asn Gly Asp Met Glu Pro Glu Ala 27
Gly Ala Gly Arg Pro Ala Ala Ser Ser 36
Ala Ala Asp Pro Ala Ile Pro Glu Glu 45
Val Trp Asn Ile Lys Gln Met Ile Lys 54
Leu Thr Gln Glu His Ile Glu Ala Leu 63
Leu Asp Lys Phe Gly Gly Glu His Asn 72
Pro Pro Ser Ile Tyr Leu Glu Ala Tyr 81
Glu Glu Tyr Thr Ser Lys Leu Asp Ala 90
Leu Gln Gln Arg Glu Gln Gln Leu Leu 99
Glu Ser Leu Gly Asn Gly Thr Asp Phe 108
Ser Val Ser Ser Ser Ala Ser Met Asp 117
Thr Val Thr Ser Ser Ser Ser Ser Ser 126
Leu Ser Val Leu Pro Ser Ser Leu Ser 135
Val Phe Gln Asn Pro Thr Asp Val Ala 144

Arg Ser Asn Pro Lys Ser Pro Gln Lys 153
Pro Ile Val Arg Val Phe Leu Pro Asn 162
Lys Gln Arg Thr Val Val Pro Ala Arg 171
Cys Gly Val Thr Val Arg Asp Ser Leu 180
Lys Lys Ala Leu Met Met Arg Gly Leu 189
Ile Pro Glu Cys Cys Ala Val Tyr Arg 198
Ile Gln Asp Gly Glu Lys Lys Pro Ile 207
Gly Trp Asp Thr Asp Ile Ser Trp Leu 216
Thr Gly Glu Glu Leu His Val Glu Val 225
Leu Glu Asn Val Pro Leu Thr Thr His 234
Asn Phe Val Arg Lys Thr Phe Phe Thr 243
Leu Ala Phe Cys Asp Phe Cys Arg Lys 252
Leu Leu Phe Gln Gly Phe Arg Cys Gln 261
Thr Cys Gly Tyr Lys Phe His Gln Arg 270
Cys Ser Thr Glu Val Pro Leu Met Cys 279
Val Asn Tyr Asp Gln Leu Asp Leu Leu 288
Phe Val Ser Lys Phe Phe Glu His His 297
Pro Ile Pro Gln Glu Glu Ala Ser Leu 306
Ala Glu Thr Ala Leu Thr Ser Gly Ser 315
Ser Pro Ser Ala Pro Ala Ser Asp Ser 324
Ile Gly Pro Gln Ile Leu Thr Ser Pro 333
Ser Pro Ser Lys Ser Ile Pro Ile Pro 342
Gln Pro Phe Arg Pro Ala Asp Glu Asp 351
His Arg Asn Gln Phe Gly Gln Arg Asp 360
Arg Ser Ser Ser Ala Pro Asn Val His 369
Ile Asn Thr Ile Glu Pro Val Asn Ile 378
Asp Asp Leu Ile Arg Asp Gln Gly Phe 387
Arg Gly Asp Gly Gly Ser Thr Thr Gly 396
Leu Ser Ala Thr Pro Pro Ala Ser Leu 405
Pro Gly Ser Leu Thr Asn Val Lys Ala 414
Leu Gln Lys Ser Pro Gly Pro Gln Arg 423
Glu Arg Lys Ser Ser Ser Ser Ser Glu 432
Asp Arg Asn Arg Met Lys Thr Leu Gly 441
Arg Arg Asp Ser Ser Asp Asp Trp Glu 450
Ile Pro Asp Gly Gln Ile Thr Val Gly 459
Gln Arg Ile Gly Ser Gly Ser Phe Gly 468
Thr Val Tyr Lys Gly Lys Trp His Gly 477
Asp Val Ala Val Lys Met Leu Asn Val 486
Thr Ala Pro Thr Pro Gln Gln Leu Gln 495
Ala Phe Lys Asn Glu Val Gly Val Leu 504
Arg Lys Thr Arg His Val Asn Ile Leu 513
Leu Phe Met Gly Tyr Ser Thr Lys Pro 522
Gln Leu Ala Ile Val Thr Gln Trp Cys 531
Glu Gly Ser Ser Leu Tyr His His Leu 540
His Ile Ile Glu Thr Lys Phe Glu Met 549
Ile Lys Leu Ile Asp Ile Ala Arg Gln 558
Thr Ala Gln Gly Met Asp Tyr Leu His 567
Ala Lys Ser Ile Ile His Arg Asp Leu 576
Lys Ser Asn Asn Ile Phe Leu His Glu 585
Asp Leu Thr Val Lys Ile Gly Asp Phe 594
Gly Leu Ala Thr Val Lys Ser Arg Trp 603
Ser Gly Ser His Gln Phe Glu Gln Leu 612

Ser Gly Ser Ile Leu Trp Met Ala Pro 621
Glu Val Ile Arg Met Gln Asp Lys Asn 630
Pro Tyr Ser Phe Gln Ser Asp Val Tyr 639
Ala Phe Gly Ile Val Leu Tyr Glu Leu 648
Met Thr Gly Gln Leu Pro Tyr Ser Asn 657
Ile Asn Asn Arg Asp Gln Ile Ile Phe 666
Met Val Gly Arg Gly Tyr Leu Ser Pro 675
Asp Leu Ser Lys Val Arg Ser Asn Cys 684
Pro Lys Ala Met Lys Arg Leu Met Ala 693
Glu Cys Leu Lys Lys Lys Arg Asp Glu 702
Arg Pro Leu Phe Pro Gln Ile Leu Ala 711
Ser Ile Glu Leu Leu Ala Arg Ser Leu 720
Pro Lys Ile His Arg Ser Ala Ser Glu 729
Pro Ser Leu Asn Arg Ala Gly Phe Gln 738
Thr Glu Asp Phe Ser Leu Tyr Ala Cys 747
Ala Ser Pro Lys Thr Pro Ile Gln Ala 756
Gly Gly Tyr Gly Ala Phe Pro Val His 765

<210> 9
<211> 111
<212> PRT
<213> Mus musculus

<220>
<223> PROTHYMOSIN ALPHA
<400> 9

Met Ser Asp Ala Ala Val Asp Thr Ser 9
Ser Glu Ile Thr Thr Lys Asp Leu Lys 18
Glu Lys Lys Glu Val Val Glu Glu Ala 27
Glu Asn Gly Arg Asp Ala Pro Ala Asn 36
Gly Asn Ala Gln Asn Glu Glu Asn Gly 45
Glu Gln Glu Ala Asp Asn Glu Val Asp 54
Glu Glu Glu Glu Glu Gly Gly Glu Glu 63
Glu Glu Glu Glu Glu Glu Gly Asp Gly 72
Glu Glu Glu Asp Gly Asp Glu Asp Glu 81
Glu Ala Glu Ala Pro Thr Gly Lys Arg 90
Val Ala Glu Asp Asp Glu Asp Asp Asp 99
Val Asp Thr Lys Lys Gln Lys Thr Glu 108
Glu Asp Asp 111

<210> 10
<211> 111
<212> PRT
<213> Homo sapiens

<220>
<223> PROTHYMOSIN ALPHA

<400> 10

Met Ser Asp Ala Ala Val Asp Thr Ser 9
Ser Glu Ile Thr Thr Lys Asp Leu Lys 18
Glu Lys Lys Glu Val Val Glu Glu Ala 27
Glu Asn Gly Arg Asp Ala Pro Ala Asn 36
Gly Asn Ala Glu Asn Glu Glu Asn Gly 45
Glu Gln Glu Ala Asp Asn Glu Val Asp 54
Glu Glu Glu Glu Glu Gly Gly Glu Glu 63
Glu Glu Glu Glu Glu Glu Gly Asp Gly 72
Glu Glu Glu Asp Gly Asp Glu Asp Glu 81
Glu Ala Glu Ser Ala Thr Gly Lys Arg 90
Ala Ala Glu Asp Asp Glu Asp Asp Asp 99
Val Asp Thr Lys Lys Gln Lys Thr Asp 108
Glu Asp Asp 111

<210> 11
<211> 233
<212> PRT
<213> Mus musculus

<220>
<223> GOLGI 4-TRANSMEMBRANE SPANNING TRANSPORTER (MTP)

<400> 11

<210> 12
<211> 233
<212> PRT
<213> Homo sapiens

<220>
<223> GOLGI 4-TRANSMEMBRANE SPANNING TRANSPORTER MTP
(KIAA0108)

<400> 12

Met Val Ser Met Ser Phe Lys Arg Asn 9
Arg Ser Asp Arg Phe Tyr Ser Thr Arg 18
Cys Cys Gly Cys Cys His Val Arg Thr 27
Gly Thr Ile Ile Leu Gly Thr Trp Tyr 36
Met Val Val Asn Leu Leu Met Ala Ile 45
Leu Leu Thr Val Glu Val Thr His Pro 54
Asn Ser Met Pro Ala Val Asn Ile Gln 63
Tyr Glu Val Ile Gly Asn Tyr Tyr Ser 72
Ser Glu Arg Met Ala Asp Asn Ala Cys 81
Val Leu Phe Ala Val Ser Val Leu Met 90
Phe Ile Ile Ser Ser Met Leu Val Tyr 99
Gly Ala Ile Ser Tyr Gln Val Gly Trp 108
Leu Ile Pro Phe Phe Cys Tyr Arg Leu 117
Phe Asp Phe Val Leu Ser Cys Leu Val 126
Ala Ile Ser Ser Leu Thr Tyr Leu Pro 135
Arg Ile Lys Glu Tyr Leu Asp Gln Leu 144
Pro Asp Phe Pro Tyr Lys Asp Asp Leu 153
Leu Ala Leu Asp Ser Ser Cys Leu Leu 162
Phe Ile Val Leu Val Phe Phe Ala Leu 171
Phe Ile Ile Phe Lys Ala Tyr Leu Ile 180
Asn Cys Val Trp Asn Cys Tyr Lys Tyr 189
Ile Asn Asn Arg Asn Val Pro Glu Ile 198
Ala Val Tyr Pro Ala Phe Glu Ala Pro 207
Pro Gln Tyr Val Leu Pro Thr Tyr Glu 216
Met Ala Val Lys Met Pro Glu Lys Glu 225
Pro Pro Pro Pro Tyr Leu Pro Ala 233

<210> 13
<211> 355
<212> PRT
<213> Mus musculus

<220>
<223> CCR-1

<400> 13

Met Glu Ile Ser Asp Phe Thr Glu Ala 9
Tyr Pro Thr Thr Thr Glu Phe Asp Tyr 18
Gly Asp Ser Thr Pro Cys Gln Lys Thr 27
Ala Val Arg Ala Phe Gly Ala Gly Leu 36
Leu Pro Pro Leu Tyr Ser Leu Val Phe 45
Ile Ile Gly Val Val Gly Asn Val Leu 54
Met Ile Leu Val Leu Met Gln His Arg 63
Arg Leu Gln Ser Met Thr Ser Ile Tyr 72
Leu Phe Asn Leu Ala Val Ser Asp Leu 81
Val Phe Leu Phe Thr Leu Pro Phe Trp 90
Ile Asp Tyr Lys Leu Lys Asp Asp Trp 99
Ile Phe Gly Asp Ala Met Cys Lys Leu 108
Leu Ser Gly Phe Tyr Tyr Leu Gly Leu 117
Tyr Ser Glu Ile Phe Phe Ile Ile Leu 126
Leu Thr Ile Asp Arg Tyr Leu Ala Ile 135
Val His Ala Val Phe Ala Leu Arg Ala 144
Arg Thr Val Thr Leu Gly Ile Ile Thr 153
Ser Ile Ile Thr Trp Ala Leu Ala Ile 162
Leu Ala Ser Met Pro Ala Leu Tyr Phe 171
Phe Lys Ala Gln Trp Glu Phe Thr His 180
Arg Thr Cys Ser Pro His Phe Pro Tyr 189
Lys Ser Leu Lys Gln Trp Lys Arg Phe 198
Gln Ala Leu Lys Leu Asn Leu Leu Gly 207
Leu Ile Leu Pro Leu Leu Val Met Ile 216
Ile Cys Tyr Ala Gly Ile Ile Arg Ile 225
Leu Leu Arg Arg Pro Ser Glu Lys Lys 234
Val Lys Ala Val Arg Leu Ile Phe Ala 243
Ile Thr Leu Leu Phe Phe Leu Leu Trp 252
Thr Pro Tyr Asn Leu Ser Val Phe Val 261
Ser Ala Phe Gln Asp Val Leu Phe Thr 270
Asn Gln Cys Glu Gln Ser Lys His Leu 279
Asp Leu Ala Met Gln Val Thr Glu Val 288
Ile Ala Tyr Thr His Cys Cys Val Asn 297
Pro Ile Ile Tyr Val Phe Val Gly Glu 306
Arg Phe Trp Lys Tyr Leu Arg Gln Leu 315
Phe Gln Arg His Val Ala Ile Pro Leu 324
Ala Lys Trp Leu Pro Phe Leu Ser Val 333
Asp Gln Leu Glu Arg Thr Ser Ser Ile 342
Ser Pro Ser Thr Gly Glu His Glu Leu 351
Ser Ala Gly Phe 355

<210> 14
<211> 355
<212> PRT
<213> Homo sapiens

<220>
<223> CCR-1

<400> 14

Met Glu Thr Pro Asn Thr Thr Glu Asp 9
Tyr Asp Thr Thr Thr Glu Phe Asp Tyr 18
Gly Asp Ala Thr Pro Cys Gln Lys Val 27
Asn Glu Arg Ala Phe Gly Ala Gln Leu 36
Leu Pro Pro Leu Tyr Ser Leu Val Phe 45
Val Ile Gly Leu Val Gly Asn Ile Leu 54
Val Val Leu Val Leu Val Gln Tyr Lys 63
Arg Leu Lys Asn Met Thr Ser Ile Tyr 72
Leu Leu Asn Leu Ala Ile Ser Asp Leu 81
Leu Phe Leu Phe Thr Leu Pro Phe Trp 90
Ile Asp Tyr Lys Leu Lys Asp Asp Trp 99
Val Phe Gly Asp Ala Met Cys Lys Ile 108
Leu Ser Gly Phe Tyr Tyr Thr Gly Leu 117
Tyr Ser Glu Ile Phe Phe Ile Ile Leu 126
Leu Thr Ile Asp Arg Tyr Leu Ala Ile 135
Val His Ala Val Phe Ala Leu Arg Ala 144
Arg Thr Val Thr Phe Gly Val Ile Thr 153
Ser Ile Ile Ile Trp Ala Leu Ala Ile 162
Leu Ala Ser Met Pro Gly Leu Tyr Phe 171
Ser Lys Thr Gln Trp Glu Phe Thr His 180
His Thr Cys Ser Leu His Phe Pro His 189
Glu Ser Leu Arg Glu Trp Lys Leu Phe 198
Gln Ala Leu Lys Leu Asn Leu Phe Gly 207
Leu Val Leu Pro Leu Leu Val Met Ile 216
Ile Cys Tyr Thr Gly Ile Ile Lys Ile 225
Leu Leu Arg Arg Pro Asn Glu Lys Lys 234
Ser Lys Ala Val Arg Leu Ile Phe Val 243
Ile Met Ile Ile Phe Phe Leu Phe Trp 252
Thr Pro Tyr Asn Leu Thr Ile Leu Ile 261
Ser Val Phe Gln Asp Phe Leu Phe Thr 270
His Glu Cys Glu Gln Ser Arg His Leu 279
Asp Leu Ala Val Gln Val Thr Glu Val 288
Ile Ala Tyr Thr His Cys Cys Val Asn 297
Pro Val Ile Tyr Ala Phe Val Gly Glu 306
Arg Phe Arg Lys Tyr Leu Arg Gln Leu 315
Phe His Arg Arg Val Ala Val His Leu 324
Val Lys Trp Leu Pro Phe Leu Ser Val 333
Asp Arg Leu Glu Arg Val Ser Ser Thr 342
Ser Pro Ser Thr Gly Glu His Glu Leu 351
Ser Ala Gly Phe 355

<210> 15
<211> 96
<212> PRT
<213> Mus musculus

<220>
<223> HMG-14

<400> 15

Met Pro Lys Arg Lys Val Ser Ala Asp 9
Gly Ala Ala Lys Ala Glu Pro Lys Arg 18
Arg Ser Ala Arg Leu Ser Ala Lys Pro 27
Ala Pro Ala Lys Val Asp Ala Lys Pro 36
Lys Lys Ala Ala Gly Lys Asp Lys Ala 45
Ser Asp Lys Lys Val Gln Ile Lys Gly 54
Lys Arg Gly Ala Lys Gly Lys Gln Ala 63
Asp Val Ala Asp Gln Gln Thr Thr Glu 72
Leu Pro Ala Glu Asn Gly Glu Thr Glu 81
Asn Gln Ser Pro Ala Ser Glu Glu Glu 90
Lys Glu Ala Lys Ser Asp 96

<210> 16
<211> 100
<212> PRT
<213> Homo sapiens

<220>
<223> HMG-14

<400> 16

Met Pro Lys Arg Lys Val Ser Ser Ala 9
Glu Gly Ala Ala Lys Glu Glu Pro Lys 18
Arg Arg Ser Ala Arg Leu Ser Ala Lys 27
Pro Pro Ala Lys Val Glu Ala Lys Pro 36
Lys Lys Ala Ala Ala Lys Asp Lys Ser 45
Ser Asp Lys Lys Val Gln Thr Lys Gly 54
Lys Arg Gly Ala Lys Gly Lys Gln Ala 63
Glu Val Ala Asn Gln Glu Thr Lys Glu 72
Asp Leu Pro Ala Glu Asn Gly Glu Thr 81
Lys Thr Glu Glu Ser Pro Ala Ser Asp 90
Glu Ala Gly Glu Lys Glu Ala Lys Ser 99
Asp 100

<210> 17
<211> 70
<212> PRT
<213> Mus musculus

<220>
<223> Deleted in split-hand/split-foot 1 region

<400> 17

Met Ser Glu Lys Lys Gln Pro Val Asp 9
Leu Gly Leu Leu Glu Glu Asp Asp Glu 18
Phe Glu Glu Phe Pro Ala Glu Asp Trp 27

Ala Gly Leu Asp Glu Asp Glu Asp Ala 36
His Val Trp Glu Asp Asn Trp Asp Asp 45
Asp Asn Val Glu Asp Asp Phe Ser Asn 54
Gln Leu Arg Ala Glu Leu Glu Lys His 63
Gly Tyr Lys Met Glu Thr Ser 70

<210> 18
<211> 70
<212> PRT
<213> Homo sapiens

<220>
<223> Deleted in split-hand/split-foot 1 region

<400> 18

Met Ser Glu Lys Lys Gln Pro Val Asp 9
Leu Gly Leu Leu Glu Glu Asp Asp Glu 18
Phe Glu Glu Phe Pro Ala Glu Asp Trp 27
Ala Gly Leu Asp Glu Asp Glu Asp Ala 36
His Val Trp Glu Asp Asn Trp Asp Asp 45
Asp Asn Val Glu Asp Asp Phe Ser Asn 54
Gln Leu Arg Ala Glu Leu Glu Lys His 63
Gly Tyr Lys Met Glu Thr Ser 70

<210> 19
<211> 596
<212> PRT
<213> Mus musculus

<220>
<223> TAK1

<400> 19

Met Thr Ser Pro Ser Pro Arg Ile Gln 9
Ile Ile Ser Thr Asp Ser Ala Val Ala 18
Ser Pro Gln Arg Ile Gln Ile Val Thr 27
Asp Gln Gln Thr Gly Gln Lys Ile Gln 36
Ile Val Thr Ala Val Asp Ala Ser Gly 45
Ser Ser Lys Gln Gln Phe Ile Leu Thr 54
Ser Pro Asp Gly Ala Gly Thr Gly Lys 63
Val Ile Leu Ala Ser Pro Glu Thr Ser 72
Ser Ala Lys Gln Leu Ile Phe Thr Thr 81
Ser Asp Asn Leu Val Pro Gly Arg Ile 90
Gln Ile Val Thr Asp Ser Ala Ser Val 99
Glu Arg Leu Leu Gly Lys Ala Asp Val 108
Gln Arg Pro Gln Val Val Glu Tyr Cys 117
Val Val Cys Gly Asp Lys Ala Ser Gly 126
Arg His Tyr Gly Ala Val Ser Cys Glu 135

Gly Cys Lys Gly Phe Phe Lys Arg Ser 144
Val Arg Lys Asn Leu Thr Tyr Ser Cys 153
Arg Ser Ser Gln Asp Cys Ile Ile Asn 162
Lys His His Arg Asn Arg Cys Gln Phe 171
Cys Arg Leu Lys Lys Cys Leu Glu Met 180
Gly Met Lys Met Glu Ser Val Gln Ser 189
Glu Arg Lys Pro Phe Asp Val Gln Arg 198
Glu Lys Pro Ser Asn Cys Ala Ala Ser 207
Thr Glu Lys Ile Tyr Ile Arg Lys Asp 216
Leu Arg Ser Pro Leu Ile Ala Thr Pro 225
Thr Phe Val Ala Asp Lys Asp Gly Ala 234
Arg Gln Thr Gly Leu Leu Asp Pro Gly 243
Met Leu Val Asn Ile Gln Gln Pro Leu 252
Ile Arg Glu Asp Gly Thr Val Leu Leu 261
Ala Ala Asp Ser Lys Ala Glu Thr Ser 270
Gln Gly Ala Leu Gly Thr Leu Ala Asn 279
Val Val Thr Ser Leu Ala Asn Leu Ser 288
Glu Ser Leu Asn Asn Gly Asp Ala Ser 297
Glu Met Gln Pro Glu Asp Gln Ser Ala 306
Ser Glu Ile Thr Arg Ala Phe Asp Thr 315
Leu Ala Lys Ala Leu Asn Thr Thr Asp 324
Ser Ala Ser Pro Pro Ser Leu Ala Asp 333
Gly Ile Asp Ala Ser Gly Gly Gly Ser 342
Ile His Val Ile Ser Arg Asp Gln Ser 351
Thr Pro Ile Ile Glu Val Glu Gly Pro 360
Leu Leu Ser Asp Thr His Val Thr Phe 369
Lys Leu Thr Met Pro Ser Pro Met Pro 378
Glu Tyr Leu Asn Val His Tyr Ile Cys 387
Glu Ser Ala Ser Arg Leu Leu Phe Leu 396
Ser Met His Trp Ala Arg Ser Ile Pro 405
Ala Phe Gln Ala Leu Gly Gln Asp Cys 414
Asn Thr Ser Leu Val Arg Ala Cys Trp 423
Asn Glu Leu Phe Thr Leu Gly Leu Ala 432
Gln Cys Ala Gln Val Met Ser Leu Ser 441
Thr Ile Leu Ala Ala Ile Val Asn His 450
Leu Gln Asn Ser Ile Gln Glu Asp Lys 459
Leu Ser Gly Asp Arg Ile Lys Gln Val 468
Met Glu His Ile Trp Lys Leu Gln Glu 477
Phe Cys Asn Ser Met Ala Lys Leu Asp 486
Ile Asp Gly Tyr Glu Tyr Ala Tyr Leu 495
Lys Ala Ile Val Leu Phe Ser Pro Asp 504
His Pro Gly Leu Thr Gly Thr Ser Gln 513
Ile Glu Lys Phe Gln Glu Lys Ala Gln 522
Met Glu Leu Gln Asp Tyr Val Gln Lys 531
Thr Tyr Ser Glu Asp Thr Tyr Arg Leu 540
Ala Arg Ile Leu Val Arg Leu Pro Ala 549
Leu Arg Leu Met Ser Ser Asn Ile Thr 558
Glu Glu Leu Phe Phe Thr Gly Leu Ile 567
Gly Asn Val Ser Ile Asp Ser Ile Ile 576
Pro Tyr Ile Leu Lys Met Glu Thr Ala 585
Glu Tyr Asn Gly Gln Ile Thr Gly Ala 594

Ser    Leu    596

<210> 20
<211> 596
<212> PRT
<213> Homo sapiens

<220>
<223> TAK1

<400> 20

Met    Thr    Ser    Pro    Ser    Pro    Arg    Ile    Gln    9
Ile    Ile    Ser    Thr    Asp    Ser    Ala    Val    Ala    18
Ser    Pro    Gln    Arg    Ile    Gln    Ile    Val    Thr    27
Asp    Gln    Gln    Thr    Gly    Gln    Lys    Ile    Gln    36
Ile    Val    Thr    Ala    Val    Asp    Ala    Ser    Gly    45
Ser    Pro    Lys    Gln    Gln    Phe    Ile    Leu    Thr    54
Ser    Pro    Asp    Gly    Ala    Gly    Thr    Gly    Lys    63
Val    Ile    Leu    Ala    Ser    Pro    Glu    Thr    Ser    72
Ser    Ala    Lys    Gln    Leu    Ile    Phe    Thr    Thr    81
Ser    Asp    Asn    Leu    Val    Pro    Gly    Arg    Ile    90
Gln    Ile    Val    Thr    Asp    Ser    Ala    Ser    Val    99
Glu    Arg    Leu    Leu    Gly    Lys    Thr    Asp    Val    108
Gln    Arg    Pro    Gln    Val    Val    Glu    Tyr    Cys    117
Val    Val    Cys    Gly    Asp    Lys    Ala    Ser    Gly    126
Arg    His    Tyr    Gly    Ala    Val    Ser    Cys    Glu    135
Gly    Cys    Lys    Gly    Phe    Phe    Lys    Arg    Ser    144
Val    Arg    Lys    Asn    Leu    Thr    Tyr    Ser    Cys    153
Arg    Ser    Asn    Gln    Asp    Cys    Ile    Ile    Asn    162
Lys    His    His    Arg    Asn    Arg    Cys    Gln    Phe    171
Cys    Arg    Leu    Lys    Lys    Cys    Leu    Glu    Met    180
Gly    Met    Lys    Met    Glu    Ser    Val    Gln    Ser    189
Glu    Arg    Lys    Pro    Phe    Asp    Val    Gln    Arg    198
Glu    Lys    Pro    Ser    Asn    Cys    Ala    Ala    Ser    207
Thr    Glu    Lys    Ile    Tyr    Ile    Arg    Lys    Asp    216
Leu    Arg    Ser    Pro    Leu    Ile    Ala    Thr    Pro    225
Thr    Phe    Val    Ala    Asp    Lys    Asp    Gly    Ala    234
Arg    Gln    Thr    Gly    Leu    Leu    Asp    Pro    Gly    243
Met    Leu    Val    Asn    Ile    Gln    Gln    Pro    Leu    252
Ile    Arg    Glu    Asp    Gly    Thr    Val    Leu    Leu    261
Ala    Thr    Asp    Ser    Lys    Ala    Glu    Thr    Ser    270
Gln    Gly    Ala    Leu    Gly    Thr    Leu    Ala    Asn    279
Val    Val    Thr    Ser    Leu    Ala    Asn    Leu    Ser    288
Glu    Ser    Leu    Asn    Asn    Gly    Asp    Thr    Ser    297
Glu    Ile    Gln    Pro    Glu    Asp    Gln    Ser    Ala    306
Ser    Glu    Ile    Thr    Arg    Ala    Phe    Asp    Thr    315
Leu    Ala    Lys    Ala    Leu    Asn    Thr    Thr    Asp    324
Ser    Ser    Ser    Ser    Pro    Ser    Leu    Ala    Asp    333
Gly    Ile    Asp    Thr    Ser    Gly    Gly    Gly    Ser    342
Ile    His    Val    Ile    Ser    Arg    Asp    Gln    Ser    351

Thr Pro Ile Ile Glu Val Glu Gly Pro 360
Leu Leu Ser Asp Thr His Val Thr Phe 369
Lys Leu Thr Met Pro Ser Pro Met Pro 378
Glu Tyr Leu Asn Val His Tyr Ile Cys 387
Glu Ser Ala Ser Arg Leu Leu Phe Leu 396
Ser Met His Trp Ala Arg Ser Ile Pro 405
Ala Phe Gln Ala Leu Gly Gln Asp Cys 414
Asn Thr Ser Leu Val Arg Ala Cys Trp 423
Asn Glu Leu Phe Thr Leu Gly Leu Ala 432
Gln Cys Ala Gln Val Met Ser Leu Ser 441
Thr Ile Leu Ala Ala Ile Val Asn His 450
Leu Gln Asn Ser Ile Gln Glu Asp Lys 459
Leu Ser Gly Asp Arg Ile Lys Gln Val 468
Met Glu His Ile Trp Lys Leu Gln Glu 477
Phe Cys Asn Ser Met Ala Lys Leu Asp 486
Ile Asp Gly Tyr Glu Tyr Ala Tyr Leu 495
Lys Ala Ile Val Leu Phe Ser Pro Asp 504
His Pro Gly Leu Thr Ser Thr Ser Gln 513
Ile Glu Lys Phe Gln Glu Lys Ala Gln 522
Met Glu Leu Gln Asp Tyr Val Gln Lys 531
Thr Tyr Ser Glu Asp Thr Tyr Arg Leu 540
Ala Arg Ile Leu Val Arg Leu Pro Ala 549
Leu Arg Leu Met Ser Ser Asn Ile Thr 558
Glu Glu Leu Phe Phe Thr Gly Leu Ile 567
Gly Asn Val Ser Ile Asp Ser Ile Ile 576
Pro Tyr Ile Leu Lys Met Glu Thr Ala 585
Glu Tyr Asn Gly Gln Ile Thr Gly Ala 594
Ser Leu 596

<210> 21
<211> 97
<212> PRT
<213> Mus musculus

<220>
<223> MCP-3

<400> 21
   Met Arg Ile Ser Ala Thr Leu Leu Cys 9
   Leu Leu Leu Ile Ala Ala Ala Phe Ser 18
   Ile Gln Val Trp Ala Gln Pro Asp Gly 27
   Pro Asn Ala Ser Thr Cys Cys Tyr Val 36
   Lys Lys Gln Lys Ile Pro Lys Arg Asn 45
   Leu Lys Ser Tyr Arg Arg Ile Thr Ser 54
   Ser Arg Cys Pro Trp Glu Ala Val Ile 63
   Phe Lys Thr Lys Lys Gly Met Glu Val 72
   Cys Ala Glu Ala His Gln Lys Trp Val 81
   Glu Glu Ala Ile Ala Tyr Leu Asp Met 90
   Lys Thr Pro Thr Pro Lys Pro 97

<210> 22
<211> 99
<212> PRT
<213> Homo sapiens

<220>
<223> MCP-3

<400> 22

Met Lys Ala Ser Ala Ala Leu Leu Cys 9
Leu Leu Leu Thr Ala Ala Ala Phe Ser 18
Pro Gln Gly Leu Ala Gln Pro Val Gly 27
Ile Asn Thr Ser Thr Thr Cys Cys Tyr 36
Arg Phe Ile Asn Lys Lys Ile Pro Lys 45
Gln Arg Leu Glu Ser Tyr Arg Arg Thr 54
Thr Ser Ser His Cys Pro Arg Glu Ala 63
Val Ile Phe Lys Thr Lys Leu Asp Lys 72
Glu Ile Cys Ala Asp Pro Thr Gln Lys 81
Trp Val Gln Asp Phe Met Lys His Leu 90
Asp Lys Lys Thr Gln Thr Pro Lys Leu 99

<210> 23
<211> 415
<212> PRT
<213> Mus musculus

<220>
<223> IL-5R-ALPHA

<400> 23

Met Val Pro Val Leu Leu Ile Leu Val 9
Gly Ala Leu Ala Thr Leu Gln Ala Asp 18
Leu Leu Asn His Lys Lys Phe Leu Leu 27
Leu Pro Pro Val Asn Phe Thr Ile Lys 36
Ala Thr Gly Leu Ala Gln Val Leu Leu 45
His Trp Asp Pro Asn Pro Asp Gln Glu 54
Gln Arg His Val Asp Leu Glu Tyr His 63
Val Lys Ile Asn Ala Pro Gln Glu Asp 72
Glu Tyr Asp Thr Arg Lys Thr Glu Ser 81
Lys Cys Val Thr Pro Leu His Glu Gly 90
Phe Ala Ala Ser Val Arg Thr Ile Leu 99
Lys Ser Ser His Thr Thr Leu Ala Ser 108
Ser Trp Val Ser Ala Glu Leu Lys Ala 117
Pro Pro Gly Ser Pro Gly Thr Ser Val 126

Thr Asn Leu Thr Cys Thr Thr His Thr 135
Val Val Ser Ser His Thr His Leu Arg 144
Pro Tyr Gln Val Ser Leu Arg Cys Thr 153
Trp Leu Val Gly Lys Asp Ala Pro Glu 162
Asp Thr Gln Tyr Phe Leu Tyr Tyr Arg 171
Phe Gly Val Leu Thr Glu Lys Cys Gln 180
Glu Tyr Ser Arg Asp Ala Leu Asn Arg 189
Asn Thr Ala Cys Trp Phe Pro Arg Thr 198
Phe Ile Asn Ser Lys Gly Phe Glu Gln 207
Leu Ala Val His Ile Asn Gly Ser Ser 216
Lys Arg Ala Ala Ile Lys Pro Phe Asp 225
Gln Leu Phe Ser Pro Leu Ala Ile Asp 234
Gln Val Asn Pro Pro Arg Asn Val Thr 243
Val Glu Ile Glu Ser Asn Ser Leu Tyr 252
Ile Gln Trp Glu Lys Pro Leu Ser Ala 261
Phe Pro Asp His Cys Phe Asn Tyr Glu 270
Leu Lys Ile Tyr Asn Thr Lys Asn Gly 279
His Ile Gln Lys Glu Lys Leu Ile Ala 288
Asn Lys Phe Ile Ser Lys Ile Asp Asp 297
Val Ser Thr Tyr Ser Ile Gln Val Arg 306
Ala Ala Val Ser Ser Pro Cys Arg Met 315
Pro Gly Arg Trp Gly Glu Trp Ser Gln 324
Pro Ile Tyr Val Gly Lys Glu Arg Lys 333
Ser Leu Val Glu Trp His Leu Ile Val 342
Leu Pro Thr Ala Ala Cys Phe Val Leu 351
Leu Ile Phe Ser Leu Ile Cys Arg Val 360
Cys His Leu Trp Thr Arg Leu Phe Pro 369
Pro Val Pro Ala Pro Lys Ser Asn Ile 378
Lys Asp Leu Pro Val Val Thr Glu Tyr 387
Glu Lys Pro Ser Asn Glu Thr Lys Ile 396
Glu Val Val His Cys Val Glu Glu Val 405
Gly Phe Glu Val Met Gly Asn Ser Thr 414
Phe 415

<210> 24
<211> 420
<212> PRT
<213> Homo sapiens

<220>
<223> IL-5R-ALPH

<400> 24

<210> 25
<211> 113
<212> PRT
<213> Mus musculus

<220>
<223> DEFENDER AGAINST CELL DEATH 1 (DAD-1)

<400> 25

Met Ser Ala Ser Val Val Ser Val Ile 9
Ser Arg Phe Leu Glu Glu Tyr Leu Ser 18
Ser Thr Pro Gln Arg Leu Lys Leu Leu 27
Asp Gly Tyr Leu Leu Tyr Ile Leu Leu 36
Thr Gly Ala Leu Gln Phe Gly Tyr Cys 45
Leu Leu Val Gly Thr Phe Pro Phe Asn 54
Ser Phe Leu Ser Gly Phe Ile Ser Cys 63
Val Gly Ser Phe Ile Leu Ala Val Cys 72
Leu Arg Ile Gln Ile Asn Pro Gln Asn 81
Lys Ala Asp Phe Gln Gly Ile Ser Pro 90
Glu Arg Ala Phe Ala Asp Phe Leu Phe 99
Gly Ser Thr Ile Leu His Leu Val Val 108
Met Asn Phe Val Gly 113


<210> 26
<211> 113
<212> PRT
<213> Homo sapiens

<220>
<223> DEFENDER AGAINST CELL DEATH 1 (DAD-1)

<400> 26

Met Ser Ala Ser Val Val Ser Val Ile 9
Ser Arg Phe Leu Glu Glu Tyr Leu Ser 18
Ser Thr Pro Gln Arg Leu Lys Leu Leu 27
Asp Ala Tyr Leu Leu Tyr Ile Leu Leu 36
Thr Gly Ala Leu Gln Phe Gly Tyr Cys 45
Leu Leu Val Gly Thr Phe Pro Phe Asn 54
Ser Phe Leu Ser Gly Phe Ile Ser Cys 63
Val Gly Ser Phe Ile Leu Ala Val Cys 72
Leu Arg Ile Gln Ile Asn Pro Gln Asn 81
Lys Ala Asp Phe Gln Gly Ile Ser Pro 90
Glu Arg Ala Phe Ala Asp Phe Leu Phe 99
Ala Ser Thr Ile Leu His Leu Val Val 108
Met Asn Phe Val Gly 113


<210> 27
<400> 27
000


<210> 28
<400> 28
000

<210> 29
<211> 97
<212> PRT
<213> Mus musculus

<220>
<223> MCP-2

<400> 29

Met Lys Ile Tyr Ala Val Leu Leu Cys 9
Leu Leu Leu Ile Ala Val Pro Val Ser 18
Pro Glu Lys Leu Thr Gly Pro Asp Lys 27
Ala Pro Val Thr Cys Cys Phe His Val 36
Leu Lys Leu Lys Ile Pro Leu Arg Val 45
Leu Lys Ser Tyr Glu Arg Ile Asn Asn 54
Ile Gln Cys Pro Met Glu Ala Val Val 63
Phe Gln Thr Lys Gln Gly Met Ser Leu 72
Cys Val Asp Pro Thr Gln Lys Trp Val 81
Ser Glu Tyr Met Glu Ile Leu Asp Gln 90
Lys Ser Gln Ile Leu Gln Pro 97


<210> 30
<211> 99
<212> PRT
<213> Homo sapiens

<220>
<223> MCP-2

<400> 30

Met Lys Val Ser Ala Ala Leu Leu Cys 9
Leu Leu Leu Met Ala Ala Thr Phe Ser 18
Pro Gln Gly Leu Ala Gln Pro Asp Ser 27
Val Ser Ile Pro Ile Thr Cys Cys Phe 36
Asn Val Ile Asn Arg Lys Ile Pro Ile 45
Gln Arg Leu Glu Ser Tyr Thr Arg Ile 54
Thr Asn Ile Gln Cys Pro Lys Glu Ala 63
Val Ile Phe Lys Thr Gln Arg Gly Lys 72
Glu Val Cys Ala Asp Pro Lys Glu Arg 81
Trp Val Arg Asp Ser Met Lys His Leu 90
Asp Gln Ile Phe Gln Asn Leu Lys Pro 99


<210> 31
<211> 411
<212> PRT
<213> Mus musculus

<220>
<223> BAF57

<400> 31

Met Ser Lys Arg Pro Ser Tyr Ala Pro 9
Pro Pro Thr Pro Ala Pro Ala Thr Gln 18
Met Pro Ser Thr Pro Gly Phe Val Gly 27
Tyr Asn Pro Tyr Ser His Leu Ala Tyr 36
Asn Asn Tyr Arg Leu Gly Gly Asn Pro 45
Gly Thr Asn Ser Arg Val Thr Ala Ser 54
Ser Gly Ile Thr Ile Pro Lys Pro Pro 63
Lys Pro Pro Asp Lys Pro Leu Met Pro 72
Tyr Met Arg Tyr Ser Arg Lys Val Trp 81
Asp Gln Val Lys Ala Ser Asn Pro Asp 90
Leu Lys Leu Trp Glu Ile Gly Lys Ile 99
Ile Gly Gly Met Trp Arg Asp Leu Thr 108
Asp Glu Glu Lys Gln Glu Tyr Leu Asn 117
Glu Tyr Glu Ala Glu Lys Ile Glu Tyr 126
Asn Glu Ser Met Lys Ala Tyr His Asn 135
Ser Pro Ala Tyr Leu Ala Tyr Ile Asn 144
Ala Lys Ser Arg Ala Glu Ala Ala Leu 153
Glu Glu Glu Ser Arg Gln Arg Gln Ser 162
Arg Met Glu Lys Gly Glu Pro Tyr Met 171
Ser Ile Gln Pro Ala Glu Asp Pro Asp 180
Asp Tyr Asp Asp Gly Phe Ser Met Lys 189
His Thr Ala Thr Ala Arg Phe Gln Arg 198
Asn His Arg Leu Ile Ser Glu Ile Leu 207
Ser Glu Ser Val Val Pro Asp Val Arg 216
Ser Val Val Thr Thr Ala Arg Met Gln 225
Val Leu Lys Arg Gln Val Gln Ser Leu 234
Met Val His Gln Arg Lys Leu Glu Ala 243
Glu Leu Leu Gln Ile Glu Glu Arg His 252
Gln Glu Lys Lys Arg Lys Phe Leu Glu 261
Ser Thr Asp Ser Phe Asn Asn Glu Leu 270
Lys Arg Leu Cys Gly Leu Lys Val Glu 279
Val Asp Met Glu Lys Ile Ala Ala Glu 288
Ile Ala Gln Ala Glu Glu Gln Ala Arg 297
Lys Arg Gln Glu Glu Arg Glu Lys Glu 306
Ala Ala Glu Gln Ala Glu Arg Ser Gln 315
Ser Ser Met Ala Pro Glu Glu Glu Gln 324
Val Ala Asn Lys Ala Glu Glu Lys Lys 333
Asp Glu Glu Ser Ile Pro Met Glu Thr 342
Glu Glu Thr His Leu Glu Asp Thr Ala 351
Glu Ser Gln Gln Asn Gly Glu Glu Gly 360
Thr Ser Thr Pro Glu Asp Lys Glu Ser 369
Gly Gln Glu Gly Val Asp Ser Met Glu 378
Val Glu Gly Thr Ser Asp Ser Asn Thr 387
Gly Ser Glu Ser Asn Ser Ala Thr Val 396
Glu Glu Pro Pro Thr Asp Pro Val Pro 405
Glu Asp Glu Lys Lys Glu 411

<210> 32
<211> 411
<212> PRT
<213> Homo sapiens

<220>
<223> BAF57

<400> 32

<210> 33
<211> 821
<212> PRT
<213> Mus musculus

<220>
<223> EPS8

<400> 33

Met Asn Gly His Met Ser Asn Arg Ser 9
Ser Gly Tyr Gly Val Tyr Pro Ser Gln 18
Leu Asn Gly Tyr Gly Ser Ser Pro Pro 27
Tyr Ser Gln Met Asp Arg Glu His Ser 36
Ser Arg Thr Ser Ala Lys Ala Leu Tyr 45
Glu Gln Arg Lys Asn Tyr Ala Arg Asp 54
Ser Val Ser Ser Val Ser Asp Val Ser 63
Gln Tyr Arg Val Glu His Leu Thr Thr 72
Phe Val Leu Asp Arg Lys Asp Ala Met 81
Ile Thr Val Glu Asp Gly Ile Arg Lys 90
Leu Lys Leu Leu Asp Ala Lys Gly Lys 99
Val Trp Thr Gln Asp Met Ile Leu Gln 108
Val Asp Asp Arg Ala Val Ser Leu Ile 117
Asp Leu Glu Ser Lys Asn Glu Leu Glu 126
Asn Phe Pro Leu Asn Thr Ile Ser His 135
Cys Gln Ala Val Val His Ala Cys Ser 144
Tyr Asp Ser Ile Leu Ala Leu Val Cys 153
Lys Glu Pro Thr Gln Ser Lys Pro Asp 162
Leu His Leu Phe Gln Cys Asp Glu Val 171
Lys Ala Asn Leu Ile Ser Glu Asp Ile 180
Glu Ser Ala Ile Ser Asp Ser Lys Gly 189
Gly Lys Gln Lys Arg Arg Pro Glu Ala 198
Leu Arg Met Ile Ala Lys Ala Asp Pro 207
Gly Ile Pro Pro Pro Pro Arg Ala Pro 216
Ala Pro Val Pro Pro Gly Thr Val Thr 225
Gln Val Asp Val Arg Ser Arg Val Ala 234
Ala Trp Ser Ala Trp Ala Ala Asp Gln 243
Gly Asp Phe Glu Lys Pro Arg Gln Tyr 252
His Glu Gln Glu Glu Thr Pro Glu Met 261
Met Ala Ala Arg Ile Asp Arg Asp Val 270
Gln Ile Leu Asn His Ile Leu Asp Asp 279
Ile Glu Phe Phe Ile Thr Lys Leu Gln 288
Lys Ala Ala Glu Ala Phe Ser Glu Leu 297
Ser Lys Arg Lys Lys Ser Lys Lys Ser 306

Lys Arg Lys Gly Pro Gly Glu Gly Val 315
Leu Thr Leu Arg Ala Lys Pro Pro Pro 324
Pro Asp Glu Phe Val Asp Cys Phe Gln 333
Lys Phe Lys His Gly Phe Asn Leu Leu 342
Ala Lys Leu Lys Ser His Ile Gln Asn 351
Pro Ser Ala Ser Asp Leu Val His Phe 360
Leu Phe Thr Pro Leu Asn Met Val Val 369
Gln Ala Thr Gly Gly Pro Glu Leu Ala 378
Ser Ser Val Leu Ser Pro Leu Leu Thr 387
Lys Asp Thr Val Asp Phe Leu Asn Tyr 396
Thr Ala Thr Ala Glu Glu Arg Lys Leu 405
Trp Met Ser Leu Gly Asp Ser Trp Val 414
Lys Val Arg Ala Glu Trp Pro Lys Glu 423
Gln Phe Ile Pro Pro Tyr Val Pro Arg 432
Phe Arg Asn Gly Trp Glu Pro Pro Met 441
Leu Asn Phe Met Gly Ala Pro Thr Glu 450
Gln Asp Met Tyr Gln Leu Ala Glu Ser 459
Val Ala Asn Ala Glu His Gln Arg Lys 468
Gln Asp Ser Lys Arg Leu Ser Thr Glu 477
His Ser Asn Val Ser Asp Tyr Pro Pro 486
Ala Asp Gly Tyr Ala Tyr Ser Ser Ser 495
Met Tyr His Arg Gly Pro His Ala Asp 504
His Gly Glu Ala Ala Met Pro Phe Lys 513
Ser Thr Pro Asn His Gln Val Asp Arg 522
Asn Tyr Asp Ala Val Lys Thr Gln Pro 531
Lys Lys Tyr Ala Lys Ser Lys Tyr Asp 540
Phe Val Ala Arg Asn Ser Ser Glu Leu 549
Ser Val Met Lys Asp Asp Val Leu Glu 558
Ile Leu Asp Asp Arg Arg Gln Trp Trp 567
Lys Val Arg Asn Ala Ser Gly Asp Ser 576
Gly Phe Val Pro Asn Asn Ile Leu Asp 585
Ile Met Arg Thr Pro Glu Ser Gly Val 594
Gly Arg Ala Asp Pro Pro Tyr Thr His 603
Thr Ile Gln Lys Gln Arg Thr Glu Tyr 612
Gly Leu Arg Ser Ala Asp Thr Pro Ser 621
Ala Pro Ser Pro Pro Pro Thr Pro Ala 630
Pro Val Pro Val Pro Leu Pro Pro Ser 639
Val Pro Ala Pro Val Ser Val Pro Lys 648
Val Pro Ala Asp Val Thr Arg Gln Asn 657
Ser Ser Ser Ser Asp Ser Gly Gly Ser 666
Ile Val Arg Asp Ser Gln Arg Tyr Lys 675
Gln Leu Pro Val Asp Arg Arg Lys Ser 684
Gln Met Glu Glu Val Gln Asp Glu Leu 693
Phe Gln Arg Leu Thr Ile Gly Arg Ser 702
Ala Ala Gln Arg Lys Phe His Val Pro 711
Arg Gln Asn Val Pro Val Ile Asn Ile 720
Thr Tyr Asp Ser Ser Pro Glu Glu Val 729
Lys Thr Trp Leu Gln Ser Lys Gly Phe 738
Asn Pro Val Thr Val Asn Ser Leu Gly 747
Val Leu Asn Gly Ala Gln Leu Phe Ser 756
Leu Asn Lys Asp Glu Leu Arg Ser Val 765
Cys Pro Glu Gly Ala Arg Val Phe Asn 774

Gln Ile Thr Val Gln Lys Ala Ala Leu 783
Glu Asp Ser Asn Gly Ser Ser Glu Leu 792
Gln Glu Ile Met Arg Arg Arg Gln Glu 801
Lys Ile Ser Ala Ala Ala Ser Asp Ser 810
Gly Val Glu Ser Phe Asp Glu Gly Ser 819
Ser His 821

<210> 34
<211> 822
<212> PRT
<213> Homo sapiens

<220>
<223> EPS8

<400> 34

Met Asn Gly His Ile Ser Asn His Pro 9
Ser Ser Phe Gly Met Tyr Pro Ser Gln 18
Met Asn Gly Tyr Gly Ser Ser Pro Thr 27
Phe Ser Gln Thr Asp Arg Glu His Gly 36
Ser Lys Thr Ser Ala Lys Ala Leu Tyr 45
Glu Gln Arg Lys Asn Tyr Ala Arg Asp 54
Ser Val Ser Ser Val Ser Asp Ile Ser 63
Gln Tyr Arg Val Glu His Leu Thr Thr 72
Phe Val Leu Asp Arg Lys Asp Ala Met 81
Ile Thr Val Asp Asp Gly Ile Arg Lys 90
Leu Lys Leu Leu Asp Ala Lys Gly Lys 99
Val Trp Thr Gln Asp Met Ile Leu Gln 108
Val Asp Asp Arg Ala Val Ser Leu Ile 117
Asp Leu Glu Ser Lys Asn Glu Leu Glu 126
Asn Phe Pro Leu Asn Thr Ile Gln His 135
Cys Gln Ala Val Met His Ser Cys Ser 144
Tyr Asp Ser Val Leu Ala Leu Val Cys 153
Lys Glu Pro Thr Gln Asn Lys Pro Asp 162
Leu His Leu Phe Gln Cys Asp Glu Val 171
Lys Ala Asn Leu Ile Ser Glu Asp Ile 180
Glu Ser Ala Ile Ser Asp Ser Lys Gly 189
Gly Lys Gln Lys Arg Arg Pro Asp Ala 198
Leu Arg Met Ile Ser Asn Ala Asp Pro 207
Ser Ile Pro Pro Pro Pro Arg Ala Pro 216
Ala Pro Ala Pro Pro Gly Thr Val Thr 225
Gln Val Asp Val Arg Ser Arg Val Ala 234
Ala Trp Ser Ala Trp Ala Ala Asp Gln 243
Gly Asp Phe Glu Lys Pro Arg Gln Tyr 252
His Glu Gln Glu Glu Thr Pro Glu Met 261
Met Ala Ala Arg Ile Asp Arg Asp Val 270
Gln Ile Leu Asn His Ile Leu Asp Asp 279
Ile Glu Phe Phe Ile Thr Lys Leu Gln 288
Lys Ala Ala Glu Ala Phe Ser Glu Leu 297
Ser Lys Arg Lys Lys Asn Lys Lys Gly 306

Lys Arg Lys Gly Pro Gly Glu Gly Val 315
Leu Thr Leu Arg Ala Lys Pro Pro Pro 324
Pro Asp Glu Phe Leu Asp Cys Phe Gln 333
Lys Phe Lys His Gly Phe Asn Leu Leu 342
Ala Lys Leu Lys Ser His Ile Gln Asn 351
Pro Ser Ala Ala Asp Leu Val His Phe 360
Leu Phe Thr Pro Leu Asn Met Val Val 369
Gln Ala Thr Gly Gly Pro Glu Leu Ala 378
Ser Ser Val Leu Ser Pro Leu Leu Asn 387
Lys Asp Thr Ile Asp Phe Leu Asn Tyr 396
Thr Val Asn Gly Asp Glu Arg Gln Leu 405
Trp Met Ser Leu Gly Gly Thr Trp Met 414
Lys Ala Arg Ala Glu Trp Pro Lys Glu 423
Gln Phe Ile Pro Pro Tyr Val Pro Arg 432
Phe Arg Asn Gly Trp Glu Pro Pro Met 441
Leu Asn Phe Met Gly Ala Thr Met Glu 450
Gln Asp Leu Tyr Gln Leu Ala Glu Ser 459
Val Ala Asn Val Ala Glu His Gln Arg 468
Lys Gln Glu Ile Lys Arg Leu Ser Thr 477
Glu His Ser Ser Val Ser Glu Tyr His 486
Pro Ala Asp Gly Tyr Ala Phe Ser Ser 495
Asn Ile Tyr Thr Arg Gly Ser His Leu 504
Asp Gln Gly Glu Ala Ala Val Ala Phe 513
Lys Pro Thr Ser Asn Arg His Ile Asp 522
Arg Asn Tyr Glu Pro Leu Lys Thr Gln 531
Pro Lys Lys Tyr Ala Lys Ser Lys Tyr 540
Asp Phe Val Ala Arg Asn Asn Ser Glu 549
Leu Ser Val Leu Lys Asp Asp Ile Leu 558
Glu Ile Leu Asp Asp Arg Lys Gln Trp 567
Trp Lys Val Arg Asn Ala Ser Gly Asp 576
Ser Gly Phe Val Pro Asn Asn Ile Leu 585
Asp Ile Val Arg Pro Pro Glu Ser Gly 594
Leu Gly Arg Ala Asp Pro Pro Tyr Thr 603
His Thr Ile Gln Lys Gln Arg Met Glu 612
Tyr Gly Pro Arg Pro Ala Asp Thr Pro 621
Pro Ala Pro Ser Pro Pro Pro Thr Pro 630
Ala Pro Val Pro Val Pro Leu Pro Pro 639
Ser Thr Pro Ala Pro Val Pro Val Ser 648
Lys Val Pro Ala Asn Ile Thr Arg Gln 657
Asn Ser Ser Ser Ser Asp Ser Gly Gly 666
Ser Ile Val Arg Asp Ser Gln Arg His 675
Lys Gln Leu Pro Val Asp Arg Arg Lys 684
Ser Gln Met Glu Glu Val Gln Asp Glu 693
Leu Ile His Arg Leu Thr Ile Gly Arg 702
Ser Ala Ala Gln Lys Lys Phe His Val 711
Pro Arg Gln Asn Val Pro Val Ile Asn 720
Ile Thr Tyr Asp Ser Thr Pro Glu Asp 729
Val Lys Thr Trp Leu Gln Ser Lys Gly 738
Phe Asn Pro Val Thr Val Asn Ser Leu 747
Gly Val Leu Asn Gly Ala Gln Leu Phe 756
Ser Leu Asn Lys Asp Glu Leu Arg Thr 765
Val Cys Pro Glu Gly Ala Arg Val Tyr 774

Ser Gln Ile Thr Val Gln Lys Ala Ala 783
Leu Glu Asp Ser Ser Gly Ser Ser Glu 792
Leu Gln Glu Ile Met Arg Arg Arg Gln 801
Glu Lys Ile Ser Ala Ala Ala Ser Asp 810
Ser Gly Val Glu Ser Phe Asp Glu Gly 819
Ser Ser His 822

<210> 35
<211> 470
<212> PRT
<213> Mus musculus

<220>
<223> KIAA1247

<400> 35

Met Leu Val Glu Thr Gly Glu Leu Asp 9
Asn Thr Tyr Ile Leu Tyr Thr Ala Asp 18
His Gly Tyr His Ile Gly Gln Phe Gly 27
Leu Val Lys Gly Lys Ser Met Pro Tyr 36
Glu Phe Asp Ile Arg Val Pro Phe Tyr 45
Val Arg Gly Pro Asn Val Glu Ala Gly 54
Ser Leu Asn Pro His Ile Val Leu Asn 63
Ile Asp Leu Gly Pro His His Thr Gly 72
Tyr Arg Trp Thr Gly His Pro Cys Arg 81
His Gly Arg Glu Val Tyr Ser Gln Thr 90
Thr Gly Leu Arg Ala Ala Ser Glu Pro 99
Val Pro Leu Glu Lys Glu Ala Glu Gly 108
Leu Gly Glu Thr Pro Ser Trp Trp Arg 117
Glu Ala Asn Leu Leu His Lys Arg Glu 126
Gly Asp Lys Val Asn Ala Gln Glu Glu 135
Asn Phe Leu Pro Lys Tyr Gln Arg Val 144
Lys Asp Leu Cys Gln Arg Ala Glu Tyr 153
Gln Thr Ala Cys Glu Gln Leu Gly Gln 162
Lys Trp Gln Cys Val Glu Asp Ala Ser 171
Gly Thr Leu Lys Leu His Lys Cys Lys 180
Gly Pro Met Arg Phe Gly Gly Gly Gly 189
Gly Ser Arg Ala Leu Ser Asn Leu Val 198
Pro Lys Tyr Asp Gly Gln Ser Ser Glu 207
Ala Cys Ser Cys Asp Ser Gly Gly Gly 216
Gly Asp Tyr Lys Leu Gly Leu Ala Gly 225
Arg Arg Lys Leu Phe Lys Lys Lys Tyr 234
Lys Thr Ser Tyr Ala Arg Asn Arg Ser 243
Ile Arg Ser Val Ala Ile Glu Val Asp 252
Gly Glu Ile Tyr His Val Gly Leu Asp 261
Thr Val Pro Gln Pro Arg Asn Leu Ser 270
Lys Pro His Trp Pro Gly Ala Pro Glu 279
Asp Gln Asp Asp Lys Asp Gly Gly Ser 288
Phe Ser Gly Thr Gly Gly Leu Pro Asp 297
Tyr Ser Ala Pro Asn Pro Ile Lys Val 306

Thr His Arg Cys Tyr Ile Leu Glu Asn 315
Asp Thr Val Gln Cys Asp Leu Asp Leu 324
Tyr Lys Ser Leu Gln Ala Trp Lys Asp 333
His Lys Leu His Ile Asp His Glu Ile 342
Glu Thr Leu Gln Asn Lys Ile Lys Asn 351
Leu Arg Glu Val Arg Gly His Leu Lys 360
Lys Lys Arg Pro Glu Glu Cys Asp Cys 369
His ??? Ile Ser Tyr His Ser Gln His 378
Lys Gly Arg Leu Lys His Lys Gly Ser 387
Ser Leu His Pro Phe Arg Lys Gly Leu 396
Gln Glu Lys Asp Lys Val Trp Leu Leu 405
Arg Asp Arg Asn Ala Arg Arg Asn Cys 414
Ala Thr Ala Gln Thr Ala Ala Glu Gln 423
Arg Tyr Val Gln His Ala Arg Pro His 432
Val Leu Tyr Pro Arg Gln Pro Pro Leu 441
Ala Asp Gly Ala Thr Leu Asp Ala Gly 450
Ala Val Leu Arg Leu His Gln Arg Gln 459
Gln Gln His Val Leu Val Leu Glu Asp 468
His Lys 470

<210> 36
<211> 690
<212> PRT
<213> Homo sapiens

<220>
<223> KIAA1247

<400> 36

Glu Lys His Gly Ser Asp Tyr Ser Lys 9
Asp Tyr Leu Thr Asp Leu Ile Thr Asn 18
Asp Ser Val Ser Phe Phe Arg Thr Ser 27
Lys Lys Met Tyr Pro His Arg Pro Val 36
Leu Met Val Ile Ser His Ala Ala Pro 45
His Gly Pro Glu Asp Ser Ala Pro Gln 54
Tyr Ser Arg Leu Phe Pro Asn Ala Ser 63
Gln His Ile Thr Pro Ser Tyr Asn Tyr 72
Ala Pro Asn Pro Asp Lys His Trp Ile 81
Met Arg Tyr Thr Gly Pro Met Lys Pro 90
Ile His Met Glu Phe Thr Asn Met Leu 99
Gln Arg Lys Arg Leu Gln Thr Leu Met 108
Ser Val Asp Asp Ser Met Glu Thr Ile 117
Tyr Asn Met Leu Val Glu Thr Gly Glu 126
Leu Asp Asn Thr Tyr Ile Val Tyr Thr 135
Ala Asp His Gly Tyr His Ile Gly Gln 144
Phe Gly Leu Val Lys Gly Lys Ser Met 153
Pro Tyr Glu Phe Asp Ile Arg Val Pro 162
Phe Tyr Val Arg Gly Pro Asn Val Glu 171
Ala Gly Cys Leu Asn Pro His Ile Val 180

Leu Asn Ile Asp Leu Ala Pro Thr Ile 189
Leu Asp Ile Ala Gly Leu Asp Ile Pro 198
Ala Asp Met Asp Gly Lys Ser Ile Leu 207
Lys Leu Leu Asp Thr Glu Arg Pro Val 216
Asn Arg Phe His Leu Lys Lys Lys Met 225
Arg Val Trp Arg Asp Ser Phe Leu Val 234
Glu Arg Gly Lys Leu Leu His Lys Arg 243
Asp Asn Asp Lys Val Asp Ala Gln Glu 252
Glu Asn Phe Leu Pro Lys Tyr Gln Arg 261
Val Lys Asp Leu Cys Gln Arg Ala Glu 270
Tyr Gln Thr Ala Cys Glu Gln Leu Gly 279
Gln Lys Trp Gln Cys Val Glu Asp Ala 288
Thr Gly Lys Leu Lys Leu His Lys Cys 297
Lys Gly Pro Met Arg Leu Gly Gly Ser 306
Arg Ala Leu Ser Asn Leu Val Pro Lys 315
Tyr Tyr Gly Gln Gly Ser Glu Ala Cys 324
Thr Cys Asp Ser Gly Asp Tyr Lys Leu 333
Ser Leu Ala Gly Arg Arg Lys Lys Leu 342
Phe Lys Lys Lys Tyr Lys Ala Ser Tyr 351
Val Arg Ser Arg Ser Ile Arg Ser Val 360
Ala Ile Glu Val Asp Gly Arg Val Tyr 369
His Val Gly Leu Gly Asp Ala Ala Gln 378
Pro Arg Asn Leu Thr Lys Arg His Trp 387
Pro Gly Ala Pro Glu Asp Gln Asp Asp 396
Lys Asp Gly Gly Asp Phe Ser Gly Thr 405
Gly Gly Leu Pro Asp Tyr Ser Ala Ala 414
Asn Pro Ile Lys Val Thr His Arg Cys 423
Tyr Ile Leu Glu Asn Asp Thr Val Gln 432
Cys Asp Leu Asp Leu Tyr Lys Ser Leu 441
Gln Ala Trp Lys Asp His Lys Leu His 450
Ile Asp His Glu Ile Glu Thr Leu Gln 459
Asn Lys Ile Lys Asn Leu Arg Glu Val 468
Arg Gly His Leu Lys Lys Lys Arg Pro 477
Glu Glu Cys Asp Cys His Lys Ile Ser 486
Tyr His Thr Gln His Lys Gly Arg Leu 495
Lys His Arg Gly Ser Ser Leu His Pro 504
Phe Arg Lys Gly Leu Gln Glu Lys Asp 513
Lys Val Trp Leu Leu Arg Glu Gln Lys 522
Arg Lys Lys Lys Leu Arg Lys Leu Leu 531
Lys Arg Leu Gln Asn Asn Asp Thr Cys 540
Ser Met Pro Gly Leu Thr Cys Phe Thr 549
His Asp Asn Gln His Trp Gln Thr Ala 558
Pro Phe Trp Thr Leu Gly Pro Phe Cys 567
Ala Cys Thr Ser Ala Asn Asn Asn Thr 576
Tyr Trp Cys Met Arg Thr Ile Asn Glu 585
Thr His Asn Phe Leu Phe Cys Glu Phe 594
Ala Thr Gly Phe Leu Glu Tyr Phe Asp 603
Leu Asn Thr Asp Pro Tyr Gln Leu Met 612
Asn Ala Val Asn Thr Leu Asp Arg Asp 621
Val Leu Asn Gln Leu His Val Gln Leu 630
Met Glu Leu Arg Ser Cys Lys Gly Tyr 639
Lys Gln Cys Asn Pro Arg Thr Arg Asn 648

Met Asp Leu Gly Leu Lys Asp Gly Gly 657
Ser Tyr Glu Gln Tyr Arg Gln Phe Gln 666
Arg Arg Lys Trp Pro Glu Met Lys Arg 675
Pro Ser Ser Lys Ser Leu Gly Gln Leu 684
Trp Glu Gly Trp Glu Gly 690

<210> 37
<211> 212
<212> PRT
<213> Mus musculus

<220>
<223> Phospholipase Inhibitor

<400> 37

Met Ile Leu Phe Arg Arg His Arg Thr 9
Phe Leu Leu Ala Phe Thr Leu Leu Cys 18
Thr Leu Leu Gly Leu Gly Cys Pro Leu 27
Thr Cys Glu Val Cys Lys Gly Ser Gly 36
His Thr Cys Ser Gly Lys Met Lys Thr 45
Cys Glu Asp Gly Lys Asp Ala Cys Val 54
Val Leu Val Ser Glu Ser Ser Thr Lys 63
Gly Arg Lys Ser Val Asn Thr Phe Lys 72
Ala Cys Met Lys Tyr Lys Asp Cys Tyr 81
Ser Gly Phe Val Ser Thr Thr Met Thr 90
Pro Ser Asp Tyr Met Val Ser Asn Ala 99
His Cys Cys Gln Ser Asp Gly Cys Asn 108
Ser Gly Ser Val Pro Pro Pro Leu Asn 117
Asn Arg Thr Glu Asn Gly Leu Met Cys 126
Pro Ser Cys Ile Ala Pro Phe Gln Glu 135
Thr Cys Pro Gly Thr Gln Ala Ala Arg 144
Cys Val Gly Arg Glu Thr His Cys Ile 153
Tyr Phe Ala Gly Asn Val Gln Ala Gly 162
Ile Ile His Thr Lys Phe Ala Thr Arg 171
Gly Cys Ala Thr Glu Ser Ala Cys His 180
Thr Lys Ala Gly Ala Glu Val Pro Ser 189
Ala Phe Tyr Leu Tyr Phe Leu Arg Arg 198
Ala Asp Cys Leu Pro Ala Pro Tyr Pro 207
Pro Gly Arg Gly Glu 212

<210> 38
<211> 204
<212> PRT
<213> Homo sapiens

<220>
<223> Phospholipase Inhibitor

<400> 38

Met Arg Leu Ser Arg Arg Pro Glu Thr 9
Phe Leu Leu Ala Phe Val Leu Leu Cys 18
Thr Leu Leu Gly Leu Gly Cys Pro Leu 27
His Cys Glu Ile Cys Thr Ala Ala Gly 36
Ser Arg Cys His Gly Gln Met Lys Thr 45
Cys Ser Ser Asp Lys Asp Thr Cys Val 54
Leu Leu Val Gly Lys Ala Thr Ser Lys 63
Gly Lys Glu Leu Val His Thr Tyr Lys 72
Gly Cys Ile Arg Ser Gln Asp Cys Tyr 81
Ser Gly Val Ile Ser Thr Thr Met Gly 90
Pro Lys Asp His Met Val Thr Ser Ser 99
Phe Cys Cys Gln Ser Asp Gly Cys Asn 108
Ser Ala Phe Leu Ser Val Pro Leu Thr 117
Asn Leu Thr Glu Asn Gly Leu Met Cys 126
Pro Ala Cys Thr Ala Ser Phe Arg Asp 135
Lys Cys Met Gly Pro Met Thr His Cys 144
Thr Gly Lys Glu Asn His Cys Val Ser 153
Leu Ser Gly His Val Gln Ala Gly Ile 162
Phe Lys Pro Arg Phe Ala Met Arg Gly 171
Cys Ala Thr Glu Ser Met Cys Phe Thr 180
Lys Pro Gly Ala Glu Val Pro Thr Gly 189
Thr Asn Val Leu Phe Leu His His Ile 198
Glu Cys Thr His Ser Pro 204

<210> 39
<211> 331
<212> PRT
<213> Mus musculus

<220>
<223> EAT/MCL-1

<400> 39

Met Phe Gly Leu Arg Arg Asn Ala Val 9
Ile Gly Leu Asn Leu Tyr Cys Gly Gly 18
Ala Ser Leu Gly Ala Gly Gly Gly Ser 27
Pro Ala Gly Ala Arg Leu Val Ala Glu 36
Glu Ala Lys Ala Arg Arg Glu Gly Gly 45
Gly Glu Ala Ala Leu Leu Pro Gly Ala 54
Arg Val Val Ala Arg Pro Pro Pro Val 63
Gly Ala Glu Asp Pro Asp Val Thr Ala 72
Ser Ala Glu Arg Arg Leu His Lys Ser 81
Pro Gly Leu Leu Ala Val Pro Pro Glu 90
Glu Met Ala Ala Ser Ala Ala Ala Ala 99
Ile Val Ser Pro Glu Glu Glu Leu Asp 108
Gly Cys Glu Pro Glu Ala Ile Gly Lys 117
Arg Pro Ala Val Leu Pro Leu Leu Glu 126

Arg Val Ser Glu Ala Ala Lys Ser Ser 135
Gly Ala Asp Gly Ser Leu Pro Ser Thr 144
Pro Pro Pro Pro Glu Glu Glu Glu Asp 153
Asp Leu Tyr Arg Gln Ser Leu Glu Ile 162
Ile Ser Arg Tyr Leu Arg Glu Gln Ala 171
Thr Gly Ser Lys Asp Ser Lys Pro Leu 180
Gly Glu Ala Gly Ala Ala Gly Arg Arg 189
Ala Leu Glu Thr Leu Arg Arg Val Gly 198
Asp Gly Val Gln Arg Asn His Glu Thr 207
Ala Phe Gln Gly Met Leu Arg Lys Leu 216
Asp Ile Lys Asn Glu Gly Asp Val Lys 225
Ser Phe Ser Arg Val Met Val His Val 234
Phe Lys Asp Gly Val Thr Asn Trp Gly 243
Arg Ile Val Thr Leu Ile Ser Phe Gly 252
Ala Phe Val Ala Lys His Leu Lys Ser 261
Val Asn Gln Glu Ser Phe Ile Glu Pro 270
Leu Ala Glu Thr Ile Thr Asp Val Leu 279
Val Arg Thr Lys Arg Asp Trp Leu Val 288
Lys Gln Arg Gly Trp Asp Gly Phe Val 297
Glu Phe Phe His Val Gln Asp Leu Glu 306
Gly Gly Ile Arg Asn Val Leu Leu Ala 315
Phe Ala Gly Val Ala Gly Val Gly Ala 324
Gly Leu Ala Tyr Leu Ile Arg 331

<210> 40
<211> 350
<212> PRT
<213> Homo sapiens

<220>
<223> EAT/MCL-1

<400> 40

<210> 41
<211> 143
<212> PRT
<213> Mus musculus

<220>
<223> TSC-22

<400> 41

Met Lys Ser Gln Trp Cys Arg Pro Val 9
Ala Met Asp Leu Gly Val Tyr Gln Leu 18
Arg His Phe Ser Ile Ser Phe Leu Ser 27
Ser Leu Leu Gly Thr Glu Asn Ala Ser 36
Val Arg Leu Asp Asn Ser Ser Gly Ala 45
Ser Val Val Ala Ile Asp Asn Lys Ile 54
Glu Gln Ala Met Asp Leu Val Lys Ser 63
His Leu Met Tyr Ala Val Arg Glu Glu 72
Val Glu Val Leu Lys Glu Gln Ile Lys 81
Glu Leu Ile Glu Lys Asn Ser Gln Leu 90
Glu Gln Glu Asn Asn Leu Leu Lys Thr 99
Leu Ala Ser Pro Glu Gln Leu Ala Gln 108
Phe Gln Ala Gln Leu Gln Thr Gly Ser 117
Pro Pro Ala Thr Thr Gln Pro Gln Gly 126
Thr Thr Gln Pro Pro Ala Gln Pro Ala 135

Ser Gln Gly Ser Gly Ser Thr Ala 143

<210> 42
<211> 144
<212> PRT
<213> Homo sapiens

<220>
<223> TSC-22

<400> 42

Met Lys Ser Gln Trp Cys Arg Pro Val 9
Ala Met Asp Leu Gly Val Tyr Gln Leu 18
Arg His Phe Ser Ile Ser Phe Leu Ser 27
Ser Leu Leu Gly Thr Glu Asn Ala Ser 36
Val Arg Leu Asp Asn Ser Ser Ser Gly 45
Ala Ser Val Val Ala Ile Asp Asn Lys 54
Ile Glu Gln Ala Met Asp Leu Val Lys 63
Ser His Leu Met Tyr Ala Val Arg Glu 72
Glu Val Glu Val Leu Lys Glu Gln Ile 81
Lys Glu Leu Ile Glu Lys Asn Ser Gln 90
Leu Glu Gln Glu Asn Asn Leu Leu Lys 99
Thr Leu Ala Ser Pro Glu Gln Leu Ala 108
Gln Phe Gln Ala Gln Leu Gln Thr Gly 117
Ser Pro Pro Ala Thr Thr Gln Pro Gln 126
Gly Thr Thr Gln Pro Pro Ala Gln Pro 135
Ala Ser Gln Gly Ser Gly Pro Thr Ala 144

<210> 43
<211> 291
<212> PRT
<213> Mus musculus

<220>
<223> gamma-Sarcoglycan

<400> 43

Met Val Arg Glu Gln Tyr Thr Thr Val 9
Thr Glu Gly Thr His Ile Glu Arg Pro 18
Glu Asn Gln His Ile Tyr Lys Ile Gly 27
Ile Tyr Gly Trp Arg Lys Arg Cys Leu 36
Tyr Leu Phe Val Leu Leu Leu Leu Ala 45
Ile Leu Val Val Asn Leu Ala Leu Thr 54
Ile Trp Ile Leu Lys Val Met Trp Phe 63
Ser Pro Ile Gly Met Gly His Leu His 72
Val Thr Ala Asp Gly Leu Arg Leu Glu 81

Gly Glu Ser Glu Phe Leu Phe Pro Leu 90
Tyr Ala Lys Glu Ile Arg Ser Arg Val 99
Asp Ser Ser Leu Leu Leu Gln Ser Thr 108
Gln Asn Val Thr Val Ser Ala Arg Asn 117
Ser Glu Gly Glu Val Thr Gly Arg Val 126
Lys Val Gly Ala Gln Met Val Glu Val 135
Gln Ser Gln His Phe Gln Ile Asn Ser 144
Glu Asp Gly Lys Pro Leu Phe Ser Ala 153
Glu Glu Gln Asp Val Val Val Gly Thr 162
Gly Arg Leu Arg Val Thr Gly Pro Glu 171
Gly Ala Leu Phe Glu His Ser Val Glu 180
Thr Pro Leu Val Arg Ala Asp Pro Phe 189
Gln Asp Leu Arg Leu Glu Ser Pro Thr 198
Arg Ser Leu Ser Met Asp Ala Pro Arg 207
Gly Val His Val Lys Ala Asn Ala Gly 216
Lys Leu Glu Ala Leu Ser Gln Met Asp 225
Ile Ile Leu Gln Ser Ser Glu Gly Val 234
Leu Val Leu Asp Ala Glu Thr Val Gly 243
Leu Thr Lys Leu Lys Gln Gly Thr Gln 252
Gly Pro Ala Gly Ser Ser Asn Gly Phe 261
Tyr Glu Ile Cys Ala Cys Pro Asp Gly 270
Lys Leu Tyr Leu Ser Met Ala Gly Glu 279
Val Thr Thr Cys Glu Glu His Ser His 288
Val Cys Leu 291

<210> 44
<211> 291
<212> PRT
<213> Homo sapiens

<220>
<223> gamma-Sarcoglycan

<400> 44

Met Val Arg Glu Gln Tyr Thr Thr Ala 9
Thr Glu Gly Ile Cys Ile Glu Arg Pro 18
Glu Asn Gln Tyr Val Tyr Lys Ile Gly 27
Ile Tyr Gly Trp Arg Lys Arg Cys Leu 36
Tyr Leu Phe Val Leu Leu Leu Leu Ile 45
Ile Leu Val Val Asn Leu Ala Leu Thr 54
Ile Trp Ile Leu Lys Val Met Trp Phe 63
Ser Pro Ala Gly Met Gly His Leu Cys 72
Val Thr Lys Asp Gly Leu Arg Leu Glu 81
Gly Glu Ser Glu Phe Leu Phe Pro Leu 90
Tyr Ala Lys Glu Ile His Ser Arg Val 99
Asp Ser Ser Leu Leu Leu Gln Ser Thr 108
Gln Asn Val Thr Val Asn Ala Arg Asn 117
Ser Glu Gly Glu Val Thr Gly Arg Leu 126
Lys Val Gly Pro Lys Met Val Glu Val 135

Gln Asn Gln Gln Phe Gln Ile Asn Ser 144
Asn Asp Gly Lys Pro Leu Phe Thr Val 153
Asp Glu Lys Glu Val Val Val Gly Thr 162
Asp Lys Leu Arg Val Thr Gly Pro Glu 171
Gly Ala Leu Phe Glu His Ser Val Glu 180
Thr Pro Leu Val Arg Ala Asp Pro Phe 189
Gln Asp Leu Arg Leu Glu Ser Pro Thr 198
Arg Ser Leu Ser Met Asp Ala Pro Arg 207
Gly Val His Ile Gln Ala His Ala Gly 216
Lys Ile Glu Ala Leu Ser Gln Met Asp 225
Ile Leu Phe His Ser Ser Asp Gly Met 234
Leu Val Leu Asp Ala Glu Thr Val Cys 243
Leu Pro Lys Leu Val Gln Gly Thr Trp 252
Gly Pro Ser Gly Ser Ser Gln Ser Leu 261
Tyr Glu Ile Cys Val Cys Pro Asp Gly 270
Lys Leu Tyr Leu Ser Val Ala Gly Val 279
Ser Thr Thr Cys Gln Glu His Ser His 288
Ile Cys Leu 291

<210> 45
<211> 228
<212> PRT
<213> Homo sapiens

<220>
<223> Phospholipase Inhibitor

<400> 45

Met Trp Val Gln Thr Arg Pro Ser Ser 9
Ala Ser Tyr Lys Ser Trp Gly Pro Gly 18
Thr Ala Asp Thr His Thr Met Arg Leu 27
Ser Arg Arg Pro Glu Thr Phe Leu Leu 36
Ala Phe Val Leu Leu Cys Thr Leu Leu 45
Gly Leu Gly Cys Pro Leu His Cys Glu 54
Ile Cys Thr Ala Ala Gly Ser Arg Cys 63
His Gly Gln Met Lys Thr Cys Ser Ser 72
Asp Lys Asp Thr Cys Val Leu Leu Val 81
Gly Lys Ala Thr Ser Lys Gly Lys Glu 90
Leu Val His Thr Tyr Lys Gly Cys Ile 99
Arg Ser Gln Asp Cys Tyr Ser Gly Val 108
Ile Ser Thr Thr Met Gly Pro Lys Asp 117
His Met Val Thr Ser Ser Phe Cys Cys 126
Gln Ser Asp Gly Cys Asn Ser Ala Phe 135
Leu Ser Val Pro Leu Thr Asn Leu Thr 144
Glu Asn Gly Leu Met Cys Pro Ala Cys 153
Thr Ala Ser Phe Arg Asp Lys Cys Met 162
Gly Pro Met Thr His Cys Thr Gly Lys 171
Glu Asn His Cys Val Ser Leu Ser Gly 180
His Val Gln Ala Gly Ile Phe Lys Pro 189

Arg Phe Ala Met Arg Gly Cys Ala Thr 198
Glu Ser Met Cys Phe Thr Lys Pro Gly 207
Ala Glu Val Pro Thr Gly Thr Asn Val 216
Leu Phe Leu His His Ile Glu Cys Thr 225
His Ser Pro 228

<210> 46
<211> 140
<212> PRT
<213> Mus musculus

<220>
<223> Cystatin C

<400> 46

Met Ala Ser Pro Leu Arg Ser Leu Leu 9
Phe Leu Leu Ala Val Leu Ala Val Ala 18
Trp Ala Ala Thr Pro Lys Gln Gly Pro 27
Arg Met Leu Gly Ala Pro Glu Glu Ala 36
Asp Ala Asn Glu Glu Gly Val Arg Arg 45
Ala Leu Asp Phe Ala Val Ser Glu Tyr 54
Asn Lys Gly Ser Asn Asp Ala Tyr His 63
Ser Arg Ala Ile Gln Val Val Arg Ala 72
Arg Lys Gln Leu Val Ala Gly Val Asn 81
Tyr Phe Leu Asp Val Glu Met Gly Arg 90
Thr Thr Cys Thr Lys Ser Gln Thr Asn 99
Leu Thr Asp Cys Pro Phe His Asp Gln 108
Pro His Leu Met Arg Lys Ala Leu Cys 117
Ser Phe Gln Ile Tyr Ser Val Pro Trp 126
Lys Gly Thr His Ser Leu Thr Lys Phe 135
Ser Cys Lys Asn Ala 140

<210> 47
<211> 146
<212> PRT
<213> Homo sapiens

<220>
<223> Cystatin C

<400> 47

Met Ala Gly Pro Leu Arg Ala Pro Leu 9
Leu Leu Leu Ala Ile Leu Ala Val Ala 18
Leu Ala Val Ser Pro Ala Ala Gly Ser 27
Ser Pro Gly Lys Pro Pro Arg Leu Val 36
Gly Gly Pro Met Asp Ala Ser Val Glu 45

Glu    Glu    Gly    Val    Arg    Arg    Ala    Leu    Asp    .54
Phe    Ala    Val    Gly    Glu    Tyr    Asn    Lys    Ala    63
Ser    Asn    Asp    Met    Tyr    His    Ser    Arg    Ala    72
Leu    Gln    Val    Val    Arg    Ala    Arg    Lys    Gln    81
Ile    Val    Ala    Gly    Val    Asn    Tyr    Phe    Leu    90
Asp    Val    Glu    Leu    Gly    Arg    Thr    Thr    Cys    99
Thr    Lys    Thr    Gln    Pro    Asn    Leu    Asp    Asn    108
Cys    Pro    Phe    His    Asp    Gln    Pro    His    Leu    117
Lys    Arg    Lys    Ala    Phe    Cys    Ser    Phe    Gln    126
Ile    Tyr    Ala    Val    Pro    Trp    Gln    Gly    Thr    135
Met    Thr    Leu    Ser    Lys    Ser    Thr    Cys    Gln    144
Asp    Ala    146

<210> 48
<211> 140
<212> PRT
<213> Mus musculus
<220>
<223> Cystatin C Variante

<400> 48

Met    Ala    Ser    Pro    Leu    Arg    Ser    Leu    Leu    9
Phe    Leu    Leu    Ala    Val    Leu    Ala    Val    Ala    18
Trp    Ala    Ala    Thr    Pro    Lys    Gln    Gly    Pro    27
Arg    Met    Leu    Gly    Ala    Pro    Glu    Glu    Ala    36
Asp    Ala    Asn    Lys    Glu    Gly    Val    Arg    Arg    45
Ala    Leu    Asp    Phe    Ala    Val    Ser    Glu    Tyr    54
Asn    Lys    Gly    Ser    Asn    Asp    Ala    Tyr    His    63
Ser    Arg    Ala    Ile    Gln    Val    Val    Arg    Ala    72
Arg    Lys    Gln    Leu    Val    Ala    Gly    Val    Asn    81
Tyr    Phe    Leu    Asp    Val    Glu    Met    Gly    Arg    90
Thr    Thr    Cys    Thr    Lys    Ser    Gln    Thr    Asn    99
Leu    Thr    Asp    Cys    Pro    Phe    His    Asp    Gln    108
Pro    His    Leu    Met    Arg    Lys    Ala    Leu    Cys    117
Ser    Phe    Gln    Ile    Tyr    Ser    Val    Pro    Trp    126
Lys    Gly    Thr    His    Ser    Leu    Thr    Lys    Phe    135
Ser    Cys    Lys    Asn    Ala    140

<210> 49

<400>

<210> 50
<211> 2231
<212> DNA

<213> Mus musculus

<220>
<223> SW1136

<400> 50

cggcgaggaa gatggcggac gggaaggcgg gagaggagaa gccagagaag 50
ccgcagcgag ccggagccgc cggaggacct gaagaagaag cagaaaaacc 100
tgtgaaaact aagactgttt cttccagtaa tggaggggaa agttccagtc 150
gcagcgctga aaagcgatca gctgaagacg aagctgcaga cctcccaaca 200
aagcctacaa agatgtccaa gtttggattt gccataggta gtcagacggc 250
acggaaagcg tccgccatct ccattagact cggagcaagt aagcctaagg 300
aaacagttcc aactcttgct ccaaaaactc tgtcggtagc agcagctttc 350
aatgaagatg aagatagtga gccagaagaa atgcctccag aagcaaagat 400
gaggatgaag aacattggaa gggacacacc aacatcagcg ggaccaaact 450
cctttaataa agggaagcat ggcttttccg ataaccagaa gctgtgggag 500
cgaaatataa aatctcatct tggaaacgtc cacgaccaag acaattaagt 550
gaagtgtctg agactggggt gtgggtgggt gcagttagaa ggagcagtct 600
cctttttgta aagaatggtg taagactagc tttggagccg tttttctttc 650
ttcttttttt cttttccttt tttctttttt tttttttttt ttaagattga 700
gtggtacact aataaatgag agtttgcaat tagaggtaat ttatgtttta 750
tatacagatt tcaagacatt tgctaatttt gtagtttcac atgattagtt 800
tccaagggtt acagataata aagaaatcac aagtggtacc tttctaagaa 850
ttgcatattt ttttagacac aactattagc acattaagag ggaagccaaa 900
agttattgtc tgcttcaaac tggaagcagt tcctctcctg acttctccct 950
cgttacccga ctgtccggct ccctgcagca gccttaccga gagggagatt 1000
ggcttgagaa gccagtgtta ctgttgtgac tgttggctga gaggaagttt 1050
agatgaggtt caagtaaacc ctttcctgcg ggcatttcgt tttgtttcgg 1100
gccattctag ctagtactgc ttcgctctca gtgggtaccg atgcttgctc 1150
tgtaaaaata aatttttttc gttaaaaatt cttatatgaa gaattgagta 1200
ttatgactag cccactctaa cggagtgtgt ctccgttgag gaccttcaga 1250
ggacactatt tgctaccaaa gtgaaccagt attctgaatg tgcttctctt 1300
ggtttctgtt ctagttccta gaagcatttc caccagaact tgaggcaaaa 1350
cataaggaag ctgtttcttt taaagtacaa acaccaccaa aaatatcagt 1400
gtacatagtg ctttatgtat ttggctggct tttatttttt aaaaggttta 1450
aataacaaaa aaggaaaaaa aggtatagca ttgtatgaag gcagatgagg 1500
cgacacacct gtgacatttg aagcatttgt gttactaact tactagatcc 1550
ggtttgaatt gaattcacaa ggtttcttac ttgtccaact ccaggcttat 1600
tttacgtaca tacaacctga ccagtggttt caaaagcaca tttagtgact 1650
gcaataggac agaaatgctt ctctagacac atcttcattg cgagcgtctc 1700
atcgcatatg ctcttagagt tctgaatgta ggatgtttgt tttgtatttt 1750
tgtattgtat atgggctttt taaatgtgac agttaaatgc atctttaata 1800
gtcacagaca aaagagatga taaaatgtcc tttgaaaatg gcaatgttga 1850
gtttggaggc agcttcgggg agtttcctgg gtggcgatga ttcactgggc 1900
agtttttaat gtagtgactt caagaagcag cctgcggata ttcctaacac 1950
ttggttcact acagtttagt ttacttcata agccccaagt aaaacgatgg 2000
aaatgtacat agcactatta gttcatacat gatttaagta tatcaagata 2050
cataaattta acatttatgt ctgcaaactg tccatttgtc cattttactg 2100
tttgttgaaa taacacctct cctacatatt ctttacttga ctcgaataac 2150
aaattaacct gacgtcaaga tggagagaga cgactgagct gaatgtcttt 2200
actaaaatta caataaattt tgtcaaactc g 2231

<210> 51
<211> 1066
<212> DNA
<213> Homo sapiens

<220>
<223> SW1136

<400> 51

ggggaggccg cggcggggaa aatggcggac gggaaggcgg gagacgagaa 50
gcctgaaaag tcgcagcgag ctggagccgc cggaggacct gaagaagaag 100
cagaaaaacc tgtgaaaact aagactgttt cttccagtaa tggaggggaa 150
agttccagtc gcagcgctga gaagcgatca gctgaagaag aagctgccga 200
cctcccaaca aagcctacaa agatctccaa gtttggattt gccataggta 250
gtcagacgac aaagaaagca tcagccatat ccatcaaact tggatcaagt 300
aagcctaaag aaactgttcc aactcttgct ccaaaaactc tttcagtagc 350
agcagctttt aatgaagatg aagatagtga accagaggaa atgcctccag 400
aagcaaagat gaggatgaag aatattggaa gggatacacc aacatcagct 450
ggaccaaact ccttcaataa aggaaagcat gggttttctg ataaccagaa 500
gctgtgggag cgaaatataa aatctcatct tggaaatgtc catgaccaag 550
acaattaaat gatgttttga aattggggtg tggggtgggt gtaaagttaa 600
aaggaacagt ttcctttttt aaagaatggt ataagactat ctttggagcc 650
gctttttttt tctttttcat ttttttaaaa gattgagtgg tacactaata 700
aatgagagtt tgaaattaga ggtaatttat gttttatata cagatttcaa 750
gacatttgct aattttgtag tttcatgtga ttagtttcca aaggttacag 800
ataataaaga aatcagaaat ggtaccmatt ttaaraattg cycatttttt 850
ttttagagcc camctattag ccccmttaag akggdgyaaa rchaawgtat 900
aaramtrcaa gmatgwtarc ctstctkars tcccttatta cctaaacttg 950
tctggctccc aggaacagcc ttatagagag agggagtatt gtattgggaa 1000
gaaaatgtta ctgaactatt gactgaaagt aaatttagat aaaatacaaa 1050
aaaaaaaaaa aaaaaa 1066


<210> 52
<211> 773
<212> DNA
<213> Mus musculus

<220>
<223> SW1295

<400> 52

attcggatcc ttgtagaaat tgctggacgc ttctgtacgt ttcgcagttt 50
gtcttcgctg atcggtgaga cttcgagcag ttaggatgcc gcgtggaagc 100
cgaagccgca cttcccgggt gactcctccg gccagccggg cccctcagat 150
gagggctgct ccccgaagag cacctgcagc tcagcctcca gcagcagctg 200
cgccatctgc agttggctca cctgccgctg cgccccggca gccaggcctg 250
atggcccaga tggctaccac cgcggccggt gtggctgtgg gctctgcagt 300
gggacacacc ctgggtcacg ccatcactgg gggcttcagc ggaggtggca 350
gtgctgagcc cgcaaagccc gacatcactt accaggagcc tcagggagcc 400
cagctgcaga accagcagtc ttttggacct tgctctctag agatcaagca 450
gtttctggag tgtgctcaga accagagcga tgtcaagctc tgtgagggct 500
tcaacgaggt gctgcggcag tgcaggattg caaatggttt aatgtaatca 550
agaaattcaa gctgaagaga tgtaacattg gttctgtata attgatagta 600
caagtgtgga cccttatatt tctaacagtt cattgctttg gaatggccgt 650
gaaagagatc taactgtgga tgcttactga ggcttgtatg tagtgttggt 700
tactggggaa gtgtttggcc tccttggact gtgttgtgat tgtgagttaa 750
aaaaaataaa ttggttattc tga 773


<210> 53

<211> 3057
<212> DNA
<213> Mus musculus

<220>
<223> KIAA1247

<400> 53

aatgctggtg gagacggggg agctggacaa cacgtacatc ctgtacaccg 50
ccgaccacgg ctaccacatt ggccagtttg ggctggtgaa gggcaagtct 100
atgccgtatg aattcgacat cagagtcccg ttctacgtga ggggccccaa 150
cgtggaagct ggctctctga acccccacat tgtcctcaac attgacctgg 200
gcccccacca tactggatat cgctggactg gacatccctg cagacatgga 250
cgggaagtct attctcaaac tactggactc agagcggcca gtgaaccggt 300
tccacttgaa aaagaagctg agggtcttgg cgagactcct tcctggtgga 350
gagaggcaaa cttgctccac aagagggagg gtgacaaagt gaatgcccag 400
gaggagaact tcctgcccaa gtaccagcgc gtgaaggacc tgtgtcagcg 450
agctgagtac cagacagcat gcgaacagct ggggcagaag tggcagtgtg 500
tggaggacgc ttctgggacg ctgaagctgc acaaatgtaa aggccccatg 550
cggtttggtg gcggcggtgg cagcagagcc ctctccaacc tggtgcccaa 600
gtatgacggc cagagcagcg aggcctgcag ctgtgacagt ggcggtggag 650
gggactacaa actgggcctg gctggacgcc gtaagctctt taagaaaaag 700
tataagacca gctatgcccg gaaccgctcc atccgttccg tggccatcga 750
ggtggacggt gagatatacc acgtaggctt ggatactgtg cctcagcccc 800
gcaaccttag caagccgcac tggccagggg cccctgaaga ccaagatgac 850
aaggatggtg gcagtttcag tggtactggt ggccttccag attattctgc 900
ccccaatccc atcaaagtga cccatcggtg ctacatcctt gagaatgaca 950
cagtccagtg cgacttggac ctgtacaagt ccctgcaggc ttggaaagac 1000
cacaagctgc acatcgacca tgagatcgaa accctgcaga acaaaattaa 1050
gaaccttcga gaagtcaggg gtcacctgaa gaagaagcga ccggaagaat 1100
gtgactgcca taraatcagt taccacagcc aacacaaagg ccgtctcaag 1150
cacaaaggct ccagcctgca ccctttcagg aagggtctgc aggagaagga 1200
caaggtgtgg ctgctgcggg acagaaacgc aagaagaaac tgcgcaactg 1250
ctcaaacggc tgcagaacaa cgatacgtgc agcatgcccg gcctcacgtg 1300
ctttacccac gacaaccacc actggcagac ggcgccactc tggacgctgg 1350
ggccgttctg cgcctgcacc agcgccaaca acaacacgta ctggtgcttg 1400
aggaccataa atgagaccca caacttcctc ttctgcgaat ttgcaaccgg 1450
cttcatagaa tactttgacc tcagtacaga cccctaccag ctgatgaacg 1500
cggtgaacac actggacagg gacgtcctta accaactgca cgtgcagctc 1550
atggagctaa ggagctgtaa aggctacaag cagtgcaacc cccggacccg 1600
caacatggac ctggggctta gagacggagg aagctatgaa caatacaggc 1650
agtttcagcg tcgaaaatgg ccagaaatga agagaccttc ttccaaatca 1700
ctgggacagc tatgggaagg ttgggaaggc taagcggcca tagagagagg 1750
aacctccaaa accaggggcc tcgtgtggct gcccaggcca tgcaaaaaac 1800
acccgattcc cagaagatga atgttggaac tgggagacct gacagaaggc 1850
agggctgctc ttgggacagg aaatcctgga ggacagcgcc tggactttcc 1900
gatgctcagt ttctttgccc tgctttgctc tggatcaaac ctcactggct 1950
gctctgggat gcgtgctcac acctggagtc tctgctcacc ctttcagagg 2000
ctcacaaaga caaaggaact aatttccatg gacacttcct ccagagatgg 2050
aaattgctgg gattcgccca ctcctcccct gcacccctcc cccagtcatc 2100
tagggaagca agcttgtttt aaccttctta ctctttggag aaagcacgga 2150
catcccaggt gctgtcaacc tcacagtctt gacaaagtct atagcacaaa 2200
acagtaccat tcaccaggct ggttgacctg gctggctcag aagctgcctt 2250
caccacatac atgaccgctc acacgtaacc aacacaggga attgtagggg 2300
aatctcacta atatgaaatc ccgcttttca agagtcgcgg tgtcaataaa 2350
cgctgtggct aggatcaagg ataatccctt gagctttcag acatttattc 2400
ctgcccggga ttcgttcctt tgttatccat cccagaactg atgtttttct 2450
aaggtaccga aaccccaagt tgatgtgtgt cctgtgtttt aatgacattg 2500
tatttgtaaa gttttgtagt ataagtacca tcttacagtg ttcctgcccc 2550
cagccaatgt ctagctattg gtatgaaaaa aaaaatcttt gaatttttgt 2600

aaaaggcwaw mwwrrramww wwwwgctctt tattattatc atttttttct 2650
tgaagcactc aagatgttgt cgaccttcag ctgagggttg gagaagcaga 2700
gggtaataca ttaatagtaa acctctccga ttcaagggac ctcataggta 2750
agtctcacca gctttggggc atcgatctgt gaagcaaagc atccaactgc 2800
accaaccctt tgttcatcca ggtagggtaa cctggtcaat atggctgcca 2850
gaaagcagct gtcagagctt cggaggtggg ggtggaaatg tcagcccaac 2900
ccgactcagc agagggggct cacatttctc taaaccagca gtttttcagc 2950
tggggctcca acgacctttc acaggggtcc taaggtcatc agagagcaca 3000
tttaatggta cagttcacac agtaacaatc acagctaaga agtagcacag 3050
tagcagc 3057

<210> 54
<211> 732
<212> cDNA
<213> Mus musculus

<220>
<223> Phospholipase Inhibitor

<400> 54

ggactacagg ttcacacacc atgattctgt ttagaagaca caggaccttc 50
ctgctggcct tcacactgct ctgtaccctc ctgggtctcg ggtgccctct 100
aacttgtgag gtgtgcaaag gctcggggca cacatgcagc gggaagatga 150
agacctgtga agacggcaaa gacgcatgcg tggtcctagt gagcgagtcc 200
agcacaaagg gccgaaagtc agtgaacacc ttcaaggcct gcatgaagta 250
caaagactgc tactcaggct tcgtatcaac caccatgact cccagtgact 300
acatggtgtc caacgcccac tgctgtcaaa gtgacggttg caacagtggc 350
tctgtgcccc ctcccttgaa caatcggaca gagaatggcc tgatgtgtcc 400
ctcctgcatt gcgcccttcc aggagacctg tccaggaacc caggcagctc 450
gctgcgtggg ccgggaaaca cactgcatct attttgctgg caatgtgcag 500
gctggtatta tccacacgaa atttgccacg aggggctgtg ctacagagag 550
cgcctgccac accaaggcag gggctgaggt cccttcagcc ttctatctct 600
acttcctgcg ccgggcagac tgccttccag ccccttaccc tcctggcagg 650
ggagagtgaa gaaaccgaga agtatgtaac ttgaggagcc ctgcagcttt 700
ccagctgcct gtaaattaaa cagatygaca gt 732

<210> 55
<211> 178
<212> PRT
<213> Mus musculus

<220>
<223> SW1136

<400> 55

Met Ala Asp Gly Lys Ala Gly Glu Glu 9
Lys Pro Glu Lys Pro Gln Arg Ala Gly 18
Ala Ala Gly Gly Pro Glu Glu Glu Ala 27
Glu Lys Pro Val Lys Thr Lys Thr Val 36
Ser Ser Ser Asn Gly Gly Glu Ser Ser 45
Ser Arg Ser Ala Glu Lys Arg Ser Ala 54
Glu Asp Glu Ala Ala Asp Leu Pro Thr 63

Lys Pro Thr Lys Met Ser Lys Phe Gly 72
Phe Ala Ile Gly Ser Gln Thr Ala Arg 81
Lys Ala Ser Ala Ile Ser Ile Arg Leu 90
Gly Ala Ser Lys Pro Lys Glu Thr Val 99
Pro Thr Leu Ala Pro Lys Thr Leu Ser 108
Val Ala Ala Ala Phe Asn Glu Asp Glu 117
Asp Ser Glu Pro Glu Glu Met Pro Pro 126
Glu Ala Lys Met Arg Met Lys Asn Ile 135
Gly Arg Asp Thr Pro Thr Ser Ala Gly 144
Pro Asn Ser Phe Asn Lys Gly Lys His 153
Gly Phe Ser Asp Asn Gln Lys Leu Trp 162
Glu Arg Asn Ile Lys Ser His Leu Gly 171
Asn Val His Asp Gln Asp Asn 178

<210> 56
<211> 178
<212> PRT
<213> Homo sapiens

<220>
<223> SW1136

<400> 56

Met Ala Asp Gly Lys Ala Gly Asp Glu 9
Lys Pro Glu Lys Ser Gln Arg Ala Gly 18
Ala Ala Gly Gly Pro Glu Glu Glu Ala 27
Glu Lys Pro Val Lys Thr Lys Thr Val 36
Ser Ser Ser Asn Gly Gly Glu Ser Ser 45
Ser Arg Ser Ala Glu Lys Arg Ser Ala 54
Glu Glu Glu Ala Ala Asp Leu Pro Thr 63
Lys Pro Thr Lys Ile Ser Lys Phe Gly 72
Phe Ala Ile Gly Ser Gln Thr Thr Lys 81
Lys Ala Ser Ala Ile Ser Ile Lys Leu 90
Gly Ser Ser Lys Pro Lys Glu Thr Val 99
Pro Thr Leu Ala Pro Lys Thr Leu Ser 108
Val Ala Ala Ala Phe Asn Glu Asp Glu 117
Asp Ser Glu Pro Glu Glu Met Pro Pro 126
Glu Ala Lys Met Arg Met Lys Asn Ile 135
Gly Arg Asp Thr Pro Thr Ser Ala Gly 144
Pro Asn Ser Phe Asn Lys Gly Lys His 153
Gly Phe Ser Asp Asn Gln Lys Leu Trp 162
Glu Arg Asn Ile Lys Ser His Leu Gly 171
Asn Val His Asp Gln Asp Asn 178

<210> 57
<211> 153
<212> PRT

<213> Mus musculus

<220>
<223> SW1295

<400> 57

Met Pro Arg Gly Ser Arg Ser Arg Thr 9
Ser Arg Val Thr Pro Pro Ala Ser Arg 18
Ala Pro Gln Met Arg Ala Ala Pro Arg 27
Arg Ala Pro Ala Ala Gln Pro Pro Ala 36
Ala Ala Ala Pro Ser Ala Val Gly Ser 45
Pro Ala Ala Ala Pro Arg Gln Pro Gly 54
Leu Met Ala Gln Met Ala Thr Thr Ala 63
Ala Gly Val Ala Val Gly Ser Ala Val 72
Gly His Thr Leu Gly His Ala Ile Thr 81
Gly Gly Phe Ser Gly Gly Gly Ser Ala 90
Glu Pro Ala Lys Pro Asp Ile Thr Tyr 99
Gln Glu Pro Gln Gly Ala Gln Leu Gln 108
Asn Gln Gln Ser Phe Gly Pro Cys Ser 117
Leu Glu Ile Lys Gln Phe Leu Glu Cys 126
Ala Gln Asn Gln Ser Asp Val Lys Leu 135
Cys Glu Gly Phe Asn Glu Val Leu Arg 144
Gln Cys Arg Ile Ala Asn Gly Leu Met 153

<210> 58
<211> 151
<212> PRT
<213> Homo sapiens

<220>
<223> SW1295

<400> 58

Met Pro Arg Gly Ser Arg Ser Arg Thr 9
Ser Arg Met Ala Pro Pro Ala Ser Arg 18
Ala Pro Gln Met Arg Ala Ala Pro Arg 27
Pro Ala Pro Val Ala Gln Pro Pro Ala 36
Ala Ala Pro Pro Ser Ala Val Gly Ser 45
Ser Ala Ala Ala Pro Arg Gln Pro Gly 54
Leu Met Ala Gln Met Ala Thr Thr Ala 63
Ala Gly Val Ala Val Gly Ser Ala Val 72
Gly His Thr Leu Gly His Ala Ile Thr 81
Gly Gly Phe Ser Gly Gly Ser Asn Ala 90
Glu Pro Ala Arg Pro Asp Ile Thr Tyr 99
Gln Glu Pro Gln Gly Thr Gln Pro Ala 108
Gln Gln Gln Gln Pro Cys Leu Tyr Glu 117
Ile Lys Gln Phe Leu Glu Cys Ala Gln 126
Asn Gln Gly Asp Ile Lys Leu Cys Glu 135

Gly Phe Asn Glu Val Leu Lys Gln Cys 144
Arg Leu Ala Asn Gly Leu Ala 151

<210> 59
<211> 19
<212> DNA
<213> Mus musculus

<220>
<223> TTF-1 Primer 1

<400> 59

cgagcgctac attgtcgct 19

<210> 60
<211> 22
<212> DNA
<213> Mus musculus

<220>
<223> TTF-1 Primer 2

<400> 60

gtcttaaatt tgcttgtgcc cc 22

<210> 61
<211> 19
<212> DNA
<213> Mus musculus

<220>
<223> GAPDH-Primer1

<400> 61

atcaacggga agcccatca 19

<210> 62
<211> 20
<212> DNA
<213> Mus musculus

<220>
<223> GAPDH-Primer2

<400> 62

gacatactca gcaccggcct 20

<210> 63
<211> 823
<212> PRT
<213> Mus musculus

<220>
<223> Fer

<400> 63

Met Gly Phe Gly Ser Asp Leu Lys Asn 9
Ser Gln Glu Ala Val Leu Lys Leu Gln 18
Asp Trp Glu Leu Arg Leu Leu Glu Thr 27
Val Lys Lys Phe Met Ala Leu Arg Ile 36
Lys Ser Asp Lys Glu Tyr Ala Tyr Thr 45
Leu Gln Asn Leu Cys Asn Gln Val Asp 54
Lys Glu Ser Thr Val Gln Val Asn Tyr 63
Val Ser Asn Val Ser Lys Ser Trp Leu 72
Leu Met Ile Gln Gln Thr Glu Gln Leu 81
Ser Arg Ile Met Lys Thr His Ala Glu 90
Asp Leu Asn Ser Gly Pro Leu His Arg 99
Leu Thr Met Met Ile Lys Asp Lys Gln 108
Gln Val Lys Lys Ser Tyr Val Gly Ile 117
His Gln Gln Ile Glu Ala Glu Met Ile 126
Lys Val Thr Lys Thr Glu Leu Glu Lys 135
Leu Lys Ser Ser Tyr Arg Gln Leu Ile 144
Lys Glu Met Asn Ser Ala Lys Glu Lys 153
Tyr Lys Glu Ala Leu Ala Lys Gly Lys 162
Glu Thr Glu Lys Ala Lys Glu Arg Tyr 171
Asp Lys Ala Thr Met Lys Leu His Met 180
Leu His Asn Gln Tyr Val Leu Ala Leu 189
Lys Gly Ala Gln Leu His Gln Ser Gln 198
Tyr Tyr Asp Thr Thr Leu Pro Leu Leu 207
Leu Asp Ser Val Gln Lys Met Gln Glu 216
Glu Met Ile Lys Ala Leu Lys Gly Ile 225
Phe Asp Asp Tyr Ser Gln Ile Thr Ser 234
Leu Val Thr Glu Glu Ile Val Asn Val 243
His Lys Glu Ile Gln Met Ser Val Glu 252
Gln Ile Asp Pro Ser Thr Glu Tyr Asn 261
Asn Phe Ile Asp Val His Arg Thr Thr 270
Ala Ala Lys Glu Gln Glu Ile Glu Phe 279
Asp Thr Ser Leu Leu Glu Glu Asn Glu 288
Asn Leu Gln Ala Asn Glu Ile Met Trp 297
Asn Asn Leu Thr Ala Asp Ser Leu Gln 306
Val Met Leu Lys Thr Leu Ala Glu Glu 315
Leu Thr Gln Thr Gln Gln Met Leu Leu 324
His Lys Glu Ala Ala Val Leu Glu Leu 333
Glu Lys Arg Ile Glu Glu Ser Phe Glu 342

Thr Cys Glu Lys Lys Ser Asp Ile Val 351
Leu Leu Leu Gly Gln Lys Gln Ala Leu 360
Glu Glu Leu Lys Gln Ser Val Gln Gln 369
Leu Arg Cys Ser Glu Ala Lys Cys Ala 378
Ala Gln Lys Ala Leu Leu Glu Gln Lys 387
Val Gln Glu Asn Asp Gly Lys Glu Pro 396
Pro Pro Val Val Asn Tyr Glu Glu Asp 405
Ala Arg Ser Val Thr Ser Met Glu Arg 414
Lys Glu Arg Leu Ser Lys Phe Glu Ser 423
Ile Arg His Ser Ile Ala Gly Ile Ile 432
Lys Ser Pro Lys Ser Val Leu Gly Ser 441
Ser Thr Gln Val Cys Asp Val Ile Ser 450
Val Gly Glu Arg Pro Leu Ala Glu His 459
Asp Trp Tyr His Gly Ala Ile Pro Arg 468
Ile Glu Ala Gln Glu Leu Leu Lys Gln 477
Gln Gly Asp Phe Leu Val Arg Glu Ser 486
His Gly Lys Pro Gly Glu Tyr Val Leu 495
Ser Val Tyr Ser Asp Gly Gln Arg Arg 504
His Phe Ile Ile Gln Phe Val Asp Asn 513
Leu Tyr Arg Phe Glu Gly Thr Gly Phe 522
Ser Asn Ile Pro Gln Leu Ile Asp His 531
His Phe Asn Thr Lys Gln Val Ile Thr 540
Lys Lys Ser Gly Val Val Leu Leu Asn 549
Pro Ile Pro Lys Asp Lys Lys Trp Val 558
Leu Asn His Glu Asp Val Ser Leu Gly 567
Glu Leu Leu Gly Lys Gly Asn Phe Gly 576
Glu Val Tyr Lys Gly Thr Leu Lys Asp 585
Lys Thr Pro Val Ala Ile Lys Thr Cys 594
Lys Glu Asp Leu Pro Gln Glu Leu Lys 603
Ile Lys Phe Leu Gln Glu Ala Lys Ile 612
Leu Lys Gln Tyr Asp His Pro Asn Ile 621
Val Lys Leu Ile Gly Val Cys Thr Gln 630
Arg Gln Pro Val Tyr Ile Ile Met Glu 639
Leu Val Pro Gly Gly Asp Phe Leu Thr 648
Phe Leu Arg Lys Arg Lys Asp Glu Leu 657
Lys Leu Lys Gln Leu Val Arg Phe Ser 666
Leu Asp Val Ala Ala Gly Met Leu Tyr 675
Leu Glu Ser Lys Asn Cys Ile His Arg 684
Asp Leu Ala Ala Arg Asn Cys Leu Val 693
Gly Glu Asn Asn Thr Leu Lys Ile Ser 702
Asp Phe Gly Met Ser Arg Gln Glu Asp 711
Gly Gly Val Tyr Ser Ser Ser Gly Leu 720
Lys Gln Ile Pro Ile Lys Trp Thr Ala 729
Pro Glu Ala Leu Asn Tyr Gly Arg Tyr 738
Ser Ser Glu Ser Asp Val Trp Ser Phe 747
Gly Ile Leu Leu Trp Glu Thr Phe Ser 756
Leu Gly Val Cys Pro Tyr Pro Gly Met 765
Thr Asn Gln Gln Ala Arg Glu Gln Val 774
Glu Arg Gly Tyr Arg Met Ser Ala Pro 783
Gln Asn Cys Pro Glu Glu Val Phe Thr 792
Ile Met Met Lys Cys Trp Asp Tyr Lys 801
Pro Glu Asn Arg Pro Lys Phe Asn Asp 810

Leu His Lys Glu Leu Thr Val Ile Lys 819
Lys Met Ile Thr 823

<210> 64
<211> 822
<212> PRT
<213> Homo sapiens

<220>
<223> Fer

<400> 64

Met Gly Phe Gly Ser Asp Leu Lys Asn 9
Ser His Glu Ala Val Leu Lys Leu Gln 18
Asp Trp Glu Leu Arg Leu Leu Glu Thr 27
Val Lys Lys Phe Met Ala Leu Arg Ile 36
Lys Ser Asp Lys Glu Tyr Ala Ser Thr 45
Leu Gln Asn Leu Cys Asn Gln Val Asp 54
Lys Glu Ser Thr Val Gln Met Asn Tyr 63
Val Ser Asn Val Ser Lys Ser Trp Leu 72
Leu Met Ile Gln Gln Thr Glu Gln Leu 81
Ser Arg Ile Met Lys Thr His Ala Glu 90
Asp Leu Asn Ser Gly Pro Leu His Arg 99
Leu Thr Met Met Ile Lys Asp Lys Gln 108
Gln Val Lys Lys Ser Tyr Ile Gly Val 117
His Gln Gln Ile Glu Ala Glu Met Ile 126
Lys Val Thr Lys Thr Glu Leu Glu Lys 135
Leu Lys Cys Ser Tyr Arg Gln Leu Ile 144
Lys Glu Met Asn Ser Ala Lys Glu Lys 153
Tyr Lys Glu Ala Leu Ala Lys Gly Lys 162
Glu Thr Glu Lys Ala Lys Glu Arg Tyr 171
Asp Lys Ala Thr Met Lys Leu His Met 180
Leu His Asn Gln Tyr Val Leu Ala Leu 189
Lys Gly Ala Gln Leu His Gln Asn Gln 198
Tyr Tyr Asp Ile Thr Leu Pro Leu Leu 207
Leu Asp Ser Leu Gln Lys Met Gln Glu 216
Glu Met Ile Lys Ala Leu Lys Gly Ile 225
Phe Asp Glu Tyr Ser Gln Ile Thr Ser 234
Leu Val Thr Glu Glu Ile Val Asn Val 243
His Lys Glu Ile Gln Met Ser Val Glu 252
Gln Ile Asp Pro Ser Thr Glu Tyr Asn 261
Asn Phe Ile Asp Val His Arg Thr Thr 270
Ala Ala Lys Glu Gln Glu Ile Glu Phe 279
Asp Thr Ser Leu Leu Glu Glu Asn Glu 288
Asn Leu Gln Ala Asn Glu Ile Met Trp 297
Asn Asn Leu Thr Ala Glu Ser Leu Gln 306
Val Met Leu Lys Thr Leu Ala Glu Glu 315
Leu Met Gln Thr Gln Gln Met Leu Leu 324
Asn Lys Glu Glu Ala Val Leu Glu Leu 333
Glu Lys Arg Ile Glu Glu Ser Ser Glu 342

Thr Cys Glu Lys Lys Ser Asp Ile Val 351
Leu Leu Leu Ser Gln Lys Gln Ala Leu 360
Glu Glu Leu Lys Gln Ser Val Gln Gln 369
Leu Arg Cys Thr Glu Ala Lys Phe Ser 378
Ala Gln Lys Glu Leu Leu Glu Gln Lys 387
Val Gln Glu Asn Asp Gly Lys Glu Pro 396
Pro Pro Val Val Asn Tyr Glu Glu Asp 405
Ala Arg Ser Val Thr Ser Met Glu Arg 414
Lys Glu Arg Leu Ser Lys Phe Glu Ser 423
Ile Arg His Ser Ile Ala Gly Ile Ile 432
Arg Ser Pro Lys Ser Ala Val Gly Ser 441
Ser Ala Leu Ser Asp Met Ile Ser Ile 450
Ser Glu Lys Pro Leu Ala Glu Gln Asp 459
Trp Tyr His Gly Ala Ile Pro Arg Ile 468
Glu Ala Gln Glu Leu Leu Lys Lys Gln 477
Gly Asp Phe Leu Val Arg Glu Ser His 486
Gly Lys Pro Gly Glu Tyr Val Leu Ser 495
Val Tyr Ser Asp Gly Gln Arg Arg His 504
Phe Ile Ile Gln Tyr Val Asp Asn Met 513
Tyr Arg Phe Glu Gly Thr Gly Phe Ser 522
Asn Ile Pro Gln Leu Ile Asp His His 531
Tyr Thr Thr Lys Gln Val Ile Thr Lys 540
Lys Ser Gly Val Val Leu Leu Asn Pro 549
Ile Pro Lys Asp Lys Lys Trp Ile Leu 558
Ser His Glu Asp Val Ile Leu Gly Glu 567
Leu Leu Gly Lys Gly Asn Phe Gly Glu 576
Val Tyr Lys Gly Thr Leu Lys Asp Lys 585
Thr Ser Val Ala Val Lys Thr Cys Lys 594
Glu Asp Leu Pro Gln Glu Leu Lys Ile 603
Lys Phe Leu Gln Glu Ala Lys Ile Leu 612
Lys Gln Tyr Asp His Pro Asn Ile Val 621
Lys Leu Ile Gly Val Cys Thr Gln Arg 630
Gln Pro Val Tyr Ile Ile Met Glu Leu 639
Val Ser Gly Gly Asp Phe Leu Thr Phe 648
Leu Arg Arg Lys Lys Asp Glu Leu Lys 657
Leu Lys Gln Leu Val Lys Phe Ser Leu 666
Asp Ala Ala Ala Gly Met Leu Tyr Leu 675
Glu Ser Lys Asn Cys Ile His Arg Asp 684
Leu Ala Ala Arg Asn Cys Leu Val Gly 693
Glu Asn Asn Val Leu Lys Ile Ser Asp 702
Phe Gly Met Ser Arg Gln Glu Asp Gly 711
Gly Val Tyr Ser Ser Ser Gly Leu Lys 720
Gln Ile Pro Ile Lys Trp Thr Ala Pro 729
Glu Ala Leu Asn Tyr Gly Arg Tyr Ser 738
Ser Glu Ser Asp Val Trp Ser Phe Gly 747
Ile Leu Leu Trp Glu Thr Phe Ser Leu 756
Gly Val Cys Pro Tyr Pro Gly Met Thr 765
Asn Gln Gln Ala Arg Glu Gln Val Glu 774
Arg Gly Tyr Arg Met Ser Ala Pro Gln 783
His Cys Pro Glu Asp Ile Ser Lys Ile 792
Met Met Lys Cys Trp Asp Tyr Lys Pro 801
Glu Asn Arg Pro Lys Phe Ser Glu Leu 810

Gln Lys Glu Leu Thr Ile Ile Lys Arg 819
Lys Leu Thr 822

<210> 65
<211> 116
<212> PRT
<213> Mus musculus

<220>
<223> MRP-3

<400> 65

Met Arg Asn Ser Lys Thr Ala Ile Ser 9
Phe Phe Ile Leu Val Ala Val Leu Gly 18
Ser Gln Ala Gly Leu Ile Gln Glu Met 27
Glu Lys Glu Asp Arg Arg Tyr Asn Pro 36
Pro Ile Ile His Gln Gly Phe Gln Asp 45
Thr Ser Ser Asp Cys Cys Phe Ser Tyr 54
Ala Thr Gln Ile Pro Cys Lys Arg Phe 63
Ile Tyr Tyr Phe Pro Thr Ser Gly Gly 72
Cys Ile Lys Pro Gly Ile Ile Phe Ile 81
Ser Arg Arg Gly Thr Gln Val Cys Ala 90
Asp Pro Ser Asp Arg Arg Val Gln Arg 99
Cys Leu Ser Thr Leu Lys Gln Gly Pro 108
Arg Ser Gly Asn Lys Val Ile Ala 116

<210> 66
<211> 120
<212> PRT
<213> Homo sapiens

<220>
<223> MRP-3

<400> 66

Met Lys Val Ser Val Ala Ala Leu Ser 9
Cys Leu Met Leu Val Thr Ala Leu Gly 18
Ser Gln Ala Arg Val Thr Lys Asp Ala 27
Glu Thr Glu Phe Met Met Ser Lys Leu 36
Pro Leu Glu Asn Pro Val Leu Leu Asp 45
Arg Phe His Ala Thr Ser Ala Asp Cys 54
Cys Ile Ser Tyr Thr Pro Arg Ser Ile 63
Pro Cys Ser Leu Leu Glu Ser Tyr Phe 72
Glu Thr Asn Ser Glu Cys Ser Lys Pro 81
Gly Val Ile Phe Leu Thr Lys Lys Gly 90
Arg Arg Phe Cys Ala Asn Pro Ser Asp 99
Lys Gln Val Gln Val Cys Met Arg Met 108
Leu Lys Leu Asp Thr Arg Ile Lys Thr 117

Arg Lys Asn 120

<210> 67
<211> 264
<212> PRT
<213> Mus musculus

<220>
<223> NNMT

<400> 67

Met Glu Ser Gly Phe Thr Ser Lys Asp 9
Thr Tyr Leu Ser His Phe Asn Pro Arg 18
Asp Tyr Leu Glu Lys Tyr Tyr Ser Phe 27
Gly Ser Arg His Cys Ala Glu Asn Glu 36
Ile Leu Arg His Leu Leu Lys Asn Leu 45
Phe Lys Ile Phe Cys Leu Gly Ala Val 54
Lys Gly Glu Leu Leu Ile Asp Ile Gly 63
Ser Gly Pro Thr Ile Tyr Gln Leu Leu 72
Ser Ala Cys Glu Ser Phe Thr Glu Ile 81
Ile Val Ser Asp Tyr Thr Asp Gln Asn 90
Leu Trp Glu Leu Gln Lys Trp Leu Lys 99
Lys Glu Pro Gly Ala Phe Asp Trp Ser 108
Pro Val Val Thr Tyr Val Cys Asp Leu 117
Glu Gly Asn Arg Met Lys Gly Pro Glu 126
Lys Glu Glu Lys Leu Arg Arg Ala Ile 135
Lys Gln Val Leu Lys Cys Asp Val Thr 144
Gln Ser Gln Pro Leu Gly Gly Val Ser 153
Leu Pro Pro Ala Asp Cys Leu Leu Ser 162
Thr Leu Cys Leu Asp Ala Ala Cys Pro 171
Asp Leu Pro Ala Tyr Arg Thr Ala Leu 180
Arg Asn Leu Gly Ser Leu Leu Lys Pro 189
Gly Gly Phe Leu Val Met Val Asp Ala 198
Leu Lys Ser Ser Tyr Tyr Met Ile Gly 207
Glu Gln Lys Phe Ser Ser Leu Pro Leu 216
Gly Trp Glu Thr Val Arg Asp Ala Val 225
Glu Glu Ala Gly Tyr Thr Ile Glu Gln 234
Phe Glu Val Ile Ser Gln Asn Tyr Ser 243
Ser Thr Thr Ser Asn Asn Glu Gly Leu 252
Phe Ser Leu Val Gly Arg Lys Pro Gly 261
Arg Ser Glu 264

<210> 68
<211> 264
<212> PRT
<213> Homo sapiens

<220>

<223> NNMT

<400> 68

Met Glu Ser Gly Phe Thr Ser Lys Asp 9
Thr Tyr Leu Ser His Phe Asn Pro Arg 18
Asp Tyr Leu Glu Lys Tyr Tyr Lys Phe 27
Gly Ser Arg His Ser Ala Glu Ser Gln 36
Ile Leu Lys His Leu Leu Lys Asn Leu 45
Phe Lys Ile Phe Cys Leu Asp Gly Val 54
Lys Gly Asp Leu Leu Ile Asp Ile Gly 63
Ser Gly Pro Thr Ile Tyr Gln Leu Leu 72
Ser Ala Cys Glu Ser Phe Lys Glu Ile 81
Val Val Thr Asp Tyr Ser Asp Gln Asn 90
Leu Gln Glu Leu Glu Lys Trp Leu Lys 99
Lys Glu Pro Glu Ala Phe Asp Trp Ser 108
Pro Val Val Thr Tyr Val Cys Asp Leu 117
Glu Gly Asn Arg Val Lys Gly Pro Glu 126
Lys Glu Glu Lys Leu Arg Gln Ala Val 135
Lys Gln Val Leu Lys Cys Asp Val Thr 144
Gln Ser Gln Pro Leu Gly Ala Val Pro 153
Leu Pro Pro Ala Asp Cys Val Leu Ser 162
Thr Leu Cys Leu Asp Ala Ala Cys Pro 171
Asp Leu Pro Thr Tyr Cys Arg Ala Leu 180
Arg Asn Leu Gly Ser Leu Leu Lys Pro 189
Gly Gly Phe Leu Val Ile Met Asp Ala 198
Leu Lys Ser Ser Tyr Tyr Met Ile Gly 207
Glu Gln Lys Phe Ser Ser Leu Pro Leu 216
Gly Arg Glu Ala Val Glu Ala Ala Val 225
Lys Glu Ala Gly Tyr Thr Ile Glu Trp 234
Phe Glu Val Ile Ser Gln Ser Tyr Ser 243
Ser Thr Met Ala Asn Asn Glu Gly Leu 252
Phe Ser Leu Val Ala Arg Lys Leu Ser 261
Arg Pro Leu 264

<210> 69
<211> 158
<212> PRT
<213> Mus musculus

<220>
<223> UBC9

<400> 69

<210> 70
<211> 158
<212> PRT
<213> Homo sapiens

<220>
<223> UBC9

<400> 70

<210> 71
<211> 109
<212> PRT
<213> Homo sapiens

<220>
<223> MCP-2

<400> 71

Met Leu Lys Leu Thr Pro Leu Pro Ser 9
Lys Met Lys Val Ser Ala Ala Leu Leu 18
Cys Leu Leu Leu Met Ala Ala Thr Phe 27
Ser Pro Gln Gly Leu Ala Gln Pro Asp 36
Ser Val Ser Ile Pro Ile Thr Cys Cys 45
Phe Asn Val Ile Asn Arg Lys Ile Pro 54
Ile Gln Arg Leu Glu Ser Tyr Thr Arg 63
Ile Thr Asn Ile Gln Cys Pro Lys Glu 72
Ala Val Ile Phe Lys Thr Gln Arg Gly 81
Lys Glu Val Cys Ala Asp Pro Lys Glu 90
Arg Trp Val Arg Asp Ser Met Lys His 99
Leu Asp Gln Ile Phe Gln Asn Leu Lys 108
Pro 109

<210> 72
<211> 462
<212> PRT
<213> Mus musculus

<220>
<223> Cathepsin C

<400> 72

Met Gly Pro Trp Thr His Ser Leu Arg 9
Ala Val Leu Leu Leu Val Leu Leu Gly 18
Val Cys Thr Val Arg Ser Asp Thr Pro 27
Ala Asn Cys Thr Tyr Pro Asp Leu Leu 36
Gly Thr Trp Val Phe Gln Val Gly Pro 45
Arg Ser Ser Arg Ser Asp Ile Asn Cys 54
Ser Val Met Glu Ala Thr Glu Glu Lys 63
Val Val Val His Leu Lys Lys Leu Asp 72
Thr Ala Tyr Asp Glu Leu Gly Asn Ser 81
Gly His Phe Thr Leu Ile Tyr Asn Gln 90
Gly Phe Glu Ile Val Leu Asn Asp Tyr 99
Lys Trp Phe Ala Phe Phe Lys Tyr Glu 108
Val Arg Gly His Thr Ala Ile Ser Tyr 117
Cys His Glu Thr Met Thr Gly Trp Val 126
His Asp Val Leu Gly Arg Asn Trp Ala 135
Cys Phe Val Gly Lys Lys Val Glu Ser 144
His Ile Glu Lys Val Asn Met Asn Ala 153
Ala His Leu Gly Gly Leu Gln Glu Arg 162
Tyr Ser Glu Arg Leu Tyr Thr His Asn 171
His Asn Phe Val Lys Ala Ile Asn Thr 180
Val Gln Lys Ser Trp Thr Ala Thr Ala 189
Tyr Lys Glu Tyr Glu Lys Met Ser Leu 198
Arg Asp Leu Ile Arg Arg Ser Gly His 207

Ser Gln Arg Ile Pro Arg Pro Lys Pro 216
Ala Pro Met Thr Asp Glu Ile Gln Gln 225
Gln Ile Leu Asn Leu Pro Glu Ser Trp 234
Asp Trp Arg Asn Val Gln Gly Val Asn 243
Tyr Val Ser Pro Val Arg Asn Gln Glu 252
Ser Cys Gly Ser Cys Tyr Ser Phe Ala 261
Ser Met Gly Met Leu Glu Ala Arg Ile 270
Arg Ile Leu Thr Asn Asn Ser Gln Thr 279
Pro Ile Leu Ser Pro Gln Glu Val Val 288
Ser Cys Ser Pro Tyr Ala Gln Gly Cys 297
Asp Gly Gly Phe Pro Tyr Leu Ile Ala 306
Gly Lys Tyr Ala Gln Asp Phe Gly Val 315
Val Glu Glu Ser Cys Phe Pro Tyr Thr 324
Ala Lys Asp Ser Pro Cys Lys Pro Arg 333
Glu Asn Cys Leu Arg Tyr Tyr Ser Ser 342
Asp Tyr Tyr Tyr Val Gly Gly Phe Tyr 351
Gly Gly Cys Asn Glu Ala Leu Met Lys 360
Leu Glu Leu Val Lys His Gly Pro Met 369
Ala Val Ala Phe Glu Val His Asp Asp 378
Phe Leu His Tyr His Ser Gly Ile Tyr 387
His His Thr Gly Leu Ser Asp Pro Phe 396
Asn Pro Phe Glu Leu Thr Asn His Ala 405
Val Leu Leu Val Gly Tyr Gly Arg Asp 414
Pro Val Thr Gly Ile Glu Tyr Trp Ile 423
Ile Lys Asn Ser Trp Gly Ser Asn Trp 432
Gly Glu Ser Gly Tyr Phe Arg Ile Arg 441
Arg Gly Thr Asp Glu Cys Ala Ile Glu 450
Ser Ile Ala Val Ala Ala Ile Pro Ile 459
Pro Lys Leu 462

<210> 73
<211> 463
<212> PRT
<213> Homo sapiens

<220>
<223> Cathepsin C

<400> 73

Met Gly Ala Gly Pro Ser Leu Leu Leu 9
Ala Ala Leu Leu Leu Leu Leu Ser Gly 18
Asp Gly Ala Val Arg Cys Asp Thr Pro 27
Ala Asn Cys Thr Tyr Leu Asp Leu Leu 36
Gly Thr Trp Val Phe Gln Val Gly Ser 45
Ser Gly Ser Gln Arg Asp Val Asn Cys 54
Ser Val Met Gly Pro Gln Glu Lys Lys 63
Val Val Val Tyr Leu Gln Lys Leu Asp 72
Thr Ala Tyr Asp Asp Leu Gly Asn Ser 81
Gly His Phe Thr Ile Ile Tyr Asn Gln 90
Gly Phe Glu Ile Val Leu Asn Asp Tyr 99

Lys Trp Phe Ala Phe Phe Lys Tyr Lys 108
Glu Glu Gly Ser Lys Val Thr Thr Tyr 117
Cys Asn Glu Thr Met Thr Gly Trp Val 126
His Asp Val Leu Gly Arg Asn Trp Ala 135
Cys Phe Thr Gly Lys Lys Val Gly Thr 144
Ala Ser Glu Asn Val Tyr Val Asn Thr 153
Ala His Leu Lys Asn Ser Gln Glu Lys 162
Tyr Ser Asn Arg Leu Tyr Lys Tyr Asp 171
His Asn Phe Val Lys Ala Ile Asn Ala 180
Ile Gln Lys Ser Trp Thr Ala Thr Thr 189
Tyr Met Glu Tyr Glu Thr Leu Thr Leu 198
Gly Asp Met Ile Arg Arg Ser Gly Gly 207
His Ser Arg Lys Ile Pro Arg Pro Lys 216
Pro Ala Pro Leu Thr Ala Glu Ile Gln 225
Gln Lys Ile Leu His Leu Pro Thr Ser 234
Trp Asp Trp Arg Asn Val His Gly Ile 243
Asn Phe Val Ser Pro Val Arg Asn Gln 252
Ala Ser Cys Gly Ser Cys Tyr Ser Phe 261
Ala Ser Met Gly Met Leu Glu Ala Arg 270
Ile Arg Ile Leu Thr Asn Asn Ser Gln 279
Thr Pro Ile Leu Ser Pro Gln Glu Val 288
Val Ser Cys Ser Gln Tyr Ala Gln Gly 297
Cys Glu Gly Gly Phe Pro Tyr Leu Ile 306
Ala Gly Lys Tyr Ala Gln Asp Phe Gly 315
Leu Val Glu Glu Ala Cys Phe Pro Tyr 324
Thr Gly Thr Asp Ser Pro Cys Lys Met 333
Lys Glu Asp Cys Phe Arg Tyr Tyr Ser 342
Ser Glu Tyr His Tyr Val Gly Gly Phe 351
Tyr Gly Gly Cys Asn Glu Ala Leu Met 360
Lys Leu Glu Leu Val His His Gly Pro 369
Met Ala Val Ala Phe Glu Val Tyr Asp 378
Asp Phe Leu His Tyr Lys Lys Gly Ile 387
Tyr His His Thr Gly Leu Arg Asp Pro 396
Phe Asn Pro Phe Glu Leu Thr Asn His 405
Ala Val Leu Leu Val Gly Tyr Gly Thr 414
Asp Ser Ala Ser Gly Met Asp Tyr Trp 423
Ile Val Lys Asn Ser Trp Gly Thr Gly 432
Trp Gly Glu Asn Gly Tyr Phe Arg Ile 441
Arg Arg Gly Thr Asp Glu Cys Ala Ile 450
Glu Ser Ile Ala Val Ala Ala Thr Pro 459
Ile Pro Lys Leu 463

<210> 74
<211> 21
<212> DNA
<213> Homo sapiens

<220>
<223> hGAPDH-Primer 1

<400> 74

CCTCCCCTCT TCAAGGGTCT A 21

<210> 75
<211> 21
<212> DNA
<213> Homo sapiens

<220>
<223> hGAPDH-Primer 2

<400> 75

AGGAGTAAGA CCCCTGGACC A 21

<210> 76
<211> 21
<212> DNA
<213> Homo sapiens

<220>
<223> CCR-1 Primer 1

<400> 76

CCCAATGGGA ATTCACTCAC C 21

<210> 77
<211> 22
<212> DNA
<213> Homo sapiens

<220>
<223> CCR-1 Primer 2

<400> 77

GCTTCCACTC TCGTAGGCTT TC 22

<210> 78
<211> 25
<212> DNA
<213> Homo sapiens

<220>
<223> Cyclophilin Primer 1

<400> 78

TCTTAACCAC CAGATCATTC CTTCT 25

<210> 79
<211> 20
<212> DNA
<213> Homo sapiens

<220>
<223> Cyclophilin Primer 2

<400> 79

GGATACTGCG AGCAAATGGG 20

<210> 80
<211> 347
<212> Protein
<213> Homo sapiens

<220>
<223> BAF57 Variante

<400> 80

Met Ser Lys Arg Pro Ser Tyr Ala Pro Pro Pro Thr 12
Pro Ala Pro Ala Thr Gln Met Pro Ser Thr Pro Gly 24
Phe Val Gly Tyr Asn Pro Tyr Ser His Leu Ala Tyr 36
Asn Asn Tyr Arg Leu Gly Gly Asn Pro Gly Thr Asn 48
Ser Arg Val Thr Ala Ser Ser Gly Ile Thr Ile Pro 60
Lys Pro Pro Lys Pro Pro Asp Lys Pro Leu Met Pro 72
Tyr Met Arg Tyr Ser Arg Lys Val Trp Asp Gln Val 84
Lys Ala Ser Asn Pro Asp Leu Lys Leu Trp Glu Ile 96
Gly Lys Ile Ile Gly Gly Met Trp Arg Asp Leu Thr 108
Asp Glu Glu Lys Gln Glu Tyr Leu Asn Glu Tyr Glu 120
Ala Glu Lys Ile Glu Tyr Asn Glu Ser Met Lys Ala 132
Tyr His Asn Ser Pro Ala Tyr Leu Ala Tyr Ile Asn 144
Ala Lys Ser Arg Ala Glu Ala Ala Leu Glu Glu Glu 156
Ser Arg Gln Arg Gln Ser Arg Met Glu Lys Gly Glu 168
Pro Tyr Met Ser Ile Gln Pro Ala Glu Asp Pro Asp 180
Asp Tyr Asp Asp Gly Phe Ser Met Lys His Thr Ala 192
Thr Ala Arg Phe Gln Arg Asn His Arg Leu Ile Ser 204
Glu Ile Leu Ser Glu Ser Val Val Pro Asp Val Arg 216

Ser Val Val Thr Thr Ala Arg Met Gln Val Leu Lys 228
Arg Gln Val Gln Ser Leu Met Val His Gln Arg Lys 240
Leu Glu Ala Glu Leu Leu Gln Ile Glu Glu Arg His 252
Gln Glu Lys Lys Arg Lys Phe Leu Glu Ser Thr Asp 264
Ser Phe Asn Asn Glu Leu Lys Arg Leu Cys Gly Leu 276
Lys Val Glu Val Asp Met Glu Lys Ile Ala Ala Glu 288
Ile Ala Gln Ala Glu Glu Gln Ala Arg Lys Arg Gln 300
Glu Glu Arg Glu Lys Glu Ala Ala Glu Gln Ala Glu 312
Arg Ser Gln Ser Ser Ile Val Pro Glu Glu Glu Gln 324
Ala Ala Asn Lys Gly Glu Glu Lys Lys Asp Asp Glu 336
Asn Ile Pro Met Glu Thr Gly Asn Ser Pro Ala 347

<210> 81
<211> 197
<212> Protein
<213> Homo sapiens

<220>
<223> Phospholipase Inhibitor GIPL, kurze Variante

<400> 81

Met Trp Val Gln Thr Arg Pro Ser Ser Ala Ser Tyr 12
Lys Ser Trp Gly Pro Gly Thr Ala Asp Thr His Thr 24
Met Arg Leu Ser Arg Arg Pro Glu Thr Phe Leu Leu 36
Ala Phe Val Leu Leu Cys Thr Leu Leu Gly Leu Gly 48
Cys Pro Leu His Cys Glu Ile Cys Thr Ala Ala Gly 60
Ser Arg Cys His Gly Gln Met Lys Thr Cys Ser Ser 72
Asp Lys Asp Thr Cys Val Leu Leu Val Gly Lys Ala 84
Thr Ser Lys Gly Lys Glu Leu Val His Thr Tyr Lys 96
Gly Cys Ile Arg Ser Gln Asp Cys Tyr Ser Gly Val 108
Ile Ser Thr Thr Met Gly Pro Lys Asp His Met Val 120
Thr Ser Ser Phe Cys Cys Gln Ser Asp Gly Cys Asn 132
Ser Ala Phe Leu Ser Val Pro Leu Thr Asn Leu Thr 144
Glu Asn Gly Leu Met Cys Pro Ala Cys Thr Ala Ser 156
Phe Arg Asp Lys Cys Met Gly Pro Met Thr His Cys 168
Thr Gly Lys Glu Asn His Cys Val Ser Leu Ser Gly 180
His Val Gln Ala Gly Glu Trp Cys Leu Asn Leu Trp 192
Lys Arg Lys Gln Asn 197

<210> 82
<211> 285
<212> Protein
<213> Homo sapiens

<220>
<223> tsg101 Spleißvariante

<400> 82

Met Ala Val Ser Glu Ser Gln Leu Lys Lys Met Val 12
Ser Lys Pro Gln Ser Asp Leu Leu Gly Leu Ile Gln 24
Val Met Ile Val Val Phe Gly Asp Glu Pro Pro Val 36
Phe Ser Arg Pro Ile Ser Ala Ser Tyr Pro Pro Tyr 48
Gln Ala Thr Gly Pro Pro Asn Thr Ser Tyr Met Pro 60
Gly Met Pro Gly Gly Ile Ser Pro Tyr Pro Ser Gly 72
Tyr Pro Pro Asn Pro Ser Gly Tyr Pro Gly Cys Pro 84
Tyr Pro Pro Gly Gly Pro Tyr Pro Ala Thr Thr Ser 96
Ser Gln Tyr Pro Ser Gln Pro Pro Val Thr Thr Val 108
Gly Pro Ser Arg Asp Gly Thr Ile Ser Glu Asp Thr 120
Ile Arg Ala Ser Leu Ile Ser Ala Val Ser Asp Lys 132
Leu Arg Trp Arg Met Lys Glu Glu Met Asp Arg Ala 144
Gln Ala Glu Leu Asn Ala Leu Lys Arg Thr Glu Glu 156
Asp Leu Lys Lys Gly His Gln Lys Leu Glu Glu Met 168
Val Thr Arg Leu Asp Gln Glu Val Ala Glu Val Asp 180
Lys Asn Ile Glu Leu Leu Lys Lys Lys Asp Glu Glu 192
Leu Ser Ser Ala Leu Glu Lys Met Glu Asn Gln Ser 204
Glu Asn Asn Asp Ile Asp Glu Val Ile Ile Pro Thr 216
Ala Pro Leu Tyr Lys Gln Ile Leu Asn Leu Tyr Ala 228
Glu Glu Asn Ala Ile Glu Asp Thr Ile Phe Tyr Leu 240
Gly Glu Ala Leu Arg Arg Gly Val Ile Asp Leu Asp 252
Val Phe Leu Lys His Val Arg Leu Leu Ser Arg Lys 264
Gln Phe Gln Leu Arg Ala Leu Met Gln Lys Ala Arg 276
Lys Thr Ala Gly Leu Ser Asp Leu Tyr 285

<210> 83
<211> 1946
<212> DNA
<213> Homo sapiens

<220>
<223> BAF57 Variante

<400> 83

CTGCCCGCGG TGTCTCAGAT TCATTCTTAA GGAACTGAGA ACTTAATCTT CCAAAATGTC 60
AAAAAGACCA TCTTATGCCC CACCTCCCAC CCCAGCTCCT GCAACACAAA TGCCCAGCAC 120
ACCAGGGTTT GTGGGATACA ATCCATACAG TCATCTCGCC TACAACAACT ACAGGCTGGG 180

AGGGAACCCG GGCACCAACA GCCGGGTCAC GGCATCCTCT GGTATCACGA TTCCAAAACC 240
CCCCAAGCCA CCAGATAAGC CGCTGATGCC CTACATGAGG TACAGCAGAA AGGTCTGGGA 300
CCAAGTAAAG GCTTCCAACC CTGACCTAAA GTTGTGGGAG ATTGGCAAGA TTATTGGTGG 360
CATGTGGCGA GATCTCACTG ATGAAGAAAA ACAAGAATAT TTAAACGAAT ACGAAGCAGA 420
AAAGATAGAG TACAATGAAT CTATGAAGGC CTATCATAAT TCCCCCGCGT ACCTTGCTTA 480
CATAAATGCA AAAAGTCGTG CAGAAGCTGC TTTAGAGGAA GAAAGTCGAC AGAGACAATC 540
TCGCATGGAG AAAGGAGAAC CGTACATGAG CATTCAGCCT GCTGAAGATC CAGATGATTA 600
TGATGATGGC TTTTCAATGA AGCATACAGC CACCGCCCGT TTCCAGAGAA ACCACCGCCT 660
CATCAGTGAA ATTCTTAGTG AGAGTGTGGT GCCAGACGTT CGGTCAGTTG TCACAACAGC 720
TAGAATGCAG GTCCTCAAAC GGCAGGTCCA GTCCTTAATG GTTCATCAGC GAAAACTAGA 780
AGCTGAACTT CTTCAAATAG AGGAACGACA CCAGGAGAAG AAGAGGAAAT TCCTGGAAAG 840
CACAGATTCA TTTAACAATG AACTTAAAAG GTTGTGCGGT CTGAAAGTAG AAGTGGATAT 900
GGAGAAAATT GCAGCTGAGA TTGCACAGGC AGAGGAACAG GCCCGCAAAA GGCAGGAGGA 960
AAGGGAGAAG GAGGCCGCAG AGCAAGCTGA GCGCAGTCAG AGCAGCATCG TTCCTGAGGA 1020
AGAACAAGCA GCTAACAAAG GCGAGGAGAA GAAAGACGAC GAGAACATTC CGATGGAGAC 1080
AGGAAACAGC CCAGCATGAC TTTAAACAAA GTGTCTAAAA GACCAACCTT ATATTATTTC 1140
TACAACTGCT TTCTCCTCGT TTAATCCATA GCACTACAGA CTAACTCTCA TTCACTGTCC 1200
AACAGAAATA AAATGCAAGT CACGTGAAAT TTTATATTTT CTACTAGTGA CATATTTTTA 1260
AAAGTTAAGC AAAACAAGTG TTTTAACAAT ATGTTTTATT TAATTCAACA TATCCTAAAT 1320
AAAATTATTT CAACACAGAA GGAACATTTT TAAAATCAGC TTTCCTTTTT TTAAACAGTC 1380
TTTATACACA TCTCAATTCA GAAGCTAAAT TTTCATCGGA AGTATTTGAT CTGCATTTAT 1440
TTAATAAATT TCATTCATTT ACAGCTAAAA AGTAGATTTT CATACCCAAA TTGTTCCAAC 1500
ATTCTTAAAA GTTCTTTAGT CGCACTGGTT CATTTCAGGG GTTTAATAAC CACATGTGGC 1560
TAGTGGCTTC TATATTGGAC AGCACAGCTG TATACTGTCA GCATAACTAG AGTAAAATTT 1620
CATCTTGGAA CTGACAGTGA CAAATGTGTG ATTACAGAGG AGACACACCT TGAAGAAACA 1680
ACAGAGAGCC AACAGAATGG TGAAGAAGGC ACGTCTACTC CTGAGGACAA GGAGAGTGGG 1740
CAGGAGGGGG TCGACAGTAT GGCAGAGGAA GGAACCAGTG ATAGTAACAC TGGCTCGGAG 1800
AGCAACAGTG CAACAGTGGA GGAGCCACCA ACAGATCCCA TACCAGAAGA TGAGAAAAAA 1860
GAATAAGTGT TGCCTTGTTT TGTGTGTTCT AAATACTTTT TTTAATGAAA AAATGTTTTT 1920
TGGTTTTAAT GGTGTTACGT GGTTTG 1946

<210> 84
<211> 932
<212> DNA
<213> Homo sapiens

<220>
<223> Phospholipase Inhibitor GIPL, kurze Variante

<400> 84

CCGGCCGTGG GAAAGGTGAA TGTGGGTCCA GACCCGCCCC TCCTCAGCTT CCTATAAAAG 60
CTGGGGACCA GGTACTGCTG ATACACACAC CATGAGGCTC TCCAGGAGAC CAGAGACCTT 120

TCTGCTGGCC TTTGTGTTGC TCTGCACCCT CCTGGGTCTT GGGTGCCCAC TACACTGCGA 180
AATATGTACG GCGGCGGGGA GCAGGTGCCA TGGCCAAATG AAGACCTGCA GCAGTGACAA 240
GGACACATGT GTGCTCCTGG TCGGGAAGGC TACTTCAAAG GGCAAGGAGT TGGTGCACAC 300
CTACAAGGGC TGCATCAGGT CCCAGGACTG CTACTCCGGC GTTATATCCA CCACCATGGG 360
CCCCAAGGAC CACATGGTAA CCAGCTCCTT CTGCTGCCAG AGCGACGGCT GCAACAGTGC 420
CTTTTTGTCT GTTCCCTTGA CCAATCTTAC TGAGAATGGC CTGATGTGCC CCGCCTGCAC 480
TGCGAGCTTC AGGGACAAAT GCATGGGGCC CATGACCCAC TGTACTGGAA AGGAAAACCA 540
CTGTGTCTCC TTATCTGGAC ACGTGCAGGC TGGTGAGTGG TGCCTGAATC TCTGGAAAAG 600
GAAACAGAAC TAGAGGTCCA AACTTCTAGG TTCGATGGGA GGAGAGGGTT CCAGAGAAGT 660
GGGTGAGGAT GTGTTCTGGG ATTATGAGGA AGAAGGGGCT GAGGTCCCTG ATTCCAGTCT 720
GAAATCTCCC TTTCAGGTAT TTTCAAACCC AGATTTGCTA TGCGGGGCTG TGCTACAGAG 780
AGTATGTGCT TTACCAAGCC TGGTGCTGAA GTACCCACAG GCACCAATGT CCTCTTCCTC 840
CATCATATAG AGTGCACTCA CTCCCCCTGA AAAGCTATCT GAACAAAGGA AGATAATGTA 900
GTGTGAAGTC CCCATTTGTC CTCAGCCTGT AA 932

## Beschreibung

Die Erfindung betrifft die Verwendung von Polypeptiden oder diese kodierende Nukleinsäuren zur Diagnose, Prävention und/oder Behandlung von Erkrankungen von Hautzellen, von Wundheilung und/oder deren krankhaften Störungen sowie ihre Verwendung zur Identifizierung von pharmakologisch aktiven Substanzen.

Wunden heilen im allgemeinen ohne therapeutischen Eingriff ab. Es gibt jedoch zahlreiche Erkrankungen, bei denen die Wundheilung krankhaft gestört ist, wie z.B. Diabetes mellitus, arterielle Verschlußkrankheiten, Schuppenflechte (Psoriasis), Morbus Crohn, Epidermolysis bullosa, altersbedingte Hautveränderungen, Kontaktdermatitis, atopische Dermatitis oder Innervationsstörungen. Wundheilungsstörungen führen zu einer verzögerten Wundheilung oder zu chronischen Wunden. Diese Störungen können durch die Art der Verwundung (z.B. großflächige Wunden, tiefgehende und mechanisch gedehnte Operationswunden, Verbrennungen, Trauma, Dekubitus), medikamentöse Behandlung der Patienten (z.B. mit Corticoiden) aber auch durch die Art der Erkrankung selber verursacht werden. So leiden z.B. 25% der Patienten mit Typ-II-Diabetes häufig an chronischen Ulzera ("diabetischer Fuß"), von denen etwa die Hälfte aufwendige stationäre Behandlungen erfordern und letztlich doch schlecht heilen. Der diabetische Fuß verursacht mehr Klinikaufenthalte als jede andere mit Diabetes assoziierte Komplikation. Die Zahl dieser Fälle bei Diabetes Typ I und II ist im Steigen begriffen und repräsentiert 2,5% aller Klinikeinweisungen. Zudem heilen Wunden mit zunehmendem Alter der Patienten schlechter. Oft ist auch eine Beschleunigung des natürlichen Wundheilungsprozesses wünschenswert, um z.B. die Gefahr von bakteriellen Infektionen oder die Liegezeiten der Patienten zu verringern.

Auch nach erfolgtem Wundverschluß kann es zu weiteren Störungen kommen. Während Wunden fötaler Haut ohne Narbenbildung heilen, kommt es nach Verletzungen in der Postnatalperiode stets zu Narbenbildungen, die oft ein großes kosmetisches Problem darstellen. Bei Patienten mit großflächigen Brandwunden kann zudem die Lebensqualität dramatisch beeinträchtigt werden, zumal vernarbter Haut auch die Anhangsgebilde, wie Haarfollikel, Schweiß- und Talgdrüsen fehlen. Bei entsprechender genetischer Disposition kann es auch zu Keloiden kommen, hypertrophischen Narben, die in die umgebende Haut einwuchern.

Der Vorgang der Wundheilung erfordert komplexe koordiniert ablaufende Wirkungen und Interaktionen verschiedener Zelltypen. Im Wundheilungsprozeß unterscheidet man folgende Schritte: die Blutgerinnung im Bereich der Wunde, die Rekrutierung von Entzündungszellen, die Reepithelialisierung, die Bildung von Granulationsgewebe sowie die Restrukturierung von Matrix. Die exakten Reaktionsmuster der beteiligten Zelltypen während der Phasen der Proliferation, der Migration, der Matrixsynthese und der Kontraktion sind, ebenso wie die Regulation von Genen wie z.B. Wachstumsfaktoren, Rezeptoren und Matrixproteinen, bislang wenig bekannt.

So sind bisher nur wenig zufriedenstellende Therapien entwickelt worden, um bei Wundheilungsstörungen eingreifen zu können. Etablierte Therapieformen beschränken sich auf physikalische Unterstützung der Wundheilung (z.B. Verbände, Kompressen, Gele) oder der Transplantation von Hautgeweben, gezüchteten Hautzellen und/oder Matrixproteinen. In den letzten Jahren sind Wachstumsfaktoren zur Verbesserung der Wundheilung erprobt worden, ohne jedoch die konventionelle Therapie entscheidend zu verbessern. Auch die Diagnose von Wundheilungsstörungen beruht auf wenig aussagekräftigen optischen Analysen der Haut, da ein tieferes Verständnis der Genregulation während der Wundheilung bisher fehlt.

Auch für andere Störungen von regenerativen Prozessen sind bisher wenig zufriedenstellende Therapien entwickelt worden. Auch hier ist die Kenntnis der Genregulation für die Entwicklung von Diagnostika und Therapien vorteilhaft. Es ist gezeigt worden (Finch et al., 1997, Am. J. Pathol. 151: 1619-28; Werner, 1998, Cytokine Growth Factor Rev. 9: 153-165), daß wundheilungsrelevante Gene auch bei dermatologischen Erkrankungen, die auf Störungen der Regeneration der Haut beruhen, und allgemein bei regenerativen Prozessen eine entscheidende Rolle spielen. So spielt der Wachstumsfaktor KGF nicht nur eine entscheidende Rolle bei der Regulation der Proliferation und Differenzierung von Keratinozyten während der Wundheilung, sondern ist auch ein wichtiger Faktor bei der Hyperproliferation der Keratinozyten bei der Schuppenflechte (Psoriasis) und Regenerationsprozessen im Darm (bei Morbus Crohn und ulcerativer Colitis)

Es ist daher Aufgabe der vorliegenden Erfindung, Polypeptide und diese kodierende Nukleinsäuren zur Verfügung zu stellen, die an Prozessen bei Erkrankungen von Hautzellen, der Wundheilung und/oder deren krankhaften Störungen , beteiligt sind, und deren Verwendung die Diagnose und/oder Behandlung sowie die Identifizierung und Entwicklung von in Zusammenhang mit diesen Erkrankungen wirksamen Pharmazeutika und Diagnostika entscheidend verbessert.

Die Erkrankungen der Haut, die Wundheilung und deren krankhafte Störungen im Sinne der Erfindung sind abzugrenzen von Hauterkrankungen, die mit einer entarteten Zellentwicklung und Zelldifferenzierung einhergehen, insbesondere von Hautkrebs. Bei letzterer Erkrankung kommt es zur Transformation einzelner Zellen, die dadurch unkontrolliert, autonom, d.h. von Interaktionen mit anderen Zelltypen isoliert zu proliferieren beginnen und dabei die pathologischen Veränderungen an die Tochterzellen vererben. Es handelt sich also um eine Erkrankung, die mit einem Verlust von Interaktionen, beispielsweise von Zell-Zell Adhäsion und von typischen Zelleigenschaften einhergeht. Hingegen beruhen Krankheiten im Sinne der Erfindung auf Störungen der Zell-Zell Interaktionen. Die Entstehung von Hauterkrankungen im Sinne der Erfindung, sind durch eine Vielzahl von Faktoren bedingt. So spielen z.B. im Falle der Psoriasis, wahrscheinlich sowohl genetische Prädispositionen als auch Fehlfunktionen der T-Zellen, Fibroblasten und Keratinozyten eine Rolle (siehe z.B. Nair et al., 1997; Hum. Molec. Genet. 6: 1349-1356; Gottlieb et al., 1995, Nat. Med. 1: 442-447; Saiag et al., 1985, Science, 230: 669-672; Pittelkow, 1998, in Roenigk 1998: 225-246). Auch der Verlauf der Wundheilung kann durch verschiedenste endogene und exogene Faktoren moduliert sein. Bereits kleine Störungen der Interaktionen zwischen den unterschiedlichen Zelltypen der Dermis und Epidermis selbst, aber auch Wechselwirkungen mit anderen Geweben und Organen wie dem Blutgefäßsystem, dem Nervensystem und dem Bindegewebe, können zu gestörter Wundheilung gefolgt von Narbenbildung führen. Weiterhin können Infektionen, Alterung, Erkrankungen, wie Diabetes und Immunerkrankungen sowie Vitaminmängel den Wundheilungsprozeß beeinträchtigen. Ähnlich komplexe Wechselwirkungen sind auch für andere Hauterkrankungen wie Vitiligo und atopische Dermatitis beschrieben. Dies unterscheidet die Hauterkrankungen wesentlich von Krebserkrankungen dieser Organe. Bevorzugte Beispiele von Hauterkrankungen sind Psoriasis, Ekzeme, insbesondere atopisches Ekzeme und Pigmentstörungen der Haut, insbesondere Vitiligo. Beispiele für gestörte Wundheilung sind Wunden diabetischer Patienten und Alkoholiker, mit Mikroorganismen infizierte Wunden, ischämische Wunden und Wunden von Patienten mit mangelnder Durchblutung und Venenstau. Besonders bevorzugte Beispiele sind schlecht heilende Wunden, diabetische, neuropathische, venösen und arterielle Ulzera, insbesondere diabetische Ulzera.

Der autonome Charakter der Krebserkrankungen zeigt sich auch auf therapeutischer Ebene. Im Falle nicht-metastasierender Tumore läßt sich Krebs chirurgisch behandeln ist. Diese physikalische Behandlungsmöglichkeit ist möglich, da zwischen Tumorzellen und den umliegenden Zellen und Geweben keine Interaktionen stattfinden, so das durch einfache Exzision des Tumors der Patient geheilt werden kann, während dies bei Hautkrankheiten im Sinne der Erfindung nicht möglich ist - die krankhaften Störungen der Zell-Zell und/oder Gewebe-Gewebe Interaktionen können nicht durch Herausschneiden betroffener Hautstellen behoben werden. Daß es sich bei den verglichenen Krankheiten um Krankheiten handelt, denen ein grundsätzlich unterschiedlicher Mechanismus zugrunde liegt, wird deutlich, wenn man die therapeutischen Ansätze vergleicht. Bei Krebserkrankungen und Krankheiten, die mit einer entarteten Zellproliferation einhergehen, ist die Therapie auf die Abtötung schnell wachsender Zellen z.B. mittels Zytostatika gerichtet. Diese toxischen Substanzen verhindern das Wachstum aktiv proliferierender Zellen während Zellen in der G0-Phase des Zellzyklus nicht beeinflußt werden. Hingegen zielt die Behandlung von Erkrankungen von Hautzellen im Sinne der Erfindung auf die Modulation der Interaktionen zwischen den verschiedenen Zelltypen, wie z.B. durch die Beeinflussung der Migration, Proliferation und Differenzierung *einzelner* Zelltypen. Hauterkrankungen im Sinne der Erfindung können nicht durch generelle Inaktivierung proliferierender Zellen geheilt werden. Der methodische Ansatz zur Identifizierung der erfindungsgemäß verwendeten Nukleinsäuren, die an der Wundheilung und/oder an Prozessen der Hauterkrankungen im Sinne der Erfindung beteiligt sind unterscheidet sich deutlich von Verfahren, die geeignet sind Nukleinsäuren zu identifizieren, die an Prozessen der Krebserkrankungen beteiligt sind. Letztere können durch Analyse differentiell exprimierter Gene des vom Krebs betroffenen Zelltyps identifiziert werden. Ziel des Assays der vorliegenden Erfindung ist es jedoch vielmehr, durch Vergleich der Expression in kranken und gesunden Gewebebiopsien Gene zu identifizieren, die an den komplexen Prozesse der Hauterkrankungen und/oder an der Wundheilung und/oder deren krankhaften Störungen beteiligt sind. Zur Identifizierung von krebsrelevanten Gene wäre dieses Verfahren ungeeignet.

Bei der Analyse der Genexpression während des Wundheilungsprozesses konnten überraschenderweise Gene identifiziert werden, die bisher nicht mit Diagnose und/oder Behandlung von Erkrankungen, Hauterkrankungen, bei der Wundheilung und/oder deren Störungen oder zur Identifizierung von pharmakologisch aktiven Substanzen in Verbindung gebracht wurden, deren Regulation aber für den Heilungsprozeß essentiell ist und die damit im kausalen Zusammenhang mit Diagnose und/oder Behandlung von Erkrankungen, Hauterkrankungen, bei der Wundheilung und/oder deren Störungen oder zur Identifizierung von pharmakologisch aktiven Substanzen stehen. Die Polypeptide dieser Gene gehören nicht zu den bisher bekannten Zielen für die Diagnose - wie beispielsweise der Indikation-und/oder der Behandlung - wie beispielsweise der Modulation - von Erkrankungen oder Wundheilung und/oder deren Störungen und/oder Hauterkrankungen oder für die Identifizierung von pharmakologisch aktiven Substanzen, so daß sich aus dieser Erfindung völlig neue Therapieansätze ergeben.

Die Aufgabe wird daher erfindungsgemäß durch die Verwendung eines oder mehrerer Polypeptide gemäß einer der SEQ ID Nr. 55 bis SEQ ID Nr. 58 und/oder funktionelle Varianten davon und/oder diese kodierende Nukleinsäuren oder Varianten davon, und/oder einer ein genanntes Polypeptid oder eine funktionelle Varianten davon oder einer genannte Nukleinsäure oder Varianten davon exprimierenden Zelle zur Herstellung eines Arzneimittels und/oder eines Diagnostikums zur Diagnose, Behandlung und/oder Prävention von Erkrankungen, insbesondere Erkrankungen von Hautzellen, von Wundheilung und/oder deren krankhaften Störungen sowie ihre Verwendung zur Identifizierung von pharmakologisch aktiven Substanzen, gegebenenfalls zusammen oder kombiniert mit geeigneten Zusatz- und/oder Hilfsstoffen, gelöst.

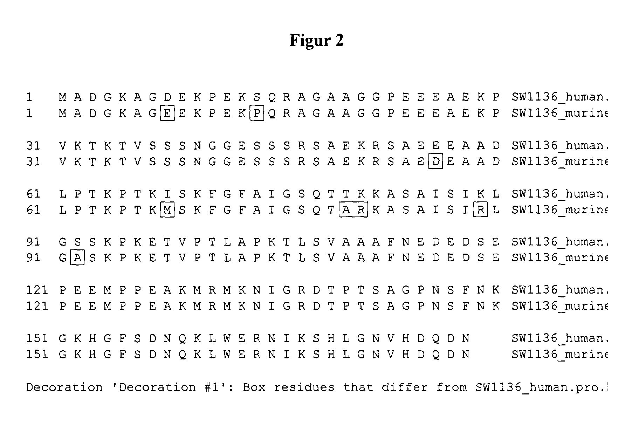

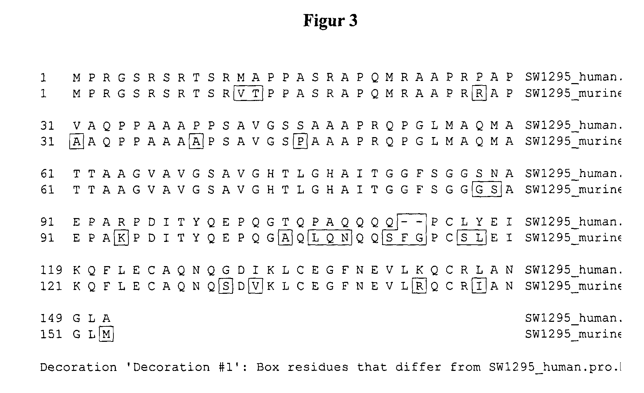

Die genauen biologischen Funktionen der erfindungsgemäß verwendeten Polypeptide der SEQ ID Nr. 55 bis SEQ ID Nr. 58 sind unbekannt. Bei den Untersuchungen im Rahmen dieser Erfindung konnte zum ersten Mal ein Zusammenhang der erfindungsgemäßen Polypeptide mit Erkrankungen, beispielsweise Hauterkrankungen, festgestellt werden. Die Zugangsnummem der erfindungsgemäßen Polypeptidsequenzen und ihrer cDNAs soweit bekannt sind in Tabelle 3 aufgelistet. Die noch nicht in öffentlichen Datenbanken zugänglichen cDNA Sequenzen der Polypeptide von SEQ ID Nr. 55 bis 57 sind unter SEQ ID Nr. 50 bis 52 angefiihrt. Figur 2 und Figur 3 zeigen den Vergleich der menschlichen und murinen Polypeptidsequenzen.

Bei der Analyse der Genexpression während des Wundheilungsprozesses konnten weitere Gene identifiziert werden, deren bereits bekannte und beschriebene Funktionen bisher nicht mit Hauterkrankungen oder Wundheilung, beispielsweise bei einer gestörten Wundheilung, in Verbindung gebracht wurden, deren Regulation aber für den Wundheilungsprozeß essentiell ist und die damit erstmals in kausalen Zusammenhang mit Hauterkrankungen, beispielsweise bei einer gestörten Wundheilung, gebracht wurden. Die Polypeptide dieser Gene gehören nicht zu den bisher bekannten Zielen von Therapien von Hauterkrankungen und/oder der Wundheilung oder deren Störungen, so daß sich aus dieser Erfindung völlig neue Therapieansätze ergeben.

Die Aufgabe der Erfindung wird weiterhin durch die Verwendung mindestens eines Polypeptids gemäß einer der SEQ ID Nr. 1 bis SEQ ID Nr. 20 und SEQ ID Nr. 31 bis SEQ ID Nr. 48 und SEQ ID Nr. 63 bis SEQ ID Nr. 70 und SEQ ID Nr. 80 bis SEQ ID Nr. 82 oder funktionellen Varianten davon und/oder diese kodierende Nukleinsäuren oder Varianten davon, und/oder einer ein genanntes Polypeptid oder funktionelle Varianten davon oder einer genannte Nukleinsäure oder Varianten davon exprimierenden Zelle zur Herstellung eines Arzneimittels und/oder eines Diagnostikums zur Diagnose, Prävention und/oder Behandlung von Erkrankungen von Hautzellen, von Wundheilung und/oder deren krankhaften Störungen sowie ihre Verwendung zur Identifizierung von pharmakologisch aktiven Substanzen, gegebenenfalls zusammen oder kombiniert mit geeigneten Zusatz- und/oder Hilfsstoffen, gelöst.

Erfindungsgemäß können folgende Polypeptide verwendet werden:
- Das tumor susceptibility gene TSG101 aus Maus (SEQ ID Nr. 1) oder Mensch (SEQ ID Nr. 2), das aus WO 97/18333 und US 5,892,016 bekannt ist (Li und Cohen, 1996, Cell 85:319-329; Li et al., 1997, Cell 88: 143-154). Die funktionelle Inaktivierung von TSG101 in Fibroblasten führt zur zellulären Transformation und zur Fähigkeit metastasierende Tumore zu bilden. TSG101-defiziente neoplastische Zellen zeigen Abnormitäten in Mitose-assoziierten Prozessen (Xie et al., 1998, Proc. Natl. Acad. Sci. U.S.A. 95:1595-1600). Zudem wird eine Rolle als transkriptioneller Modulator vermutet (Sun et al., 1999, Cancer 86:689-696). Zusätzlich zu dem Polypeptid aus Mensch gemäß SEQ ID Nr. 2 kann auch die Spleißvariante gemäß SEQ ID Nr. 82 (SWISSProt: Q99816) verwendet werden.
- Das aus US 5,905,023, US 5,801,001, US 5,470,970 und WO 94/05804 bekannte Tumorsuppressor-Protein Maspin aus Maus (SEQ ID Nr. 3) oder Mensch (SEQ ID Nr. 4) (Zou et al., 1994, Science 263, 526-529). MASPIN ist ein Serin-Protease-Inhibitor (Zhang et al.,1997, Mol. Med. 3:49-59), der in normalen Brust- und Prostataepithelzellen exprimiert wird (Zhang et al., 1997, Proc. Natl. Acad. Sci. U.S.A. 94:5673-5678) und eine wesentliche Rolle bei der Entwicklung der Brustdrüsen spielt (Zhang et al., 1999, Dev. Biol. 215:278-287).
- Der RNA-Polymerase I Terminationsfaktor TTF-I aus Maus (SEQ ID Nr. 5) oder Mensch (SEQ ID Nr. 6) (Evers und Grummt, 1995, Proc. Natl. Acad. Sci. U.S.A. 92:5827-5831). Das Protein vermittelt die Termination der Transkription ribosomaler Gene (Kuhn et al., 1990, Nature 344:559-62) sowie die transkriptionelle Aktivierung der ribosomalen Gene im Chromatin (Langst et al., 1998, EMBO J. 17:3135-45).
- Das aus WO 91/02077 und US 7,745,381 bekannte B-raf Protoonkogen aus Maus (SEQ ID Nr. 7) oder Mensch (SEQ ID Nr. 8) (Miki et al., 1991, Proc. Natl. Acad. Sci. U.S.A. 88:5167-5171; Stephens et al., 1992, Mol. Cell. Biol. 12:3733-3742). Das B-raf Protoonkogen gehört zur Raf-Familie, die Serin/Threonin-Proteinkinasen umfaßt, die die Stimulation von Wachstumsfaktorrezeptoren und die Aktivierung von mitogen-aktivierten Proteinkinase koppeln (Mason et al., 1999, EMBO J. 18:2137-48). U.a. kann B-Raf Apoptose inhibieren (Erhardt et al., 1999, Mol. Cell. Biol. 19:5308-15).
- Das aus US 4,716,148 und US 4,659,694 bekannte Prothymosin alpha aus Maus (SEQ ID Nr. 9) oder Mensch (SEQ ID Nr. 10) (Schmidt und Werner D.; 1991, Biochim. Biophys. Acta 1088:442-444, Eschenfeldt und Berger, 1986, Proc. Natl. Acad. Sci. U.S.A. 83:9403-9407). Es kodiert ein kleines azidisches Kernprotein, das eine Rolle bei der Proliferation von Zellen spielt (Tao et al., 1999, J. Cell. Physiol. 178:154-63).
- Der GOLGI 4-TRANSMEMBRANE SPANNING TRANSPORTER oder MTP (mouse transporter protein) aus Maus (SEQ ID Nr. 11) oder Mensch (SEQ ID Nr. 12) (Hogue et al., 1996, J. Biol. Chem. 271:9801-9808; Nagase et al., 1995, DNA Res. 2:37-43). Es ist ein stark konserviertes Membranprotein, das in Säugerzellen in Lysosomen und Endosomen lokalisiert ist. Das Protein ist fiir die subzelluläre Verteilung verschiedener kleiner hydrophober Moleküle verantwortlich und trägt zur Sensitivität bzw. Resistenz von Säugerzellen gegenüber bestimmten Wirkstoffen bei (Hogue et al., 1999, J. Biol. Chem. 274:12877-82). Für das Maus-homolog wird auch ein alternatives, um 89 Aminosäuren C-terminal verkürztes Polypeptid postuliert, das vermutlich durch einen alternativen Translationsinitiationsort entsteht (siehe SwissProt: Q60961). Auch dieses murine Polypeptid kann erfindungsgemäß verwendet werden.
- CCR-1 aus Maus (SEQ ID Nr. 13) oder Mensch (SEQ ID Nr. 14) (Post et al., 1995, J. Immunol. 155:5299-5305), das ein Eosinophil-Rezeptor für das CC Chemokin Eotaxin ist (Gao et al., 1996, Biochem. Biophys. Res. Comm. 223:679-84). CCR-1 wird in Herz, Milz und Lunge exprimiert (Gao und Murphy, 1995, Genomics 29:294-96).
- Das Nukleosomen-Bindeprotein HMG-14 aus Maus (SEQ ID Nr. 15) oder Mensch (SEQ ID Nr. 16) (Landsman und Bustin, 1990, Nucleic Acids Res. 18:5311-5311; Landsman et al., 1986, J. Biol. Chem. 261:16082-16086), das höher geordnete Chromatinstrukturen öffnet und somit das Transkriptions-und Replikationspotential von Chromatin erhöht (Herrera et al., 1999, Mol. Cell. Biol. 19:3466-73).
- Das Split hand/foot deleted 1 aus Maus (SEQ ID Nr. 17) oder Mensch (SEQ ID Nr. 18), das ein Kandidaten-Gen für die autosomal dominante Form der "split hand/split foot malformation disorder" ist. Es wird in den Knospen der Gliedmaßen, in der "cranofacial primordia" und in der Haut exprimiert (Crackower et al., 1996, Hum. Mol. Genet. 5:571-9).
- Der Orphan Receptor TAK1 oder TR4 aus Maus (SEQ ID Nr. 19) oder Mensch (SEQ ID Nr. 20) (Hirose et al., 1995, Gene 163:239-242, Hirose et al., 1994, Mol. Endocrinol. 8:1667-1680), der zur Superfamilie der nukleären Hormonrezeptoren gehört (Hirose et al., 1994, Mol. Endocrinol. 8:1667-80). Als Homodimer beeinflußt TR4 eine Vielzahl von Signaltransduktionswegen, darunter die von Retinolsäure, Thyreoidhormon, Vitamin D3 und "ciliary neutrophic factor". Außerdem bildet TR4 Heterodimere mit dem Androgenrezeptor (Lee et al., 1999, Proc. Natl. Acad. Sci. U.S.A. 96:14724-9).
- Das aus WO 95/14772 bekannte BAF57 aus Maus (SEQ ID Nr. 31) oder Mensch (SEQ ID Nr. 32), das Bestandteil der Chromatin-remodellierenden SWI/SNF-Komplexe höherer Eukaryonten ist (Wang et al., 1998, Proc. Natl. Acad. Sci. U.S.A. 95:492-498). Die SWI/SNF-Komplexe regulieren die Transkription spezifischer Gene indem sie die Chromatin-vermittelte Repression der Transkription aufheben (Wolffe und Guschin, 2000, J. Struct. Biol. 129:102-122). Außerdem wurde eine Rolle bei der Umstellung der Expression von fötalem zu adultem Globin in Mäusen gezeigt (Armstrong et al., 1999, Proc. Natl. Acad. Sci. U.S.A. 96:349-54). Zusätzlich zu den bekannten Polypeptiden aus Mensch und Maus kann auch das in dieser Arbeit erstmals erwähnte eng verwandte humane Polypeptid mit signifikant abweichender Sequenz (SEQ ID Nr. 80) verwendet werden. Die das Polypeptid gemäß SEQ ID Nr. 80 kodierende cDNA ist unter SEQ ID Nr. 83 angegeben.
- Das aus US 7,935,311 bekannte Epidermal Growth Factor Receptor Kinase Substrat EPS 8 aus Maus (SEQ ID Nr. 33) oder Mensch (SEQ ID Nr. 34), das EGF-abhängige mitogene Signale verstärkt (Wong et al., 1994, Oncogene 9:3057-3061; Fazioli et al., 1993, EMBO J. 12:3799-3808). Sowohl Überexpression als auch konstitutive Phosphorylierung von EPS8 wurden im Zusammenhang mit Tumorentwicklung beschrieben (Matoskova et al., 1995, Mol. Cell Biol. 15:3805-3812).
- KIAA1247 aus Mensch (SEQ. ID Nr. 36), das laut WO 99/34004 als Marker-Protein bei Krebsmetastasen eingesetzt werden kann. Außerdem ist ein KIAA1247 Homolog aus der Ratte aus WO 98/53071 als Protein bekannt, dessen Expression in verletzten oder regenerierenden Geweben, vor allem in Nierengewebe der Ratte, induziert wird. Zusätzlich zu dem bekannten Polypeptid aus Mensch können auch das in dieser Arbeit erstmals erwähnte Polypeptid aus der Maus (SEQ. ID Nr. 35) sowie zwei in dieser Arbeit erstmals erwähnten Spleißvarianten des humanen Gens, die für verkürzte Varianten des Polypeptids gemäß SEQ ID Nr. 36 kodieren, verwendet werden. Den den beiden letztgenannten Polypeptid-Isoformen, die aus differentiellem Spleißen resultieren, fehlen die Aminosäuren Nummer 664 bis 681 bzw. die Aminosäuren 652 bis 654 und 664 bis 681 des Polypeptids gemäß SEQ ID Nr. 36. Weiterhin können Varianten der erfindungsgemäß verwendbaren KIAA1247 Polypeptide verwendet werden, die sich durch ein alternatives Translationsinitiations-ATG Kodon ergeben. Beispiele solcher Varianten sind in WO 00/73454 und WO 00/58473 offenbart.
- Der aus US 5,948,626, US 5,663,059 und US 5,811,520 bekannte Phospholipase Inhibitor GIPL aus Mensch (SEQ ID Nr. 38). Zusätzlich zu dem bekannten Polypeptid aus Mensch können auch das in dieser Arbeit erstmals erwähnte Polypeptid aus der Maus (SEQ ID Nr. 37) und die in dieser Arbeit erstmals erwähnten eng verwandten humanen Polypeptide mit signifikant abweichender Sequenz (SEQ ID Nr. 45 und SEQ ID Nr. 81) verwendet werden. Die das Polypeptid gemäß SEQ ID Nr. 81 kodierende cDNA ist unter SEQ ID Nr. 84 angegeben.
- Das aus WO 95/28497 bekannte EAT/MCL-1 aus Maus (SEQ ID Nr. 39) oder Mensch (SEQ ID Nr. 40), das in zahlreichen Geweben exprimiert wird (Krajewski et al., 1995, Am. J. Pathol. 146:1309-19) und eine Rolle bei cutanen malignen Melanomen spielt (Tang et al., 1998, Clin. Cancer Res. 4:1865-71).
- Das aus US 5,958,690 bekannte TSC-22 (TGF beta-stimulated clone 22 gene) aus Mensch (SEQ ID Nr. 42) und Maus (SEQ ID Nr. 41) (Jay et al., 1996, Biochem. Biophys. Res. Commun. 222:821-826; Shibanuma et al., 1992, J. Biol. Chem. 267: 10219-10224), das zu der Familie der "Leucin-Zipper" Transkriptionsfaktoren gehört (Kester et al., 1999, J. Biol. Chem. 274:27439-47). Die Transkription von TSC-22 wird durch verschiedene Stimuli wie Wachstumsinhibitoren induziert (Kester et al., 1999, J. Biol. Chem. 274:27439-47). Außerdem wurde während der Entwicklung des Maus-Embryos eine verstärkte Expression von TSC-22 an Orten beobachtet, an denen Mesenchym-Epithel-Interaktionen stattfinden (Dohrmann et al., 1999, Mech. Dev. 84:147-51).
- Das aus JP 100 57 065 und US 5,837,537 bekannte gamma-Sarcoglycan aus Mensch (SEQ ID Nr. 44) oder Maus (SEQ ID Nr. 43) (Noguchi et al., 1995, Science 270:819-822; Noguchi et al., 1999, Biochem. Biophys. Res, Commun. 262:88-93). Gamma-Sarcoglycan ist eine Komponente des Sarcoglycan-Komplexes, der wiederum ein Subkomplex des Dystrophin-Gycoprotein-Komplexes ist. Dieser stellt eine Verbindung zwischen der extrazellulären Matrix und dem Aktin-Cytoskelett her (Hack et al., 2000, Microsc. Res. Tech. 48:167-80). Mutationen des gamma-Sarcoglycan wurden als primärer genetischer Defekt einer muskulären Dystrophie (SCARMD) beschrieben (Noguchi et al., 1995, Science 270:819-822).
- Der aus WO 99/38882, WO 88/09384, DE 372 4 581, JP 012 02 287, JP 010 74 988 und US 5,212,297 bekannte Cystein Proteinase Inhibitor Cystatin C aus Mensch (SEQ ID Nr. 47) oder Maus (SEQ ID Nr. 46) (Abrahamson et al., 1987, FEBS Lett. 216:229-233; Solem et al., 1990, Biochem. Biophys. Res. Commun. 172:945-951). Cystein Protease Inhibitoren spielen eine Rolle bei Entzündungskrankheiten, wie z.B. Rheuma (Lenarcic et al., 1988, Biol. Chem. Hoppe Seyler 369 (Suppl.):257-261 und bei vaskulären Krankheiten (Shi et al., 1999, J. Clin. Invest. 104:1191-1197). Zusätzlich zu der bekannten Polypeptidvariante aus der Maus (SEQ ID Nr. 46) (Solem et al., 1990, Biochem. Biophys. Res. Commun. 172:945-951) kann auch das in dieser Arbeit erstmals erwähnte eng verwandte Polypeptid mit abweichender Sequenz verwendet werden (SEQ ID Nr. 48).
- Die Tyrosinkinase Fer aus Maus (SEQ ID Nr. 63) oder Mensch (SEQ ID Nr. 64) (SwissProt: P70451; Hao et al., 1989, Mol. Cell. Biol. 9:1587-1593), die sowohl im Kern als auch im Zytoplasma lokalisiert ist (Hao et al., 1991, Mol. Cell. Biol. 11:1180-1183). Für Fer wurde sowohl eine Rolle bei der Zell-Zell-Adhäsion (Rosato et al., 1998, Mol. Cell. Biol. 18:5762-5770) als auch eine Rolle als Proto-Onkogen (Morris et al., 1990, Cytogenet. Cell. Genet. 53:196-200) postuliert.
- Das aus WO 99/28473, WO 96/34891 und WO 98/14582 bekannte C-C Cytokin MRP-3 (Macrophage Inflammatory Protein 3) aus Maus (SEQ ID Nr. 65) oder Mensch (SEQ ID Nr. 66), das auch C10, MPIF-1 (Myeloid Progenitor Inhibitory Factor-1), CK-beta-8 oder Small Inducible Cytokine A23 genannt wird (Orlofsky et al., 1991, Cell Regul. 2:403-412; Li und Ruben, 1996, US 5,504,003). Bei chronischer Bauchfellentzündung wurde eine hohe MRP-3-Expression in Makrophagen beobachtet (Wu et al., 1999, Cytokine 11:523-30). Als typisches C-C Cytokin ist MRP-3 ein Chemoattraktant für Leukozyten (Haelens et al., 1996, Immunobiology 195:499-521), es wirkt aber auch auf Osteoclasten (Votta et al., 2000, J. Cell Physiol. 183:196-207). Außerdem wurde beobachtet, daß MRP-3 mRNA nicht signifikant durch Stimuli, die mit Wundheilung im Zusammenhang stehen, hochreguliert wird (Orlofsky et al., Cell Regul., 1991, 2:403-412).
- Die Nikotinamid N-Methyltransferase NNMT aus Maus (SEQ ID Nr. 67) oder Mensch (SEQ ID Nr. 68) (Aksoy et al., 1994, J. Biol. Chem. 269:14835-14840; Yan et al., 1997, Biochem. Pharmacol. 54:1139-1149), die den Methyltransfer von S-Adenosylmethionin auf Nikotinamid katalysiert. Es existieren Hinweise, daß NNMT das Wachstum von Leberzellen regulieren kann (Seifert et al., 1984, Biochim. Biophys. Acta 801:259-64). Zudem wurde eine Rolle des Enzyms bei Leberkrebs vorgeschlagen (Hoshino et al., 1982, Biochim. Biophys. Acta 719:518-526).
- Die Ubiquitin-Protein Ligase UBC9 aus Maus (SEQ ID Nr. 69) oder Mensch (SEQ ID Nr. 70) (Yasugi und Howley; 1996, Nucleic Acids Res. 24:2005-2010; SwissProt: P50550), die eine wichtige Komponente des Proteosomvermittelten Proteinabbaus darstellt (Hershko und Ciechanover, 1998, Annu. Rev. Biochem. 67:425-479). Der Ubiqitin-abhängige Proteinabbau spielt eine Rolle in verschiedensten Prozessen wie Zellzykluskontrolle, Signaltransduktion oder der Immunantwort. Es existieren Hinweise, daß UBC9 eine Rolle bei beschleunigter Alterung spielt (Kawabe et al., 2000, J. Biol. Chem.). Zudem katalysiert UBC9 die Sumoylierung von p53 und aktiviert somit seine Funktion als Transkriptionsfaktor (Rodriguez et al., 1999, EMBO J. 18:6455-61):

Für keines dieser Polypeptide, deren kodierende Nukleinsäure oder der beschriebenen cDNA wurde bisher ein Zusammenhang mit Hauterkrankungen oder Wundheilung, beispielsweise bei einer gestörten Wundheilung, beschrieben oder nahegelegt. Es war daher unerwartet, daß diese Verbindungen erfindungsgemäß verwendet werden können. Die Zugangsnummern der erfindungsgemäßen Polypeptide und ihrer cDNAs sind in Tabelle 4 aufgeführt. Die noch nicht in öffentlichen Datenbanken zugänglichen cDNA Sequenzen der Polypeptide der SEQ ID Nr. 31, der SEQ ID Nr. 35, der SEQ ID Nr. 37, der SEQ ID Nr. 80 und SEQ ID Nr. 81 sind unter SEQ ID Nr. 49 und unter SEQ ID Nr. 53 bis SEQ ID Nr. 54 und unter SEQ ID Nr. 83 bis SEQ ID Nr. 84 angeführt.

Bei der Analyse der Genexpression während des Wundheilungsprozesses konnten zusätzlich Gene identifiziert werden, deren bereits bekannte und beschriebene Funktionen bisher nicht mit der Wundheilung in Verbindung gebracht wurden, deren Regulation aber fiir den Wundheilungsprozeß essentiell ist und die damit erstmals in kausalen Zusammenhang mit der Wundheilung gebracht wurden. Die Polypeptide dieser Gene gehören nicht zu den bisher bekannten Zielen von Therapien in Zusammenhang mit der krankhaften Veränderung der Wundheilung, so daß sich aus dieser Erfindung völlig neue Therapieansätze ergeben.

Die Aufgabe der Erfindung wird daher zusätzlich durch die Verwendung mindestens eines Polypeptids gemäß einer der SEQ ID Nr. 21 bis SEQ ID Nr. 26 und SEQ ID Nr. 29 bis SEQ ID Nr. 30 und SEQ ID Nr. 71 bis SEQ ID Nr. 73 oder funktionellen Varianten davon und/oder diese kodierende Nukleinsäuren oder Varianten davon, und/oder einer ein genanntes Polypeptid oder funktionelle Varianten davon oder einer genannte Nukleinsäure oder Varianten davon exprimierende Zelle zur Herstellung eines Arzneimittels und/oder eines Diagnostikums zur Diagnose, Prävention und/oder Behandlung bei der Wundheilung und/oder deren krankhaften Störungen, sowie zur Identifizierung von pharmakologisch aktiven Substanzen gelöst.

Erfindungsgemäß können folgende Polypeptide verwendet werden:
- Das aus WO 95/04158, WO 99/12968 und EP 0 488 900 bekannte Monocyte Chemotactic Protein-3, MCP-3 aus Maus (SEQ ID Nr. 21) oder Mensch (SEQ ID Nr. 22)(Kulmburg et al., 1992, J. Exp. Med. 176:1773-1778; Minty et al., 1993, Eur. Cytokine Netw. 4:99-110). MCP-3 ist ein CC-Chemokin, das der Chemoattraktion und Aktivierung von Monozyten, T-Lymphozyten, Eosinophilen und Basophilen Granulozyten, Killerzellen und dendritischen Zellen dient. Die Aktivierung der Zielzellen erfolgt über die Chemokinrezeptoren CCR2 und CCR3 (Wang et al., 2000, Biochim. Biophys. Acta 1500:41-8). MCP-3 spielt eine Rolle bei allergischen Reaktionen in der Haut (Ying et al., 1999, J. Immunol. 163:3976-84).
- Die aus EP 0 475 746 und WO 98/47923 bekannte alpha-Kette des heterodimeren Interleukin-5-Rezeptors aus Maus (SEQ ID Nr. 23) oder Mensch (SEQ ID Nr. 24) (Takaki 1990, EMBO J. 9:4367-4374, Tavernier et al., 1992, Proc. Natl. Acad. Sci. U.S.A. 89:7041-7045). Sie vermittelt die spezifische Bindung des Liganden Interleukin-5 (Van Ostade et al., 1999, Eur. J. Biochem. 259:954-60) und wird auf der Zellmembran von Eosinophilen exprimiert (Weltman und Karim, 1998, Allergy Asthma Proc. 19:257-61). Eine Rolle des Interleukin-5 Rezeptors bei der Atopischen Dermatitis wurde beschrieben (Taha et al., 1998, J. Allergy Clin. Immunol. 102:245-50). Interleukin-5 spielt eine wesentliche Rolle in der Differenzierung, Proliferation und funktionellen Aktivierung von Eosinophilen (Iwama et al.,1999, Mol. Cell. Biol. 19:3940-50) und, im Gegensatz zum hier beschriebenen Rezeptor, wurde eine Funktion von Interleukin-5 in der Wundheilung beschrieben (Yang et al., 1997, Am J Pathol 151:813-9).
- Das aus WO 99/04265 bekannte integrale Membranprotein Dad1 aus Maus (SEQ ID Nr. 25) oder Mensch (SEQ ID Nr. 26) (Nakashima et al., 1993, Mol. Cell. Biol. 13:6367-6374; Apte et al., 1995, FEBS Lett. 363:304-306). Es ist Bestandteil des Oligosaccharyltransferase-Enzymkomplexes, der die N-Glukosylierung einleitet (Sanjay et al., 1998, J. Biol. Chem., 273:26094-9). Dad1 spielt eine Rolle bei der Verhinderung von Apoptose in bestimmten Zelltypen (Hong et al., 1999, J. Immunol. 163:1888-93) und in Keloiden (Sayah et al., 1999, J. Surg. Res. 87: 209-16).
- Das aus EP 0 905 240, EP 0 905 241, WO 98/02459, EP 0 906 954, WO 95/04158, WO 99/12968, WO 97/25427 bekannte MCP-2 (C-C chemokine monocyte chemotactic protein 2) aus Mensch (SEQ ID Nr. 30) oder Maus (SEQ ID Nr. 29) (van Coillie et al., 1997, Genomics 40:323-331; EMBL: AB023418). MCP-2 gehört zu den C-C Chemokinen und wirkt die als Chemoattraktant für verschiedene Zellen wie Makrophagen, Basophile und Eosinophile (Taub et al., 1995, J. Clin. Invest. 95:1370-6; Proost et al., 1996, J. Leukoc. Biol. 59:67-74). MCP-2 ist ein Signalmolekül, das bei Entzündungsprozessen die gerichtete Migration von T-Zellen und Monozyten stimuliert und diese rekrutiert (Taub et al., 1995, J. Clin. Invest. 95:1370-6). Zusätzlich zu der bekannten Polypeptidvariante aus dem Mensch (SEQ ID Nr. 31)(van Coillie et al., 1997, Genomics 40:323-331) kann auch das in dieser Arbeit erstmals erwähnte eng verwandte Polypeptid mit abweichender Sequenz verwendet werden (SEQ ID Nr. 71).
- Die aus WO9633278-A1 bekannte Cystein-Protease Cathepsin C aus Maus (SEQ ID Nr. 72) oder Mensch (SEQ ID Nr. 73)(Paris et al., 1995, FEBS Lett. 369:326-330; McGuire et al., 1997, Biochim. Biophys. Acta 1351:267-273), die in Lysosomen verschiedener Zellen vorkommt (Turk et al., 1997, Biol. Chem. 378:141-150). Cathepsin C spielt eine wichtige Rolle bei der Aktivierung von Granzym A und B und somit bei der Induktion von Apoptose durch cytotoxische Lymphozyten (Pham und Ley, 1999, Proc. Natl. Acad. Sci. U.S.A. 96:8627-8632). Außerdem wurde beobachtet, daß loss-of-function Mutationen im Cathepsin C Gen zu palmoplanarer Keratose und Periodonditis führen (Hart et al., 1999, J. Med. Genet. 36:881-887; Toomes et al., 1999, Nat. Genet. 23:421-424).

Für keines dieser Polypeptide oder diese kodierende Nukleinsäuren wurde bisher ein Zusammenhang mit der Wundheilung beschrieben oder nahegelegt. Es war daher unerwartet, daß diese Polypeptide erfindungsgemäß verwendet werden können. Die Zugangsnummern der erfindungsgemäßen Polypeptide und ihrer cDNAs sind in Tabelle 5 aufgeführt.

Allgemein ist die Analyse von differentiell exprimierten Genen in Geweben mit deutlich mehr Fehlern in Form von falsch positiven Klone behaftet, als die Analyse von Zellkultursystemen. Dieses Problem kann nicht durch die Verwendung eines definierten Zellkultursystems umgangen werden, da bestehende, einfache Zellkultursysteme die Komplexität der Wundheilungsprozesse im Gewebe nicht ausreichend simulieren können.

Das Problem besteht insbesondere bei der Haut, die aus einer Vielzahl von verschiedenen Zelltypen besteht. Darüber hinaus ist der Prozeß der Wundheilung ein höchst komplizierter Vorgang, der zeitliche und räumliche Änderungen zellulärer Vorgänge, wie Proliferation und Differenzierung bei den unterschiedlichen Zelltypen umfaßt. Der Ansatz, nicht nur das komplexe Zellsystem Haut, sondern darüber hinaus den physiologischen Prozeß der Wundheilung und sogar verschiedenste Wundheilungsstadien auf der Ebene differentiell exprimierter Gene zu untersuchen ist daher für einen Fachmann keine erfolgversprechende Strategie. Der Erfolg der Rasteruntersuchung war aufgrund dieser Schwierigkeiten wesentlich von der Wahl der experimentellen Parameter abhängig. Während die verwendeten Methoden (z.B. subtraktiver Hybridisierung) Standardmethoden sind, ist die Rasteruntersuchungs- und Verifizierungsstrategie durch die durchdachte und definierte Wahl von Parametern an sich bereits erfinderisch. Beispielsweise ist der Zeitpunkt der Biopsienahme kritisch für den Erfolg der Rasteruntersuchung: Wundheilungsstörungen und Hautkrankheiten liegen häufig Störungen bei der Zellproliferation und Zellmigration zugrunde. Diese Prozesse werden einen Tag nach der Verwundung initiiert, weshalb eine Analyse der molekularen Prozesse vor diesem Zeitpunkt wenig Aufschluß über die Vorgänge liefern würde, die für eine normal verlaufende Wundheilung essentiell sind. Andererseits verändert sich im Verlauf der Wundheilung später als einen Tag nach der Verwundung die Zusammensetzung der Zelltypen in der Wunde stark. Dies kann dazu führen, daß eine differentielle Expression eines bestimmten Gens in der Wunde gemessen wird, die nicht auf veränderter Expression in den Zellen beruht, sondern nur auf der unterschiedlichen Zellzusammensetzung. Dies verdeutlicht, daß die Wahl des Tages der Biopsienahme entscheidend den Erfolg der Rasteruntersuchung beeinflußte. Trotz der definierten Parameter wurde eine Überrepräsentation von Genen beobachtet, die während der Wundheilung differentiell exprimiert werden, die aber für die Verwendung in der Wundheilung oder bei Hautkrankheiten ungeeignet sind. Diese Gene umfassen beispielsweise Gene, die fiir Enzyme des Primärstoffwechsels, wie Glykolyse, Citratzyklus, Glukoneogenese und Atmungskette kodieren, aber auch Gene, die für ribosomale Proteine kodieren, z.B. L41 und S20. Nur eine vergleichsweise kleine Zahl an Genen konnte als geeignet identifiziert werden. Daher war eine Identifizierung der erfindungsgemäß verwendbaren Gene als wundheilungsrelevante Gene überraschend.

Zudem gibt es enorme Variabilitäten des Wundzustands zum Zeitpunkt einer möglichen Biopsie des Patienten beim Erstkontakt mit dem Arzt. Daher wurde zur Identifikation der vorangehend beschriebenen Nukleinsäuren ein Tiermodell verwendet. Es wurden BALB/c Mäuse verwundet und zu verschiedenen Zeitpunkten Wundbiopsien entnommen. Dieses Verfahren hat den Vorteil, das sich die Randbedingungen wie genetischer Hintergrund, Art der Wunde, Zeitpunkt der Biopsie etc. exakt kontrollieren lassen und so erst eine reproduzierbare Analyse der Genexpression erlauben. Selbst unter den definierten Maus-Bedingungen ergeben sich weitere methodische Probleme wie Redundanz der analysierten Klone und Unterrepräsentation von schwach exprimierten Genen, die die Identifikation von relevanten Genen erschweren.

Die Nukleinsäuren der erfindungsgemäß verwendbaren Polypeptide wurden aus cDNA Bibliotheken isoliert, die aus intakter und verwundeter Haut hergestellt wurden. Dabei wurden die cDNAs ausgewählt, die unterschiedliche Häufigkeiten in gut heilenden im Vergleich zu schlecht heilenden Wunden aufwiesen (Beispiele 1 und 3). Dies geschah beispielsweise mit Hilfe von subtraktiver Hybridisierung (Diatchenko et al., 1996, Proc. Natl. Acad. Sci. USA 93: 6025-30) und/oder mit dem vergleichenden Auszählen von Klonen in cDNA Bibliotheken mittels Analyse von Restriktionsfragmentmustern (Halle et al., 1999, EP 0965642A1) und/oder mit Hilfe von "Differential Display RT-PCR" (Liang et al., 1992, Cancer Res. 52: 6996-6998; Liang und Pardee, 1992, Science 257: 967-971; Prashar und Weissman, 1996, Proc. Natl. Acad. Sci. U.S.A. 93:659-663). Die so ausgewählten cDNAs entstammen Genen, die bei Wundheilungsstörungen entweder stärker oder schwächer exprimiert werden als bei normal verlaufender Wundheilung.

Nach der primären Identifikation eines Gens ist es notwendig, die wundheilungsspezifische Expression durch eine weitere Methode zu bestätigen. Dies erfolgte mit Hilfe von sogenannten "Reverse Northern Blots" und "TaqMan Analyse". Mit diesen Methoden wurde die Menge an mRNA in Geweben aus verschiedenen Wundheilungszuständen und in Hauterkrankungen (Psoriasis) bestimmt bzw. hauterkrankungsspezifische lokale Änderungen des Expressionsmusters in Biopsien nachgewiesen (Beispiele 2, 4 bis 7).

Bei der vorliegenden Analyse der Genexpression wurden während des Wundheilungsprozesses neben Genen, deren Funktion bisher gänzlich unbekannt war, auch Gene identifiziert, die bisher nicht mit Wundheilungsstörungen in Verbindung gebracht wurden. Von zwei der bekannten Gene wurden weiterhin neuartige Varianten mit Sequenzen identifiziert, die signifikant von den bisher veröffentlichten und/oder patentierten Sequenzen abweicht.

Von dem bisher nicht mit Wundheilungsstörungen in Zusammenhang gebrachten Teil der identifizierten Gene war bisher bekannt, daß sie eine Funktion bei der Proliferation (Tsg101: Xie et al., 1998, Proc. Natl. Acad. Sci. U.S.A. 95:1595-1600; MASPIN: Sager et al., 1997, Adv. Exp. Med. Biol. 425:77-88; B-Raf: Mason et al., 1999, EMBO J. 18:2137-48; Ikawa et al., 1988, Mol. Cell Biol. 8:2651-2654; Prothymosin alpha: Tao et al., 1999, J. Cell. Physiol. 178:154-163; Eps8: Wong et al., 1994, Oncogene 9:3057-3061; KIAA1247: WO 99/34004; EAT/MCL-1: Tang et al., 1998, Clin. Cancer Res. 4:1865-1871; TSC-22: Kester et al., 1999, J. Biol. Chem. 274:27439-47; Fer: Morris et al., 1990, Cytogenet. Cell. Genet. 53:196-200), Differenzierung (MASPIN: Zhang et al., 1999, Dev. Biol. 215:278-87; Split hand/foot deleted 1: Crackower et al., 1996, Hum. Mol. Genet. 5:571-9), Zell-Migration (MRP-3: Haelens et al., 1996, Immunobiology 195:499-521; MCP-3: Taub et al., 1995, J. Clin. Invest. 95:1370-6; MCP-2: Taub et al., 1995, J. Clin. Invest. 95:1370-6) und/oder Apoptose (B-Raf: Erhardt et al., 1999, Mol. Cell. Biol. 19:5308-15) haben. Diese Gene wurden bisher jedoch nicht mit Wundheilung in Verbindung gebracht.

Zusätzlich zu den bekannten Polypeptiden von Mensch Phospholipase Inhibitor GIPL (US 5,948,626), MCP-2 (van Coillie et al., 1997, Genomics 40: 323-331), BAF57 (WO 95/14772) und Maus Cystatin C (Solem et al., 1990, Biochem. Biophys. Res. Commun. 172:945-951) wurden eng verwandte Polypeptide mit signifikant abweichender Sequenz identifiziert. Von den bekannten Polypeptiden von Mensch Phospholipase GIPL (US 5,948,626) und Mensch KIAA1247 (WO 99/34004) wurden erstmals die Sequenzen des entsprechenden Polypeptids der Maus identifiziert.

Die Polypeptide dieser Gene gehören nicht zu den bisher bekannten Zielen von Therapien von Wundheilungsstörungen, so daß sich aus dieser Erfindung völlig neue Therapieansätze ergeben. Von den restlichen identifizierten Genen existiert noch keine Funktionsbeschreibung (Tabelle 3).

Zur Überprüfung bzw. Generierung von Vollängen cDNA Sequenzen der vorangehend beschriebenen Nukleinsäuren wurden Vollängen-Klone mit Hilfe von Kolonie-Hybridisierung (Sambrook et al., 1989, Molecular cloning: A Laboratory Manual, Cold Spring Harbor, Cold Spring Harbor Laboratory Press, New York, Kapitel 8-10) und/oder PCR basierten Methoden ("RACE", Frohman et al., 1988, Proc. Natl. Acad. Sci. USA 85: 8998-9002, Chenchik et al., 1996, in A Laboratory Guide to RNA: Isolation, Analysis, and Synthesis, Ed. Krieg, Wiley-Liss, Seiten 272-321; "LDPCR", Barnes, 1994, Proc. Natl. Acad. Sci. USA 91: 2216-20) sowohl für die Maus-Gene als auch für die menschlichen Gene generiert und die Sequenz diese Klone bestimmt.

Der Begriff "funktionelle Varianten" eines Polypeptids im Sinne der vorliegenden Erfindung umfaßt Polypeptide, die beispielsweise wie die erfindungsgemäß verwendeten Polypeptide während Erkrankung, insbesondere Hauterkrankungen, oder bei regenerativen Prozessen der Haut, insbesondere jedoch bei Wundheilungsstörungen, reguliert werden. Zu funktionellen Varianten zählen beispielsweise auch Polypeptide, die von einer Nukleinsäure kodiert werden, die aus nichthautspezifischen Gewebe, z.B. Embryonalgewebe, isoliert werden, jedoch nach Expression in einer an der Wundheilung oder Hauterkrankung beteiligten Zelle die bezeichneten Funktionen besitzen.

Funktionelle Varianten im Sinne der vorliegenden Erfindung sind auch Polypeptide, die eine Sequenzhomologie, insbesondere eine Sequenzidentität, von ca. 70%, vorzugsweise ca. 80%, insbesondere ca. 90%, vor allem ca. 95% zu dem Polypeptid mit der Aminosäuresequenz gemäß einer der SEQ ID Nr. 1 bis SEQ ID Nr. 48 und SEQ ID Nr. 55 bis SEQ ID Nr. 58 und SEQ. ID Nr. 63 bis SEQ ID Nr. 73 und SEQ ID Nr. 80 bis SEQ ID Nr. 82 aufweisen. Beispiele solcher funktionellen Varianten, sind demnach die zu einem erfindungsgemäß verwendbaren Polypeptid homologen Polypeptide, die aus anderen Organismen als dem Menschen bzw. der Maus, vorzugsweise aus nicht-menschlichen Säugetieren wie z. B. Affen, Schweinen und Ratten stammen. Andere Beispiele von funktionellen Varianten sind Polypeptide, die durch unterschiedliche Allele des Gens, in verschiedenen Individuen oder in verschiedenen Organen eines Organismus kodiert werden.

Um festzustellen, ob es sich bei einem Polypeptid um eine funktionale Variante eines erfindungsgemäß verwendbaren Polypeptids handelt, kann mittels funktionaler Tests die Aktivität des zu prüfenden Polypeptids mit der Aktivität eines erfindungsgemäß verwendbaren Polypeptids verglichen werden. Unter der Vorraussetzung, daß das zu prüfende Polypeptid die Anforderungen an eine funktionelle Variante auf der Ebene der Sequenzidentität erfüllt, handelt es sich bei dem zu prüfenden Polypeptid dann um eine funktionelle Variante, wenn dessen Aktivität im funktionalen Test mit der des erfindungsgemäß verwendbaren Polypeptids ähnlich oder identisch ausfällt.

Diese funktionalen Tests umfassen beispielsweise die Applikation eines Expressionsvektors, der eine das zu prüfende Polypeptid kodierende Nukleinsäure enthält, oder die Applikation des zu prüfende Polypeptids selbst oder eines gegen das zu prüfende Polypeptid gerichteten Antikörpers oder eines Antisense-Oligonukleotids in Wunden. Nach Inkubation, beispielsweise eines Expressionsvektors wird der Fortgang der Wundheilung bei Gabe der unterschiedlichen Expressionsvektoren enthaltend entweder eine das zu prüfende Polypeptid kodierende Nukleinsäure oder erfindungsgemäß verwendbares Polypeptid kodierende Nukleinsäure oder ohne Insert verglichen. Diese funktionalen Tests können beispielsweise auch auf die Aktivität des zu prüfenden Polypeptids bei Störungen der Wundheilung, zum Beispiel bei schlecht heilenden, Dexamethason behandelten Wunden von Tieren, angewandt werden. So führte beispielsweise die Applikation von PDGF-A und PDGF-B Polypeptiden auf schlecht heilende Wunden von Kaninchen zu vergleichbarer Verbesserung der Wundheilung (J. Surg. Res., 2000, 93:230-236). Vergleichbare Tests können für Hauterkrankungen, beispielsweise Psoriasis durchgeführt werden. Dabei wird ein Expressionsvektor, der eine das zu prüfende Polypeptid kodierende Nukleinsäure enthält, oder das zu prüfende Polypeptid selbst oder ein gegen das zu prüfende Polypeptid gerichteter Antikörper oder ein Antisense-Oligonukleotid beispielsweise auf eine betroffene humane Hautpartie, die auf SCID-Mäuse transplantiert wurde, aufgetragen und der Verlauf der Hauterkrankung, beispielsweise die Heilung, ermittelt. Dies kann beispielsweise im Falle der Psoriasis durch Ermittlung des "PASI-scores" erfolgen.

Unter "Sequenzidentität" versteht man die Übereinstimmung (= % Positive) zwischen zwei Sequenzen, die im Falle von Polypeptidsequenzen beispielsweise mittels BlastP 2.0.1 und im Falle von Polynukleotidsequenzen beispielsweise mit BLASTN 2.0.14 bestimmt wird, wobei der Filter=off gesetzt ist (Altschul et al., 1997, Nucleic Acids Res., 25:3389-3402).

Funktionelle Varianten des Polypeptids können auch Teile des erfindungsgemäß verwendeten Polypeptids mit mindestens 6 Aminosäuren Länge, vorzugsweise mit mindestens 8 Aminosäuren Länge, insbesondere mit mindestens 12 Aminosäuren Länge sein. Umfaßt sind auch N- und/oder C-terminale und/oder interne Deletionen des erfindungsgemäß verwendeten Polypeptids im Bereich von ca. 1-60, vorzugsweise von ca. 1-30, insbesondere von ca. 1-15, vor allem von ca. 1-5 Aminosäuren. Beispielsweise kann die erste Aminosäure Methionin fehlen, ohne daß die Funktion des Polypeptids wesentlich verändert wird.

Der Begriff "kodierende Nukleinsäure" bezieht sich auf eine DNA-Sequenz, die für ein isolierbares bioaktives erfindungsgemäßes Polypeptid oder einen Vorläufer, z.B. mit einer Signalsequenz, kodiert. Das Polypeptid kann durch eine Sequenz in voller Länge oder jeden Teil der kodierenden Sequenz kodiert werden, solange die spezifische, beispielsweise enzymatische Aktivität erhalten bleibt.

Es ist bekannt, daß Veränderungen in der Sequenz der vorangehend beschriebenen Nukleinsäuren vorhanden sein können, zum Beispiel durch die Degenerierung des genetischen Codes, oder daß nicht translatierte Sequenzen beispielsweise am 5'-und/oder 3'-Ende der Nukleinsäure angehängt sein können, ohne daß dessen Aktivität wesentlich verändert wird. Auch können die unten näher beschriebenen Modifikationen durchgeführt werden. Diese Erfindung umfaßt deshalb auch sogenannte "Varianten" der vorangehend beschriebenen Nukleinsäuren.

Unter "Varianten" der Nukleinsäuren sind alle DNA-Sequenzen zu verstehen, die komplementär zu einer DNA-Sequenz sind, die unter stringenten Bedingungen mit der Referenzsequenz hybridisieren und eine im wesentlichen gleiche Aktivität aufweisen wie das von der Referenzsequenz kodierte Polypeptid.

Unter "stringenten Hybridisierungsbedingungen" sind solche Bedingungen zu verstehen, bei denen eine Hybridisierung beispielsweise bei 60°C in 2,5 x SSC-Puffer, gefolgt von mehreren Waschschritten bei 37°C in einer geringeren Pufferkonzentration erfolgt und stabil bleibt.

Varianten der Nukleinsäure können auch Teile der erfindungsgemäß verwendeten Nukleinsäure mit mindestens 8 Nukleotiden Länge, vorzugsweise mit mindestens 18 Nukleotiden Länge, insbesondere mit mindestens 24 Nukleotiden Länge sein, besonders bevorzugt mit mindestens 30 Nukleotiden, vorzugsweise bevorzugt mit mindestens 42 Nukleotiden sein.

Unter dem Begriff "pharmakologisch aktive Substanz" im Sinne der vorliegenden Erfindung sind alle diejenigen Moleküle, Verbindungen und/oder Zusammensetzungen und Stoffgemische zu verstehen, die mit den vorangehend beschriebenen Nukleinsäuren, Polypeptiden, Antikörpern oder Antikörperfragment, gegebenenfalls zusammen mit geeigneten Zusatz- und/oder Hilfsstoffen, unter geeigneten Bedingungen in Wechselwirkung treten können. Mögliche pharmakologisch aktiven Substanzen sind einfache chemische organische oder anorganische Moleküle oder Verbindungen, können aber auch Nukleinsäuren, Peptide, Proteine oder Komplexe davon umfassen. Beispiele pharmakologisch aktiver Substanzen sind organische Moleküle, die Substanzbibliotheken entstammen, die auf ihre pharmakologische Aktivität hin untersucht wurden. Die pharmakologisch aktive Substanzen können aufgrund ihrer Wechselwirkung die Funktion(en) der Nukleinsäuren, Polypeptide oder Antikörper in vivo oder in vitro beeinflussen oder auch nur an die vorangehend beschriebenen Nukleinsäuren, Polypeptiden, Antikörpern oder Antikörperfragmenten binden oder mit ihnen andere Wechselwirkungen kovalenter oder nicht-kovalenter Weise eingehen.

Unter dem Begriff "Regulation" wird beispielsweise die Erhöhung oder Erniedrigung der Polypeptidmenge oder diese kodierende Nukleinsäure verstanden, wobei diese Änderung beispielsweise auf transkriptioneller oder translationeller Ebene stattfinden kann.

Die erfindungsgemäß verwendbaren Polypeptide können weiterhin dadurch gekennzeichnet sein, daß diese synthetisch hergestellt werden. So kann das gesamte Polypeptid oder Teile davon zum Beispiel mit Hilfe der klassischen Synthese (Merrifield-Technik) synthetisiert werden. Teile der erfindungsgemäßen Polypeptide eignen sich insbesondere zur Gewinnung von Antiseren, mit deren Hilfe geeignete Genexpressionsbanken durchsucht werden können, um so zu weiteren funktionelle Varianten des erfindungsgemäßen Polypeptids zu gelangen.

Bevorzugterweise handelt es sich bei den erfindungsgemäß verwendbaren Nukleinsäuren um DNA oder RNA, vorzugsweise eine DNA, insbesondere eine doppelsträngige DNA. Weiterhin kann die Sequenz der Nukleinsäuren dadurch gekennzeichnet sein, daß sie mindestens ein Intron und/oder eine polyA-Sequenz aufweist. Die erfindungsgemäß verwendeten Nukleinsäuren können auch in Form ihrer antisense-Sequenz vorliegen.

Für die Expression des betreffenden Gens ist im allgemeinen eine doppelsträngige DNA bevorzugt, wobei der für das Polypeptid kodierende DNA-Bereich besonders bevorzugt ist. Dieser Bereich beginnt mit dem ersten in einer Kozak Sequenz (Kozak, 1987, Nucleic. Acids Res. 15:8125-48) liegenden Start-Codon (ATG) bis zum nächsten Stop-Codon (TAG, TGA bzw. TAA), das im gleichen Leseraster zum ATG liegt.

Eine weitere erfindungsgemäße Verwendung der Nukleinsäuresequenzen ist die Konstruktion von anti-sense Oligonukleotiden (Zheng und Kemeny, 1995, Clin. Exp. Immunol. 100:380-2; Nellen und Lichtenstein, 1993, Trends Biochem. Sci. 18:419-23; Stein, 1992, Leukemia 6:967-74) und/oder Ribozymen (Amarzguioui, et al. 1998, Cell. Mol. Life Sci. 54:1175-202; Vaish, et al., 1998, Nucleic Acids Res. 26:5237-42; Persidis, 1997, Nat. Biotechnol. 15:921-2; Couture und Stinchcomb, 1996, Trends Genet. 12:510-5). Mit anti-sense Oligonukleotiden kann man die Stabilität von Nukleinsäuren verringert werden und/oder die Translation von Nukleinsäuren inhibiert werden. So kann durch die erfindungsgemäße Verwendung der Nukleinsäuresequenzen beispielsweise die Expression der entsprechenden Gene in Zellen sowohl *in vivo* als auch *in vitro* verringert werden. Antisense Oligonukleotide und Ribozyme können sich daher als Therapeutikum eignen. Diese Strategie eignet sich beispielsweise auch für Haut, epidermale und dermale Zellen, insbesondere, wenn die antisense Oligonukleotide mit Liposomen komplexiert werden (Smyth et al., 1997, J. Invest. Dermatol. 108:523-6; White et al., 1999, J. Invest. Dermatol. 112:699-705; White et al., 1999, J. Invest. Dermatol. 112:887-92). Für die Verwendung als Sonde oder als "antisense" Oligonukleotid ist eine einzelsträngige DNA oder RNA bevorzugt.

Weiterhin kann zur Durchführung der Erfindung eine Nukleinsäure verwendet werden, die synthetisch hergestellt worden ist. So kann die erfindungsgemäß verwendete Nukleinsäure beispielsweise chemisch anhand der in den Figuren 4 bis 6 beschriebenen DNA Sequenzen und/oder anhand der in diesen Figuren ebenfalls beschriebenen Proteinsequenzen unter Heranziehen des genetischen Codes z. B. nach der Phosphotriester-Methode synthetisiert werden (siehe z. B. Uhlmann, E. & Peyman, A. (1990) Chemical Reviews, 90, 543-584, No. 4).

Oligonukleotide werden in der Regel schnell durch Endo- oder Exonukleasen, insbesondere durch in der Zelle vorkommende DNasen und RNasen, abgebaut. Deshalb ist es vorteilhaft, die Nukleinsäure zu modifizieren, um sie gegen den Abbau zu stabilisieren, so daß über einen langen Zeitraum eine hohe Konzentration der Nukleinsäure in der Zelle beibehalten wird (Beigelman et al., 1995, Nucleic Acids Res. 23:3989-94; Dudycz, 1995, WO 95/11910; Macadam et al., 1998, WO 98/37240; Reese et al., 1997, WO 97/29116). Typischerweise kann eine solche Stabilisierung durch die Einführung von einer oder mehrerer Internukleotid-Phosphorgruppen oder durch die Einführung einer oder mehrerer Nicht-Phosphor-Internukleotide, erhalten werden.

Geeignete modifizierte Internukleotide sind in Uhlmann und Peymann (1990 Chem. Rev. 90, 544) zusammengefaßt (siehe auch Beigelman et al., 1995 Nucleic Acids Res. 23: 3989-94; Dudycz, 1995, WO 95/11910; Macadam et al., 1998, WO 98/37240; Reese et al., 1997, WO 97/29116). Modifizierte Intemukleotid-Phosphatreste und/oder Nicht-Phosphorbrücken in einer Nukleinsäure, die bei einer der erfindungsgemäßen Verwendungen eingesetzt werden können, enthalten zum Beispiel Methylphosphonat, Phosphorothioat, Phosphoramidat, Phosphorodithioat, Phophatester, während Nicht-Phosphor-Intemukleotid-Analoge, beispielsweise Siloxanbrücken, Carbonatbrücken, Carboxymethylester, Acetamidatbrücken und/oder Thioetherbrücken enthalten. Es ist auch beabsichtigt, daß diese Modifizierung die Haltbarkeit einer pharmazeutischen Zusammensetzung, die bei einer der erfindungsgemäßen Verwendungen eingesetzt werden kann, verbessert.

In einer weiteren Ausführungsform der erfindungsgemäßen Verwendung sind die vorangehend beschriebenen Nukleinsäuren in einem Vektor, vorzugsweise in einem "shuttle" Vektor, Phagemid, Cosmid, Expressionsvektor und/oder gentherapeutisch wirksamen Vektor enthalten. Weiterhin können die vorangehend beschriebenen Nukleinsäuren in "knock-out" Genkonstrukten oder Expressionskassetten enthalten sein.

Vorzugsweise enthält ein gentherapeutisch wirksamer Vektor wund- bzw. hautspezifische regulatorische Sequenzen, die funktionell mit der vorangehend beschriebenen Nukleinsäure verbunden sind.

Die Expressionsvektoren können prokaryotische oder eukaryotische Expressionsvektoren sein. Beispiele für prokaryotische Expressionsvektoren sind für die Expression in *E. coli* z.B. die Vektoren pGEM oder pUC-Derivate und für eukaryotische Expressionsvektoren für die Expression in *Saccharomyces cerevisiae* z. B. die Vektoren p426Met25 oder p426GALl (Mumberg et al. (1994) Nucl. Acids Res., 22, 5767-5768), für die Expression in Insektenzellen z. B. *Baculovirus*Vektoren wie in EP-B1-0 127 839 oder EP-B1-0 549 721 offenbart, und für die Expression in Säugerzellen z. B. die Vektoren Rc/CMV und Rc/RSV oder SV40Vektoren, welche alle allgemein erhältlich sind.

Im allgemeinen enthalten die Expressionsvektoren auch für die jeweilige Zelle geeignete Promotoren, wie z. B. den trp-Promotor für die Expression in *E*. *coli* (siehe z. B. EP-B1-0 154 133), den Met 25, GAL 1 oder ADH2-Promotor für die Expression in Hefen (Russel et al. (1983), J. Biol. Chem. 258, 2674-2682; Mumberg, supra), den Baculovirus-Polyhedrin-Promotor, für die Expression in Insektenzellen (siehe z. 13. EP-B1-0 127 839). Für die Expression in Säugetierzellen sind beispielsweise Promotoren geeignet, die eine konstitutive, regulierbare, gewebsspezifische, zellzyklusspezifische oder metabolischspezifische Expression in eukaryotischen Zellen erlauben. Regulierbare Elemente gemäß der vorliegenden Erfindung sind Promotoren, Aktivatorsequenzen, Enhancer, Silencer und/oder Repressorsequenzen.

Beispiel für geeignete regulierbare Elemente, die konstitutive Expression in Eukaryonten ermöglichen, sind Promotoren, die von der RNA Polymerase III erkannt werden oder virale Promotoren, CMV-Enhancer, CMV-Promotor, SV40Promotor oder LTR-Promotoren z. B. von MMTV (mouse mammary tumour virus; Lee et al. (1981) Nature 214, 228-232) und weitere virale Promotor- und Aktivatorsequenzen, abgeleitet aus beispielsweise HBV, HCV, HSV, HPV, EBV, HTLV oder HIV.

Beispiele für regulierbare Elemente, die regulierbare Expression in Eukaryonten ermöglichen, sind der Tetracyclinoperator in Kombination mit einem entsprechenden Repressor (Gossen M. et al. (1994) Curr. Opin. Biotechnol. 5, 516-20).

Vorzugsweise erfolgt die Expression von wundheilungs- oder hauterkrankungsrelevanten Genen unter der Kontrolle von gewebespezifischen Promotoren, wobei hautspezifische Promotoren wie beispielsweise der humane K10 Promotor (Bailleul et al., 1990. Cell 62: 697-708), der humane K14 Promotor (Vassar et al., 1989, Proc. Natl. Acad. Sci. USA 86: 1563-67) oder der bovine Cytokeratin IV Promotor (Fuchs et al., 1988; The biology of wool and hair (Hrsg.: G.E. Rogers, et al.), S. 287-309. Chapman und Hall, London/New York) besonders zu bevorzugen sind.

Weitere Beispiele für regulierbare Elemente, die gewebsspezifische Expression in Eukaryonten ermöglichen, sind Promotoren oder Aktivatorsequenzen aus Promotoren oder Enhancern von solchen Genen, die für Proteine kodieren, die nur in bestimmten Zelltypen exprimiert werden.

Beispiele für regulierbare Elemente, die zellzyklusspezifische Expression in Eukaryonten ermöglichen, sind Promotoren folgender Gene: cdc25A, cdc25B, cdc25C, Cyclin A, Cyclin E, cdc2, E2F-1 bis E2F-5, B-myb oder DHFR (Zwicker J. und Müller R. (1997) Trends Genet. 13, 3-6). Die Verwendung von zellzyklusregulierten Promotoren ist besonders bevorzugt in Fällen, in denen die Expression der erfindungsgemäß verwendeten Polypeptide oder Nukleinsäuren auf proliferierende Zellen beschränkt werden soll.

Ein Beispiel für ein regulierbares Element, das die Keratinozyten-spezifische Expression in der Haut erlaubt, ist das FiRE-Element (Jaakkola et al., 2000, Gen.

Ther., 7: 1640-1647). Das FiRE Element ist AP-1-getriebenes, durch FGF-induzierbares sogenanntes Response Element des Syndecan-1 Gens (Jaakkola et al., 1998, FASEB J., 12: 959-9).

Beispiele fiir regulierbare Elemente, die metabolischspezifische Expression in Eukaryonten ermöglichen, sind Promotoren, die durch Hypoxie, durch Glukosemangel, durch Phosphatkonzentration oder durch Hitzeschock reguliert werden.

Um die Einführung von den vorangehend beschriebenen Nukleinsäuren und damit die Expression des Polypeptids in einer eu- oder prokaryotischen Zelle durch Transfektion, Transformation oder Infektion zu ermöglichen, kann die Nukleinsäure als Plasmid, als Teil eines viralen oder nicht-viralen Vektors vorliegen. Als virale Vektoren eignen sich hierbei besonders: Baculoviren, Vakziniaviren, Adenoviren, adenoassoziierte Viren und Herpesviren. Als nicht-virale Vektoren eignen sich hierbei besonders: Virosomen, Liposomen, kationische Lipide, oder poly-Lysin konjugierte DNA.

Beispiele von gentherapeutisch wirksamen Vektoren sind Virusvektoren, beispielsweise Adenovirusvektoren oder retroviralen Vektoren (Lindemann et al., 1997, Mol. Med. 3: 466-76; Springer et al., 1998, Mol. Cell. 2: 549-58). Eukaryotische Expressionsvektoren eignen sich in isolierter Form für die gentherapeutische Anwendung, da nackte DNA bei topischer Applikation in Hautzellen eindringen kann (Hengge et al., 1996, J. Clin. Invest. 97: 2911-6; Yu et al., 1999, J. Invest. Dermatol. 112: 370-5).

Gentherapeutisch wirksame Vektoren lassen sich auch dadurch erhalten, daß man die vorangehend beschriebenen Nukleinsäure mit Liposomen komplexiert, da damit eine sehr hohe Transfektionseffizienz, insbesondere von Hautzellen, erreicht werden kann (Alexander und Akhurst, 1995, Hum. Mol. Genet. 4: 2279-85). Bei der Lipofektion werden kleine unilamellare Vesikel aus kationischen Lipiden durch Ultraschallbehandlung der Liposomensuspension herstellt. Die DNA wird ionisch auf der Oberfläche der Liposomen gebunden, und zwar in einem solchen Verhältnis, daß eine positive Nettoladung verbleibt und die Plasmid-DNA zu 100% von den Liposomen komplexiert wird. Neben den von Felgner et al. (1987, supra) eingesetzten Lipidmischungen DOTMA (1,2-Dioleyloxpropyl-3-trimethylammoniumbromid) und DPOE (Dioleoylphosphatidylethanolamin) wurden inzwischen zahlreiche neue Lipidformulierungen synthetisiert und auf ihre Effizienz bei der Transfektion verschiedener Zellinien getestet (Behr et al. (1989), Proc. Natl. Acad. Sci. USA 86, 6982-6986; Gao und Huang (1991), Biochim. Biophys. Acta 1189, 195-203; Felgner et al. (1994) J. Biol. Chem. 269, 2550-2561). Beispiele der neuen Lipidformulierungen sind DOTAP N-[1-(2,3-Dioleoyloxy)propyl]-N,N,N-trimethylammoniumethylsulfat oder DOGS (TRANSFECTAM; Dioctadecylamidoglycylspermin). Als sehr gut geignet für die Transfektion von Keratinozyten in vitro und in vivo haben sich auch die kationischen Lipide Cytofectin GS 2888 erwiesen (US 5,777,153; Lewis et al., 1996, Proc. Natl. Acad. Sci. USA, 93: 3176-3181). Hilfsstoffe, die den Transfer von Nukleinsäuren in die Zelle erhöhen, können beispielsweise Proteine oder Peptide, die an DNA gebunden sind oder synthetische Peptid-DNA-Moleküle, die den Transport der Nukleinsäure in den Kern der Zelle ermöglichen, sein (Schwartz et al. (1999) Gene Therapy 6, 282; Brandén et al. (1999) Nature Biotech. 17, 784). Hilfsstoffe umfassen auch Moleküle, die die Freisetzung von Nukleinsäuren in das Zytoplasma der Zelle ermöglichen (Planck et al. (1994) J. Biol. Chem. 269, 12918; Kichler et al. (1997) Bioconj. Chem. 8, 213) oder beispielsweise Liposomen (Uhlmann und Peymann (1990) supra). Eine andere besonders geeignete Form von gentherapeutischen Vektoren läßt sich dadurch erhalten, daß man die vorangehend beschriebene Nukleinsäure auf Goldpartikeln aufbringt und diese mit Hilfe der sogenannten "Gene Gun" in Gewebe, bevorzugt in die Haut, oder Zellen schießt (Wang et al., 1999, J. Invest. Dermatol., 112:775-81, Tuting et al., 1998, J. Invest. Dermatol. 111:183-8 ).

Eine weitere Ausführungsform eines gentherapeutisch wirksamen erfindungsgemäß verwendbaren Vektors läßt sich durch das Einbringen von "nackten" Expressionsvektoren in eine biokompatible Matrix, beispielsweise eine Kollagenmatrix, herstellen. Diese Matrix kann in Wunden eingebracht werden, um die einwandernden Zellen mit dem Expressionsvektor zu transfizieren und die erfindungsgemäßen Polypeptide in den Zellen zu exprimieren (Goldstein und Banadio, US 5,962,427).

Für die gentherapeutische Anwendung der vorangehend beschriebenen Nukleinsäure ist es auch von Vorteil, wenn der Teil der Nukleinsäure, der fiir das Polypeptid kodiert, ein oder mehrere nicht kodierende Sequenzen einschließlich Intronsequenzen, vorzugsweise zwischen Promotor und dem Startcodon des Polypeptids, und/oder eine polyA-Sequenz, insbesondere die natürlich vorkommende polyA-Sequenz oder eine SV40 Virus polyA-Sequenz, vor allem am 3'-Ende des Gens enthält, da hierdurch eine Stabilisierung der mRNA erreicht werden kann (Palmiter et al., 1991, Proc. Natl. Acad. Sci. USA 88:478-482 ; Jackson,1993, Cell 74:9-14).

Knock-out Genkonstrukte sind dem Fachmann zum Beispiel aus den US-Patenten 5,625,122; US 5,698,765; US 5,583,278 und US 5,750,825 bekannt.

Ein weitere bevorzugte Ausführungsform der vorliegenden Erfindung ist die Verwendung einer Zelle, bevorzugterweise einer autologen oder einer heterologen Zelle, insbesondere eine Hautzelle, die mit einem erfindungsgemäß verwendbaren Vektor oder knock-out Genkonstrukt zur Diagnose und/oder Prävention und/oder Behandlung von Erkrankungen von Hautzellen und/oder von Wundheilung und/oder deren krankhaften Störungen sowie zur Identifizierung von pharmakologisch aktiven Substanzen transformiert ist. Zellen können sowohl prokaryotische als auch eukaryotische Zellen sein, Beispiele für prokaryotische Zellen sind *E*. *coli* und für eukaryotische Zellen *Saccharomyces cerevisiae* oder Insektenzellen.

Ein besonders bevorzugte erfindungsgemäß verwendbare transformierte Zelle ist eine transgene embryonale nichtmenschliche Stammzelle, die dadurch gekennzeichnet ist, daß sie mindestens ein erfindungsgemäß verwendbares knock-out Genkonstrukt und/oder mindestens eine erfindungsgemäß verwendbare Expressionskassette, wie vorangehend beschrieben, enthält. Verfahren zur Transformation von Zellen und/oder Stammzellen sind dem Fachmann gut bekannt und umfassen zum Beispiel Elektroporation oder Mikroinjektion.

Transgene nichtmenschliche Säugetiere, deren Genom mindestens ein erfindungsgemäß verwendbares knock-out Genkonstrukt und/oder mindestens eine erfindungsgemäß verwendbare Expressionskassette, wie vorangehend beschrieben enthalten, können zur Diagnose und/oder Prävention und/oder Behandlung von Erkrankungen von Hautzellen und/oder von Wundheilung und/oder deren krankhaften Störungen oder zur Identifizierung von pharmakologisch aktiven Substanzen, verwendet werden. Transgene Tiere, die eine der vorangehend beschriebenen Expressionskassetten enthalten, zeigen in Abhängigkeit des verwendeten Promotors im allgemeinen eine gewebespezifisch erhöhte Expression der Nukleinsäuren und/oder Polypeptide und lassen sich zur Analyse von Wundheilungsstörungen verwenden. So weist beispielsweise eine Activin A transgene Maus eine verbesserte Wundheilung auf (Munz et al., 1999, EMBO J. 18:5205-15) während eine transgene Maus mit dominant negativem KGF Rezeptor eine verzögerte Wundheilung aufweist (Werner et al., 1994, Science 266:819-22). Darüber hinaus können vorangehend beschriebene transgene Tiere mit beschleunigter Wundheilung ausgestattet werden.

Verfahren zur Herstellung von transgenen Tieren, insbesondere von transgenen Mäusen, sind dem Fachmann ebenfalls aus der DE 196 25 049 und den US 4,736,866; US 5,625,122; US 5,698,765; US 5,583,278 und US 5,750,825 bekannt und umfassen transgene Tiere, die beispielsweise über direkte Injektion von Expressionsvektoren (s.o.) in Embryonen oder Spermatozyten oder über die Transfektion von Expressionsvektoren in embryonale Stammzellen erzeugt werden können (Polites und Pinkert: DNA Microinjection and Transgenic Animal Produktion, Seite 15 bis 68 in Pinkert, 1994: Transgenic animal technology: a laboratory handbook, Academic Press, London, UK; Houdebine, 1997, Harwood Academic Publishers, Amsterdam, The Netherlands; Doetschman: Gene Transfer in Embryonic Stem Cells, Seite 115 bis 146 in Pinkert, 1994, supra; Wood: Retrovirus-Mediated Gene Transfer, Seite 147 bis 176 in Pinkert, 1994, supra; Monastersky: Gene Transfere Technology: Alternative Techniques and Applications, Seite 177 bis 220 in Pinkert, 1994, supra).

Werden die vorangehend beschriebenen erfindungsgemäß verwendbaren Nukleinsäuren in sogenannte "Targeting" Vektoren oder "knock-out" Genkonstrukte integriert (Pinkert, 1994, supra) können nach Transfektion von embryonalen Stammzellen und homologer Rekombination beispielsweise knock-out Mäuse generiert werden, die im allgemeinen als heterozygote Mäuse verringerte Expression der Nukleinsäure zeigen, während homozygote Mäuse keine Expression der Nukleinsäure mehr aufweisen. Auch die so erzeugten Tiere lassen sich zur Analyse von Wundheilungsstörungen verwenden. So weisen beispielsweise die eNOS-(Lee et al., 1999, Am. J. Physiol. 277:H1600-H1608), Nf-1 (Atit et al., 1999, J. Invest. Dermatol. 112:835-42) und Osteopontin (Liaw et al., 1998, J. Clin. Invest. 101:967-71) knock-out Mäuse eine verschlechterte Wundheilung auf. Auch hier ist eine gewebespezifische Reduktion der Expression wundheilungsrelevanter Gene, beispielsweise in hautspezifischen Zellen unter Verwendung des Cre-loxP Systems (stat3 knock-out, Sano et al., EMBO J. 1999 18:4657-68), besonders zu bevorzugen. So erzeugte transgene und knock-out Zellen oder Tiere lassen sich auch zur Rasteruntersuchung (Screening) und zur Identifizierung von pharmakologisch aktiven Substanzen bzw. gentherapeutisch aktiven Vektoren verwenden.

Erfindungsgemäß verwendbare Polypeptids können nach allgemein bekannten rekombinanten Verfahren hergestellt werden. Weiterhin können erfindungsgemäß verwendbare Polypeptide aus einem Organismus oder aus Gewebe oder Zellen isoliert und erfindungsgemäß verwendet werden. So ist es beispielsweise möglich, erfindungsgemäß verwendbare Polypeptide aus Säugetiergewebe, beispielsweise aus Haut oder aus Körperflüssigkeiten, beispielsweise Blut, Serum, Speichel, Synovialflüssigkeit oder Wundflüssigkeit aufzureinigen. Weiterhin können aus erfindungsgemäß verwendbare Polypeptide exprimierenden Zellen Zellinien hergestellt werden, die dann zur Isolierung von erfindungsgemäß verwendbare Polypeptiden verwendet werden können. Beispielsweise können Expressionsvektoren, enthaltend die erfindungsgemäß verwendbaren Nukleinsäuren in Hautzellen, beispielsweise HaCaT Zellen transformiert werden. Die Expression kann beispielsweise konstitutiv oder induzierbar sein.

Das Polypeptid wird beispielsweise durch Expression der vorangehend beschriebenen Nukleinsäuren in einem geeigneten Expressionssystem, wie oben bereits erwähnt, nach dem Fachmann allgemein bekannten Methoden hergestellt. Als Zellen eignen sich beispielsweise die E. coli Stämme DHS, HB101 oder BL21, der Hefestamm *Saccharomyces cerevisiae,* die Insektenzellinie Lepidopteran, z. B. von *Spodoptera frugiperda*, oder die tierischen Zellen COS, Vero, 293, HaCaT, und HeLa, die alle allgemein erhältlich sind.

Eine weitere Ausführungsform betrifft die Verwendung der erfindungsgemäßen Polypeptide, wobei die Polypeptide in Form eines Fusionsproteins eingesetzt werden. Erfindungsgemäß verwendbare Fusionsproteine können beispielsweise hergestellt werden, indem erfindungsgemäß verwendbare Nukleinsäuren in einer geeigneten Zelle exprimiert werden.

Die Fusionsproteine selbst weisen bereits die Funktion eines Polypeptids der Erfindung auf oder sind erst nach Abspaltung des Fusionsanteils funktionell. Vor allem zählen hierzu Fusionsproteine mit einem Anteil von ca. 1-300, vorzugsweise ca. 1-200, insbesondere ca. 1-100, vor allem ca. 1-50 fremden Aminosäuren. Beispiele solcher Peptidsequenzen sind prokaryotische Peptidsequenzen, die z. B. aus der Galactosidase von *E. coli* abgeleitet sein können. Weiterhin können auch virale Peptidsequenzen, wie zum Beispiel vom Bakteriophagen M13 verwendet werden, um so Fusionsproteine für das dem Fachmann bekannte "phage display"Verfahren zu erzeugen.

Weitere bevorzugte Beispiele fiir Peptidsequenzen für erfindungsgemäß verwendbare Fusionsproteine sind Peptide, die die Detektion der Fusionsproteine erleichtern, hierzu zählen beispielsweise "Green-fluorescent-protein" (WO 95/07463) oder funktionelle Varianten davon.

Zur Aufreinigung der vorangehend beschriebenen Proteine kann ein weiteres/weiterer Polypeptid ("tag") angefügt sein. Erfindungsgemäße Protein-tags erlauben beispielsweise die hochaffine Absorption an eine Matrix, stringentes Waschen mit geeigneten Puffern, ohne den Komplex in nennenswertem Maße zu eluieren und anschließend gezielte Elution des absorbierten Komplexes. Beispiele der dem Fachmann bekannten Protein-tags sind ein (His)₆-tag, ein Myc-tag, ein FLAG-tag, ein Hämagglutinin-tag, Glutathion-Transferase (GST)-tag, Intein mit einem Affinitäts-Chitin-binding-tag oder Maltose-binding protein (MBP)-tag. Diese Protein-tags können sich N-, C-terminal und/oder intern befinden.

Eine weitere Ausführungsform der Erfindung betrifft die Verwendung eines gegen ein erfindungsgemäß verwendbares Polypeptid oder gegen eine funktionelle Variante davon gerichteten Antikörpers oder Antikörperfragments, vorzugsweise eines polyklonalen oder monoklonalen Antikörpers oder Antikörperfragments, gegebenenfalls zusammen oder kombiniert mit geeigneten Hilfs- und/oder Zusatzstoffen, zur Herstellung eines Arzneimittels und/oder eines Diagnostikums zur Analyse, Diagnose, Prävention und/oder Behandlung von Erkrankungen von Hautzellen, von Wundheilung und/oder deren krankhaften Störungen sowie seine Verwendung zur Identifizierung von pharmakologisch aktiven Substanzen.

So kann beispielsweise die lokale Injektion von monoklonalen Antikörpern gegen TGF beta 1 im Tiermodell die Wundheilung verbessern (Ernst et al., 1996, Gut 39:172-5).

Das Verfahren zur Herstellung eines Antikörpers oder Antikörperfragments, vorzugsweise eines polyklonalen oder monoklonalen Antikörpers erfolgt nach dem Fachmann allgemein bekannten Methoden durch Immunisieren eines Säugetiers, beispielsweise eines Kaninchens, mit dem erfindungsgemäß verwendbaren Polypeptid oder funktioneller Varianten davon, vorzugsweise mit Teilen davon, mit mindestens 6 Aminosäuren Länge, vorzugsweise mit mindestens 8 Aminosäuren Länge, insbesondere mit mindestens 12 Aminosäuren Länge, gegebenenfalls in Anwesenheit von z. B. Freund's Adjuvant und/oder Aluminiumhydroxidgelen (siehe z. B. Diamond, B. A. et al. (1981) The New England Journal of Medicine, 1344-1349). Die im Tier aufgrund einer immunologischen Reaktion entstandenen polyklonalen Antikörper lassen sich anschließend nach allgemein bekannten Methoden leicht aus dem Blut isolieren und z. B. über Säulenchromatographie reinigen. Monoklonale Antikörper können beispielsweise nach der bekannten Methode von Winter & Milstein (Winter, G. & Milstein, C. (1991) Nature, 349, 293-299) hergestellt werden. Als Alternative zu den klassischen Antikörpern können beispielsweise auf Lipocalin basierende sogenannte "Anticaline" verwendet werden (Beste et al., 1999, Proc. Natl. Acad. Sci. USA, 96:1898-1903). Die natürlichen Liganden-bindenden Sites der Lipocaline, wie z.B. das Retinol-bindende Protein oder das Bilin-bindende Protein können beispielsweise durch einen "kombinatorischen Protein Design" Ansatz in einer Weise verändert werden, daß sie an ausgewählte Haptene, beispielsweise an die erfindungsgemäß verwendbaren Polypeptide binden (Skerra, 2000, Biochim. Biophys. Acta 1482:337-50). Weitere bekannte "scaffolds" als Alternativen für Antikörper für die molekulare Erkennung sind bekannt (Skerra, J. Mol. Recognit., 2000, 13:167-187).

Der erfindungsgemäß verwendbare Antikörper oder das Antikörperfragment sind gegen ein erfindungsgemäßes Polypeptid gerichtet ist und reagieren spezifisch mit den erfindungsgemäßen Polypeptiden , wobei die oben genannten Teile des Polypeptids entweder selbst immunogen sind oder durch Kopplung an geeignete Träger, wie z.B. bovines Serumalbumin, immunogen gemacht bzw. in ihrer Immunogenität gesteigert werden können. Dieser erfindungsgemäß verwendbare Antikörper ist entweder polyklonal oder monoklonal, bevorzugt ist ein monoklonaler Antikörper. Unter dem Begriff Antikörper oder Antikörperfragment versteht man gemäß der vorliegenden Erfindung auch gentechnisch hergestellte und gegebenenfalls modifizierte Antikörper bzw. antigenbindende Teile davon, wie z.B. chimäre Antikörper, humanisierte Antikörper, multifunktionelle Antikörper, bi- oder oligospezifische Antikörper, einzelsträngige Antikörper, F(ab)- oder F(ab)₂-Fragmente (siehe z.B. EP-B1-0 368 684, US 4,816,567, US 4,816,397, WO 88/01649, WO 93/06213, WO 98/24884).

Die identifizierten pharmakologisch aktiven Substanzen können, gegebenenfalls kombiniert oder zusammen mit geeigneten Zusatz- und/oder Hilfsstoffen, zur Herstellung eines Diagnostikums oder eines Arzneimittels zur Prävention, Behandlung und/oder Diagnose von Erkrankungen von Hautzellen und/oder bei der Wundheilung und/oder deren krankhaften Störungen eingesetzt werden. Die pharmakologisch aktiven Substanzen können beispielsweise anorganische oder organische Moleküle, beispielsweise Nukleinsäuren oder Nukleinsäurenanaloga, Peptide oder Proteine, insbesondere Antikörper, Varianten erfindungsgemäß verwendbarer Polypeptide oder diese kodierende Nukleinsäuren oder Liganden erfindungsgemäß verwendbarer Polypeptide sein. Beispiele pharmakologisch aktiver Substanzen sind weiterhin organische Moleküle, die Substanzbibliotheken entstammen, die auf ihre pharmakologische Aktivität hin überprüft wurden.

Die Verwendung von Nukleinsäuren als Diagnostika kann beispielsweise auf der Basis der Polymerasekettenreaktion wie weiter unten beschrieben erfolgen. Die Verwendung von Nukleinsäuren als Arzneimittel kann beispielsweise gentherapeutisch unter Verwendung eines gentherapeutisch aktiven Vektors oder mittels Applikation von antisense Nukleotids wie beschrieben erfolgen.

Die Verwendung von anderen organischen oder anorganischen pharmakologisch aktiven Substanzen als Arzneimittel kann beispielsweise durch Applikation wie oben beschrieben erfolgen. Die Verwendung beispielsweise von Antikörpern als Diagnostika kann wie unten weiter unten beschrieben mittels immunologischer Techniken erfolgen, beispielsweise unter Verwendung von Antikörpern, die mit einem Enzym markiert sind. Der spezifische Antikörper-Peptid-Komplex kann dadurch leicht und ebenso schnell über eine enzymatische Farbreaktion nachgewiesen werden.

Um pharmakologisch aktive Substanzen als Diagnostikum zu verwenden, können die Substanzen einen detektierbaren Marker enthalten, beispielsweise kann die Substanz radioaktiv markiert, Fluoreszenz-markiert oder Lumineszenz-markiert sein. Weiterhin können Substanzen an Enzyme gekoppelt werden, die eine indirekte Detektion erlauben, beispielsweise wie oben beschrieben über enzymatische Katalyse mittels eines Peroxidase-Assays mit einem chromogenen Substrat oder durch Bindung eines markierten oder detektierbaren Antikörpers. Die Substanzen können dann mit einer zu untersuchenden Probe in Kontakt gebracht werden und so die Menge eines erfindungsgemäß verwendbaren Polypeptids oder einer funktionellen Variante davon oder dieses kodierende Nukleinsäure oder einer Variante davon, oder einer ein erfindungsgemäß verwendbares Polypeptid oder einer funktionellen Variante davon oder einer dieses kodierende Nukleinsäure oder eine Variante davon exprimierenden Zelle, oder eines gegen ein erfindungsgemäß verwendbares Polypeptid gerichteten Antikörpers oder eines Antikörperfragments in der Probe bestimmt werden. Die Ergebnisse dieser Probe, die einem zu untersuchenden Organismus entstammt, kann dann verglichen werden mit dem Ergebnis einer Probe, die einem gesunden oder kranken Organismus entstammt.

Die Erfindung betrifft weiterhin die Verwendung eines Arzneimittels zur Prävention und/oder Behandlung von Erkrankungen von Hautzellen, von Wundheilung und/oder deren krankhaften Störungen, wobei mindestens ein erfindungsgemäß verwendbares Polypeptid oder eine funktionelle Variante davon und/oder dieses kodierende Nukleinsäure, und/oder einer ein erfindungsgemäß verwendbares Polypeptid oder eine funktionelle Variante davon oder einer dieses kodierende Nukleinsäure oder eine Variante davon exprimierenden Zelle, und/oder eines gegen ein erfindungsgemäß verwendbares Polypeptid oder eine funktionelle Variante davon gerichteten Antikörpers oder eines Antikörperfragments, gegebenenfalls kombiniert oder zusammen mit geeigneten Zusatz- und/oder Hilfsstoffen eingesetzt wird.

Die Therapie von Erkrankungen von Hautzellen und/oder von Wundheilung und/oder deren krankhaften Störungen, kann auf herkömmliche Weise, z.B. durch Verbände, Pflaster, Kompressen oder Gele erfolgen, die die erfindungsgemäßen Arzneimittel enthalten. So ist es möglich, die geeignete Zusatz- oder Hilfsstoffe, wie z. B. physiologische Kochsalzlösung, entmineralisiertes Wasser, Stabilisatoren, Proteinaseinhibitoren, Gelformulierungen, wie z.B. weiße Vaseline, dünnflüssiges Paraffin und/oder gelbes Wachs, etc., enthaltenden Arzneimittel topisch und lokal zu verabreichen, um die Wundheilung sofort und unmittelbar zu beeinflussen. Die Verabreichung der erfindungsgemäßen Arzneimittel kann weiterhin gegebenenfalls in Form von Liposomenkomplexen bzw. Goldpartikelkomplexen ebenfalls topisch und lokal im Bereich der Wunde erfolgen. Weiterhin kann die Behandlung mittels eines transdermalen therapeutischen Systems (TTS) erfolgen, das eine zeitlich gesteuerte Abgabe der erfindungsgemäß verwendbaren Arzneimittel ermöglicht. Die Behandlung mittels der erfindungsgemäß verwendbaren Arzneimittel kann aber auch über orale Dosierungsformen, wie z.B. Tabletten oder Kapseln, über die Schleimhäute, zum Beispiel der Nase oder der Mundhöhle, oder in Form von unter die Haut implantierten Dispositorien erfolgen. TTS sind zum Beispiel aus den EP 0 944 398, EP 0 916 336, EP 0 889 723 oder EP 0 852 493 bekannt.

Für die gentherapeutische Anwendung beim Menschen ist vor allem ein Arzneimittel geeignet, das die beschriebenen Nukleinsäure in nackter Form oder in Form eines der oben beschriebenen gentherapeutisch wirksamen Vektoren oder in mit Liposomen bzw. Goldpartikeln komplexierter Form enthält. Der pharmazeutische Träger ist beispielsweise eine physiologische Pufferlösung, vorzugsweise mit einem pH von ca. 6,0-8,0, vorzugsweise von ca. 6,8-7,8. Insbesondere von ca. 7,4 und/oder einer Osmolarität von ca. 200-400 milliosmol/Liter, vorzugsweise von ca. 290-310 milliosmol/Liter. Zusätzlich kann der pharmazeutische Träger geeignete Stabilisatoren, wie z.B. Nukleaseinhibitoren, vorzugsweise Komplexbildner wie EDTA und/oder andere dem Fachmann bekannte Hilfsstoffe enthalten.

Die Verabreichung der beschriebenen Nukleinsäure gegebenenfalls in Form der oben näher beschriebenen Virusvektoren oder als Liposomenkomplexe bzw. Goldpartikelkomplex erfolgt üblicherweise topisch und lokal im Bereich der Wunde. Es ist auch möglich, das Polypeptid selbst mit geeigneten Zusatz- oder Hilfsstoffen, wie z. B. physiologische Kochsalzlösung, entmineralisiertes Wasser, Stabilisatoren, Proteinaseinhibitoren, Gelformulierungen, wie z.B. weiße Vaseline, dünnflüssiges Paraffin und/oder gelbes Wachs, etc., zu verabreichen, um die Wundheilung sofort und unmittelbar zu beeinflussen.

Beispiele von Erkrankungen der Hautzellen im Sinne der vorliegenden Erfindung sind Psoriasis, Ekzeme, insbesondere atopisches Ekzeme, Akne, Urtikaria, Pigmentstörungen der Haut, insbesondere Vitiligo, "senile skin" und Störungen des Haarwachstums oder -metabolismus.

Unter Wundheilung im Sinne der vorliegenden Erfindung ist der Heilungsprozeß einer mechanischen Verletzung der Haut zu verstehen, wie z.B. Schnittwunden, Schürfwunden oder das Aufreiben der Haut beispielsweise durch andauernde Belastung, beispielsweise Dekubitus oder nekrotische Prozesse, beispielsweise *Nekrobiosis lipoidica*.

Beispiele für gestörte Wundheilung im Sinne der vorliegenden Erfindung sind Wunden diabetischer Patienten und Alkoholiker, mit Organismen oder Viren infizierte Wunden, ischämische Wunden, Wunden von Patienten mit arteriellen Verschlußkrankheiten oder venöser Insuffizienz, und Narben, bevorzugt überschießende Narben, insbesondere Keloide. Besonders bevorzugte schlecht heilende Wunden sind diabetische, neuropathische, venöse und arterielle Ulzera, insbesondere diabetische Ulzera.

Die vorliegende Erfindung betrifft weiterhin die Verwendung mindestens eines erfindungsgemäß verwendbaren Polypeptids oder einer funktionellen Variante davon und/oder dieses kodierende Nukleinsäure oder eine Variante davon, und/oder einer ein erfindungsgemäß verwendbares Polypeptid oder einer funktionellen Variante davon oder einer dieses kodierende Nukleinsäure oder Variante davon exprimierenden Zelle, und/oder eines gegen ein erfindungsgemäß verwendbares Polypeptid oder eine funktionelle Variante davon gerichteten Antikörpers oder eines Antikörperfragments, gegebenenfalls kombiniert oder zusammen mit geeigneten Zusatz- und/oder Hilfsstoffen, zur Herstellung eines Diagnostikum zur Diagnose von Erkrankungen von Hautzellen und/oder bei der Wundheilung und/oder deren krankhaften Störungen.

Beispielsweise kann gemäß der vorliegenden Erfindung anhand einer der beschriebenen Nukleinsäure ein Diagnostikum auf der Basis der Polymerasekettenreaktion (Beispiele 2, 4, 5, 6, 7, PCR-Diagnostik, z. B. gemäß EP 0 200 362) oder eines RNase-Protection-Assays (siehe z. B. Sambrook et al., supra Kapitel 7, Seite 7.71-7.78; Werner et al., 1992, Growth Factors and Receptors: A Practical Approach 175-197; Werner, 1998, Proc. Natl. Acad. Sci. U.S.A. 89: 6896-6900) hergestellt werden. Diese Tests beruhen auf der spezifischen Hybridisierung einer Nukleinsäure mit ihrem komplementären Gegenstrang, üblicherweise der entsprechenden mRNA oder ihrer cDNA. Die erfindungsgemäß verwendbaren Nukleinsäuren können hierbei auch modifiziert sein, wie z. B. in EP 0 063 879 offenbart. Vorzugsweise wird ein solches DNA-Fragment mittels geeigneter Reagenzien, z. B. radioaktiv mit α-P³²-dCTP oder nicht-radioaktiv mit Biotin oder Digoxigenin, nach allgemein bekannten Methoden markiert und mit isolierter RNA, die vorzugsweise vorher an geeignete Membranen aus z. B. Nitrocellulose oder Nylon gebunden wurde, inkubiert. Bei gleicher Menge an untersuchter RNA aus jeder Gewebeprobe kann somit die Menge an mRNA bestimmt werden, die spezifisch durch die Sonde markiert wurde. Alternativ kann die Bestimmung der mRNA Menge auch direkt in Gewebeschnitten mit Hilfe der in situ Hybridisierung (siehe z.B. Werner et al., 1992, Proc. Natl. Acad. Sci. U. S. A. 89:6896-900) erfolgen.

Die vorliegende Erfindung betrifft weiterhin die Verwendung eines Diagnostikums zur Diagnose von Erkrankungen von Hautzellen und/oder bei der Wundheilung und/oder deren krankhaften Störungen, wobei mindestens ein erfindungsgemäß verwendbares Polypeptids oder eine funktionelle Variante davon und/oder dieses kodierende Nukleinsäure oder eine Variante davon, und/oder einer ein erfindungsgemäß verwendbares Polypeptid oder einer funktionellen Variante davon oder einer dieses kodierende Nukleinsäure oder Variante davon exprimierenden Zelle, und/oder ein gegen ein erfindungsgemäß verwendbares Polypeptid oder eine funktionelle Variante davon gerichteter Antikörper oder ein Antikörperfragment, gegebenenfalls kombiniert oder zusammen mit geeigneten Zusatz- und/oder Hilfsstoffen, zur Diagnose von Erkrankungen von Hautzellen und/oder bei der Wundheilung und/oder deren krankhaften Störungen eingesetzt wird.

Mit Hilfe des erfindungsgemäß verwendbaren Diagnostikums kann somit auch *in vitro* die Expressionsstärke des jeweiligen Gens in einer Gewebeprobe spezifisch gemessen werden, um beispielsweise eine Wundheilungsstörung oder Hauterkrankungen sicher diagnostizieren zu können (Beispiele 2, 4, 5, 6, 7). Insbesondere eignet sich ein solches Verfahren zur frühzeitigen Prognose von Störungen.

Ein bevorzugtes erfindungsgemäßes Diagnostikum enthält ein erfindungsgemäß verwendbares Polypeptid bzw. die oben näher beschriebenen immunogenen Teile davon. Das Polypeptid bzw. die Teile davon, die vorzugsweise an eine Festphase, z.B. aus Nitrocellulose oder Nylon, gebunden sind, können beispielsweise mit der zu untersuchenden Körperflüssigkeit, z.B. Wundsekret, *in vitro* in Berührung gebracht werden, um so beispielsweise mit Autoimmunantikörper reagieren zu können. Der Antikörper-Peptid-Komplex kann anschließend beispielsweise anhand markierter Antihuman-IgG- oder Antihuman-IgM-Antikörper nachgewiesen werden. Bei der Markierung handelt es sich beispielsweise um ein Enzym, wie Peroxidase, das eine Farbreaktion katalysiert. Die Anwesenheit und die Menge an anwesenden Autoimmunantikörper kann somit über die Farbreaktion leicht und schnell nachgewiesen werden.

Ein weiteres erfindungsgemäß verwendbares Diagnostikum, das Gegenstand der vorliegenden Erfindung ist, enthält die erfindungsgemäß verwendbaren Antikörper selbst. Mit Hilfe dieser Antikörper kann beispielsweise eine Gewebeprobe leicht und schnell dahingehend untersucht werden, ob das betreffende Polypeptid in einer erhöhten Menge vorhanden ist, um dadurch einen Hinweis auf eine mögliche Erkrankung, insbesondere Hauterkrankungen und Wundheilungsstörung zu erhalten. In diesem Fall sind die erfindungsgemäßen Antikörper beispielsweise mit einem Enzym, wie oben bereits beschrieben, markiert. Der spezifische Antikörper-Peptid-Komplex kann dadurch leicht und ebenso schnell über eine enzymatische Farbreaktion nachgewiesen werden.

Ein weiteres erfindungsgemäß verwendbares Diagnostikum umfaßt eine Sonde, vorzugsweise eine DNA-Sonde, und/oder Primer. Dies eröffnet eine weitere Möglichkeit, die beschriebenen Nukleinsäuren, zum Beispiel durch die Isolierung aus einer geeigneten Genbank, beispielsweise aus einer wundspezifischen Genbank, anhand einer geeigneten Sonde zu erhalten (siehe z. B. J. Sambrook et al., 1989, Molecular Cloning. A Laboratory Manual 2. Ausgabe, Cold Spring Harbor Laboratory, Cold Spring Harbor, NY Kapitel 8 Seite 8.1 bis 8.81, Kapitel 9 Seite 9.47 bis 9.58 und Kapitel 10 Seite 10.1 bis 10.67).

Als Sonde eignen sich beispielsweise DNA- oder RNA-Fragmente mit einer Länge von ca. 100-1000 Nukleotiden, vorzugsweise mit einer Länge von ca. 200-500 Nukleotiden, insbesondere mit einer Länge von ca. 300-400 Nukleotiden deren Sequenz aus den Polypeptiden gemäß den SEQ ID Nr. 1 bis SEQ ID Nr. 48 und SEQ ID Nr. 55 bis SEQ ID Nr. 58 und SEQ ID Nr. 63 bis SEQ ID Nr. 73 und SEQ ID Nr. 80 bis SEQ ID Nr. 82 des Sequenzprotokolls und/oder anhand der cDNA Sequenzen der in den Tabellen 3 bis 5 angegebenen Datenbankeinträge oder anhand des Sequenzprotokolls gemäß einer SEQ ID Nr. 50 bis SEQ ID Nr. 54 und SEQ ID Nr. 83 bis SEQ ID Nr. 84 abgeleitet werden kann.

Alternativ können anhand der abgeleiteten Nukleinsäuresequenzen Oligonukleotide synthetisiert werden, die sich als Primer für eine Polymerase Kettenreaktion eignen. Mit diesen kann die vorangehend beschriebene Nukleinsäure oder Teile dieser aus cDNA, beispielsweise wundspezifischer cDNA, amplifiziert und isoliert werden (Beispiele 2, 4, 5, 6, 7). Als Primer eignen sich beispielsweise DNA-Fragmente mit einer Länge von ca. 10 bis 100 Nukleotiden, vorzugsweise mit einer Länge von ca. 15 bis 50 Nukleotiden, insbesondere mit einer Länge von 20 bis 30 Nukleotiden deren Sequenz aus den Polypeptiden gemäß den SEQ ID Nr. 1 bis SEQ ID Nr. 48 und SEQ ID Nr. 55 bis SEQ ID Nr. 58 und SEQ ID Nr. 63 bis SEQ ID Nr. 73 und SEQ ID Nr. 80 bis SEQ ID Nr. 82 des Sequenzprotokolls und/oder anhand der cDNA Sequenzen der in den Tabellen 3 bis 5 angegebenen Datenbankeinträge oder anhand des Sequenzprotokolls gemäß einer SEQ ID Nr. 50 bis SEQ ID Nr. 54 und SEQ ID Nr. 83 bis SEQ ID Nr. 84 abgeleitet werden kann.

Die vorliegende Erfindung betrifft weiterhin die Verwendung mindestens eines erfindungsgemäß verwendbaren gegen ein erfindungsgemäß verwendbares Polypeptid gerichteten Antikörpers oder Antikörperfragments zur Identifizierung von pharmakologisch aktiven Substanzen, wobei der/die Antikörper oder Antikörperfragmente an eine Festphase gekoppelt sind.

Die vorliegende Erfindung betrifft weiterhin die Verwendung mindestens eines erfindungsgemäß verwendbaren Polypeptids oder einer funktionellen Variante davon und/oder dieses kodierende Nukleinsäure oder eine Variante davon, und/oder einer ein erfindungsgemäß verwendbares Polypeptid oder einer funktionellen Variante davon oder einer dieses kodierende Nukleinsäure oder Variante davon exprimierenden Zelle, und/oder eines gegen ein erfindungsgemäß verwendbares Polypeptid oder eine funktionelle Variante davon gerichteten Antikörpers oder eines Antikörperfragments, gegebenenfalls kombiniert oder zusammen mit geeigneten Zusatz- und/oder Hilfsstoffen, zur Herstellung eines Tests zur Auffindung von pharmakologisch aktiven Substanzen in Zusammenhang mit Hauterkrankungen und/oder in Zusammenhang mit Wundheilung, insbesondere Wundheilungsstörungen.

Die vorliegende Erfindung betrifft weiterhin die Verwendung mindestens eines erfindungsgemäß verwendbaren Polypeptids oder einer funktionellen Variante davon und/oder dieses kodierende Nukleinsäure oder eine Variante davon, und/oder einer ein erfindungsgemäß verwendbares Polypeptid oder einer funktionellen Variante davon oder einer dieses kodierende Nukleinsäure oder Variante davon exprimierenden Zelle, und/oder eines gegen ein erfindungsgemäß verwendbares Polypeptid oder eine funktionelle Variante davon gerichteten Antikörpers oder ein Antikörperfragments, gegebenenfalls kombiniert oder zusammen mit geeigneten Zusatz- und/oder Hilfsstoffen in Form eines Tests zur Auffindung von pharmakologisch aktiven Substanzen in Zusammenhang mit Erkrankungen von Hautzellen und/oder bei der Wundheilung und/oder deren krankhaften Störungen.

Ein geeignetes System läßt sich beispielsweise durch die stabile Transformation von epidermalen bzw. dermalen Zellen mit Expressionsvektoren, die selektierbare Markergene und die beschriebenen Nukleinsäuren enthalten, herstellen. Bei diesem Verfahren wird die Expression der beschriebenen Nukleinsäuren in den Zellen so verändert, daß sie der pathologisch gestörten Expression in vivo entspricht. Auch anti-sense Oligonukleotide, die die beschriebenen Nukleinsäure enthalten, können zu diesem Zweck eingesetzt werden. Von besonderem Vorteil für diese Systeme ist es daher, das Expressionsverhalten der Gene bei gestörten regenerativen Prozessen, wie in dieser Anmeldung offengelegt, zu kennen. Oft kann so das pathologische Verhalten der Zellen in vitro nachgeahmt werden und es können Substanzen gesucht werden, die das normale Verhalten der Zellen wieder herstellen und die ein therapeutisches Potential besitzen.

Für diese erfindungsgemäß verwendbaren Testsysteme eignen sich z.B. HaCaT-Zellen, die allgemein erhältlich sind, und der Expressionsvektor pCMV4 (Anderson et al., 1989, J. Biol. Chem. 264:8222-9). Die vorangehend beschriebene Nukleinsäure kann dabei sowohl in sense als auch in anti-sense Orientierung in die Expressionsvektoren integriert werden, so daß die funktionelle Konzentration an mRNA der entsprechenden Gene in den Zellen entweder erhöht, oder durch Hybridisierung mit der antisense-RNA erniedrigt wird. Nach der Transformation und Selektion stabiler Transformanden zeigen die Zellen in Kultur im allgemeinen ein verändertes Proliferations-, Migrations, und/oder Differenzierungsverhalten im Vergleich zu Kontrollzellen. Dieses Verhalten in vitro ist häufig mit der Funktion der entsprechenden Gene bei regenerativen Prozessen im Organismus korreliert (Yu et al., 1997, Arch. Dermatol. Res. 289:352-9; Mils er al., 1997, Oncogene 14: 15555-61; Charvat et al., 1998, Exp Dermatol 7: 184-90; Werner, 1998, Cytokine Growth Factor Rev. 9:153-65; Mythily et al., 1999, J. Gen. Virol. 80:1707-13) und läßt sich mit einfachen und schnell durchzuführenden Tests nachweisen, so daß man darauf basierend Testsysteme fiir pharmakologisch aktive Substanzen aufbauen kann. So läßt sich das Proliferationsverhalten von Zellen sehr schnell durch z.B. den Einbau von markierten Nukleotiden in die DNA der Zellen (siehe z.B. Savino und Dardenne, 1985, J. Immunol. Methods 85:221-6; Perros und Weightman, 1991, Cell Prolif. 24:517-23; de Fries und Mitsuhashi, 1995, J. Clin. Lab. Anal. 9:89-95), durch Anfärbung der Zellen mit spezifischen Farbstoffen (Schulz et al., 1994, J. Immunol. Methods 167:1-13) oder über immunologische Verfahren (Frahm et al., 1998, J. Immunol. Methods 211:43-50) nachweisen. Die Migration läßt sich einfach durch den "Migration Index" Test (Charvat et al., supra) und vergleichbare Testsysteme (Benestad et al., 1987, Cell Tissue Kinet. 20:109-19, Junger et al., 1993, J. Immunol. Methods 160:73-9) nachweisen. Als Differenzierungsmarker eignen sich z.B. Keratin 6, 10 und 14 sowie Loricrin und Involucrin (Rosenthal et al., 1992, J. Invest. Dermatol. 98:343-50), deren Expression z.B. über allgemein erhältliche Antikörper leicht nachzuweisen ist.

Ein anderes geeignetes erfindungsgemäß verwendbares Testsystem basiert auf der Identifikation von Interaktionen mit dem sogenannten "Two-Hybrid System" (Fields und Sternglanz, 1994, Trends in Genetics, 10, 286-292; Colas und Brent, 1998 TIBTECH, 16, 355-363). Bei diesem Test werden Zellen mit Expressionsvektoren transformiert, die Fusionsproteine aus dem erfindungsgemäßen Polypeptid und einer DNA-Bindungsdomäne eines Transkriptionsfaktors wie beispielsweise Gal4 oder LexA exprimieren. Die transformierten Zellen enthalten außerdem ein Reportergen, dessen Promotor Bindungsstellen für die entsprechende DNA Bindungsdomäne enthalten. Durch Transformation eines weiteren Expressionsvektors, der ein zweites Fusionsprotein aus einem bekannten bzw. unbekannten Polypeptid mit einer Aktivierungsdomäne, beispielsweise von Gal4 oder Herpes simplex Virus VP16, exprimiert, kann die Expression des Reportergens stark gesteigert werden, wenn das zweite Fusionsprotein mit dem erfindungsgemäßen Polypeptid interagiert. Diese Expressionssteigerung kann man ausnutzen, um neue pharmakologisch aktive Substanzen zu identifizieren, beispielsweise indem man zur Konstruktion des zweiten Fusionsproteins eine cDNA-Bibliothek aus sich regenerierenden Gewebe herstellt. Zudem läßt sich dieses Testsystem zur Rasteruntersuchung von Substanzen ausnutzen, die eine Interaktion zwischen dem erfindungsgemäßen Polypeptid und einer pharmakologisch aktiven Substanz inhibieren. Solche Substanzen verringern die Expression des Reportergens in Zellen, die Fusionsproteine des erfindungsgemäßen Polypeptids und der pharmakologisch aktiven Substanz exprimieren (Vidal und Endoh, 1999, Trends in Biotechnology, 17:374-81). So lassen sich schnell neue Wirkstoffe identifizieren, die zur Therapie von Störungen regenerativer Prozesse eingesetzt werden können.

Weiterhin kann ein Testsystem darauf beruhen, daß erfindungsgemäß verwendbaren Polypeptide oder funktionelle Varianten davon und/oder diese kodierende Nukleinsäuren oder Varianten davon, und/oder gegen erfindungsgemäß verwendbare Polypeptide oder funktionelle Varianten davon gerichtete Antikörper oder Antikörperfragmente an eine Festphase gebunden werden und Substanzen auf Interaktion, beispielsweise Bindung oder Änderung der Konformation getestet werden. Geeignete Systeme wie die Affinitätschromatographie und Fluoreszenzspektroskopie sind dem Fachmann bekannt.

Die Festphasen-gebundenen erfindungsgemäß verwendbaren Polypeptide oder funktionellen Varianten davon oder diese kodierenden Nukleinsäuren oder eine Variante davon, oder gegen ein erfindungsgemäß verwendbares Polypeptid oder eine funktionelle Variante davon gerichteter Antikörper oder ein Antikörperfragment können auch Teil eines Arrays sein.

In einer bevorzugten Ausführungsform der Erfindung kann mindestens ein erfindungsgemäß verwendbares Polypeptid und/oder eine dieses kodierende Nukleinsäure und/oder mindestens ein erfindungsgemäß verwendbarer Antikörper oder Antikörperfragment in Form auf einem Trägermaterial fixiertem Array zur Analyse im Zusammenhang mit Erkrankungen von Hautzellen und/oder von Wundheilung und/oder deren krankhaften Störungen eingesetzt werden.

Verfahren zur Herstellung von solchen Arrays sind zum Beispiel aus der US 5,744,305 mittels Festphasenchemie und photolabilen Schutzgruppen bekannt. Diese Arrays können ebenfalls mit Substanzen oder Substanzbibliotheken in Kontakt gebracht werden und auf Interaktion, beispielsweise Bindung oder Konformationsänderung, getestet werden.

So kann beispielsweise eine zu testende Substanz einen detektierbaren Marker enthalten, beispielsweise kann die Substanz radioaktiv markiert, Fluoreszenz-markiert oder Lumineszenz-markiert sein. Weiterhin können Substanzen an Proteine gekoppelt werden, die eine indirekte Detektion erlauben, beispielsweise über enzymatische Katalyse mittels eines Peroxidase-Assays mit einem chromogenen Substrat oder durch Bindung eines detektierbaren Antikörpers. Änderungen der Konformation eines erfindungsgemäß verwendbaren Polypeptide durch Interaktion mit einer Testsubstanz können beispielsweise durch Änderung der Fluoreszenz eines endogenen Tryptophan-Restes im Polypeptid nachgewiesen werden.

Pharmakologisch aktive Substanzen der erfindungsgemäß verwendbaren Polypeptide können auch Nukleinsäuren sein, die über Selektionsverfahren, wie beispielsweise SELEX (siehe Jayasena, 1999, Clin. Chem. 45:1628-50; Klug und Famulok, 1994, M. Mol. Biol. Rep. 20:97-107; Toole et al., 1996, US 5,582,981) isoliert wereden. Im SELEX-Verfahren werden typischerweise aus einem großen Pool unterschiedlicher, einzelsträngige RNA Moleküle durch wiederholte Amplifikation und Selektion diejenigen Moleküle isoliert, die an ein erfindungsgemäß verwendbares Polypeptid mit hoher Affinität binden (Aptamere). Aptamere können auch in ihrer spiegelbildlichen Form, beispielsweise als L-Ribonukleotid, synthetisiert und selektioniert werden (Nolte et al., 1996, Nat. Biotechnol. 14:1116-9; Klussmann et al., 1996, Nat. Biotechnol. 14:1112-5). So isolierte Formen haben den Vorteil, das sie nicht von natürlich vorkommenden Ribonukleasen abgebaut werden und daher größere Stabilität besitzen.

In einer weiteren bevorzugten Ausführungsform der Erfindung besteht ein Test zur Identifizierung pharmakologisch aktiver Substanzen darin, daß pharmakologisch aktiven Substanzen getestet werden, ob sie die Expression mindestens einer erfindungsgemäß verwendbaren Nukleinsäure beeinflussen.
Assays zur Identifikation von pharmakologischen Substanzen, die die Expression von Genen beeinflussen, sind dem Fachmann allgemein bekannt (siehe beispielsweise Sivaraja et al., 2001, US 6,183,956).

So können Zellen, die erfindungsgemäß verwendbare Nukleinsäuren exprimieren, beispielsweise HeLa Zellen, als Testsystem zur Analyse der Genexpression *in vitro* kultiviert werden, wobei Hautzellen, insbesondere Keratinozyten, Fibroblasten oder Endothelzellen zu bevorzugen sind. Ein mögliches Testsystem stellt dabei die menschliche Keratinozytenzellinie HaCaT dar, die allgemein erhältlich ist.

Die Analyse der Genexpression erfolgt beispielsweise auf der Ebene der mRNA oder der Proteine. Dabei wird die Menge an erfindungsgemäß verwendbarer Nukleinsäure oder Protein nach Zugabe einer oder mehrerer Substanzen zur Zellkultur gemessen und mit der entsprechenden Menge in einer Kontrollkultur verglichen. Dies geschieht beispielsweise mit Hilfe einer Hybridisierung einer anti-sense Sonde, mit der im Lysat der Zellen enthaltene mRNA von die erfindungsgemäß verwendbaren Targetgenen nachgewiesen werden kann. Die Hybridisierung kann beispielsweise über die Bindung eines spezifischen Antikörpers an den mRNA-Sonden Komplex quantifiziert werden (siehe Stuart and Frank, 1998, US 4,732,847). Dabei ist es möglich, die Analyse im Hochdurchsatzverfahren durchzuführen und sehr viele Substanzen auf ihre Eignung als Modulator der Expression von erfindungsgemäß verwendbaren Nukleinsäuren zu analysieren (Sivaraja et al., 2001, US 6,183,956). Die zu analysierenden Substanzen können dabei aus Substanzbibliotheken (siehe z.B. DE19816414, DE19619373) entnommen werden, die beispielsweise bis zu mehrere tausend, oft sehr heterogene Substanzen enthalten. Alternativ kann zunächst die gesamt RNA oder mRNA aus Zellen isoliert werden, und anschließend die absolute Menge oder der relative Anteil der mRNA von erfindungsgemäß verwendbaren Targetgenen beispielsweise mit Hilfe der quantitativen RT-PCR (siehe EP 0 200 362; Wittwer et al., 1997, BioTechniques 22:130-8; Morrison et al., 1998, BioTechniques 24: 954-62) oder des RNAse Protection Assays (siehe z. B. Sambrook et al., 1989, Molecular cloning: A Laboratory Manual, Cold Spring Harbor, Cold Spring Harbor Laboratory Press, New York, Kapitel 7; EP 0 063 879) bestimmt werden. Eine weitere Möglichkeit stellt die Analyse der Proteinmenge im Zelllysat mit Antikörpern dar, die erfindungsgemäß verwendbare Polypeptide erkennen. Hier kann die Quantifizierung kann durch Methoden erfolgen, beispielsweise mit Hilfe eines ELISAs oder eines Western-Blots, die allgemein bekannt sind. Um die Spezifität der Substanzen auf die Expression erfindungsgemäß verwendbarer Nukleinsäuren zu bestimmen, kann der Einfluss von Substanzen auf die Targetgen Expression mit dem Einfluss auf die Expression andere Gene, wie beispielsweise Gene des Stoffwechsels wie GAPDH, verglichen werden. Dies kann entweder in separaten Analysen erfolgen, oder parallel zur Analyse der erfindungsgemäß verwendbaren Nukleinsäuren.

Die mit Hilfe der erfindungsgemäß verwendbaren Testverfahren identifizierten pharmakologisch aktiven Substanzen können, gegebenenfalls kombiniert oder zusammen mit geeigneten Zusatz- und/oder Hilfsstoffen, zur Herstellung eines Diagnostikums oder Arzneimittels zur Diagnose, Prävention und/oder Behandlung von Erkrankungen von Hautzellen, von Wundheilung und/oder deren krankhaften Störungen eingesetzt werden.

Ein weiterer Gegenstand der Erfindung betrifft die Verwendung mindestens eines erfindungsgemäß verwendbaren Polypeptids oder einer funktionellen Variante davon und/oder dieses kodierende Nukleinsäure oder eine Variante davon, und/oder eines gegen ein erfindungsgemäß verwendbares Polypeptid oder eine funktionelle Variante davon gerichteten Antikörpers oder eines Antikörperfragments, gegebenenfalls kombiniert oder zusammen mit geeigneten Zusatz-und/oder Hilfsstoffen, zur Herstellung eines auf einem Trägermaterial fixierten Arrays zur Analyse in Zusammenhang mit Erkrankungen von Hautzellen und/oder von Wundheilung und/oder deren krankhaften Störungen.

Verfahren zur Herstellung von solchen Arrays sind zum Beispiel aus der US 5,744,305 mittels Festphasenchemie und photolabilen Schutzgruppen bekannt.

Die vorliegende Erfindung betrifft weiterhin die Verwendung mindestens eines erfindungsgemäß verwendbaren Polypeptids oder einer funktionellen Variante davon und/oder dieses kodierende Nukleinsäure oder eine Variante davon, und/oder eines gegen ein erfindungsgemäß verwendbares Polypeptid oder gegen eine funktionelle Variante davon gerichteten Antikörpers oder eines Antikörperfragments, gegebenenfalls kombiniert oder zusammen mit geeigneten Zusatz-und/oder Hilfsstoffen, in Form eines Arrays zur Analyse in Zusammenhang mit Erkrankungen von Hautzellen, bei der Wundheilung und/oder deren krankhaften Störungen.

Zur Analyse in Zusammenhang mit Erkrankungen von Hautzellen und/oder von Wundheilung und/oder deren krankhaften Störungen können beispielsweise auch DNA-Chips und/oder Protein-Chips, die mindestens eine Nukleinsäure, mindestens ein Polypeptid, und/oder mindestens einen Antikörper oder Antikörperfragment, wie vorangehend beschrieben, umfassen. DNA-Chips sind zum Beispiel aus der US 5,837,832 bekannt.

Die Erfindung soll nun im folgenden anhand der Figuren und Beispiele weiter verdeutlicht werden, ohne daß die Erfindung hierauf eingeschränkt wird.

### Beschreibung der Tabellen, Figuren und Sequenzen:

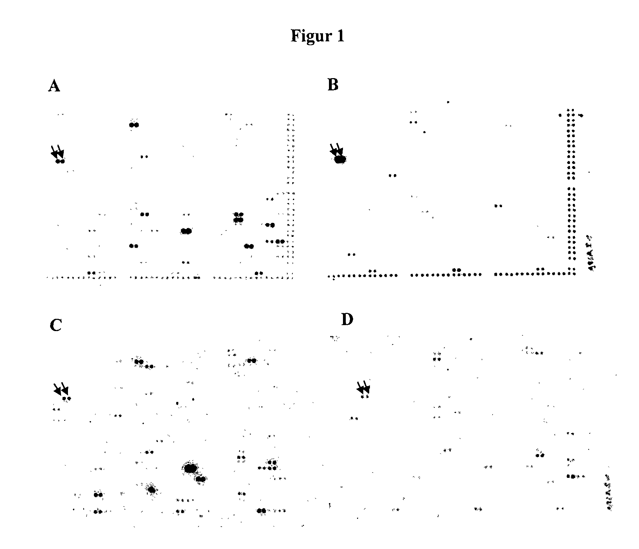

- Figur 1:: Autoradiogramme der Hybridisierungen von Membranen (Maus ATLAS Array, Clontech Laboratories GmbH, Heidelberg) mit gleichem Muster von aufgebrachten cDNA-Fragmenten mit vier verschiedenen Sonden. Alle Sonden wurden aus cDNAs hergestellt, die aus subtraktiven Hybridisierungen stammten. A: wundspezifische Sonde (Subtraktion Wunde versus intakte Haut), B: hautspezifische Sonde (Subtraktion intakte Haut versus Wunde), C: Sonde spezifisch für schlecht heilende Wunden (Subtraktion Wunde Dexamethason behandelte Tiere versus Wunde Kontrolltiere), D: Sonde spezifisch für gut heilende Wunden (Subtraktion Wunde Kontrolltiere versus Wunde Dexamethason behandelte Tiere). Die Positionen der TTF-1 cDNAs (jeweils doppelt aufgetragen) sind mit Pfeilen gekennzeichnet.
- Figur 2:: Vergleich der Polypeptidsequenz der identifizierten Pro-teine von SW1136 aus Maus (murine) und Mensch (human). Abweichungen zur humanen Sequenz von SW1136 sind markiert.
- Figur 3:: Vergleich der Polypeptidsequenz der identifizierten Proteine von SW1295 aus Maus (murine) und Mensch (human). Abweichungen zur humanen Sequenz von SW1295 sind markiert.
- Tabelle 1:: Tabellarische Aufstellung der veränderten Expression verschiedener wundheilungsrelevanter Gene in Wunden von 10 Wochen alten BALB/c Mäusen und in Wunden von jungen (4 Wochen alt) und alten (12 Monate) Mäusen; sowie in intakten Haut und Wunden von Dexamethasonbehandelten, schlecht heilenden Wunden und von Kontroll-Mäusen.
- Tabelle 2:: Tabellarische Aufstellung der veränderten Expression verschiedener wundheilungsrelevanter Gene in intakten Haut und in Wunden von Mäusen mit Diabetes und von Kontroll-Mäusen.
- Tabelle 3:: Tabellarische Übersicht über die bei der Analyse der Genexpression während des Wundheilungsprozesses identifizierten Polypeptidsequenzen mit unbekannter biologischer Funktion und ihre cDNAs und Zugangsnummern oder SEQ ID Nummern.
- Tabelle 4:: Tabellarische Übersicht über die bei der Analyse der Genexpression während des Wundheilungsprozesses identifizierten Polypeptidsequenzen mit bereits bekannten und beschriebenen Funktionen und ihre cDNAs und Zugangsnummern oder SEQ ID Nummern.
- Tabelle 5:: Tabellarische Übersicht über die bei der Analyse der Genexpression während des Wundheilungsprozesses zusätzlich identifizierten Polypeptidsequenzen mit bereits bekannten und beschriebenen Funktionen und ihre cDNAs und Zugangsnummern oder SEQ ID Nummern.
- Tabelle 6:: Analyse der Kinetik wundrelevanter Gene während der Wundheilung der Maus mittels "TaqMan Analyse".
- Tabelle 7:: Analyse der Kinetik wundrelevanter Gene bezüglich Cyclophilin während der Wundheilung des Menschen mittels "TaqMan Analyse".
- Tabelle 8:: Analyse der relativen Expression erfindungsgemäß verwendbarer Gene in Wundgrund bzw. Wundrand relativ zu intakter Haut von Ulkus-Patienten.
- Tabelle 9:: Analyse der Expression erfindungsgemäß verwendbarer Gene in intakter Haut gesunder Probanden, sowie in läsionaler und nicht-läsionaler Haut von Psoriasis-Patienten.

SEQ ID Nr. 1 bis SEQ ID Nr. 58 und SEQ ID Nr. 63 bis SEQ ID Nr. 73 und SEQ ID Nr. 80 bis SEQ ID Nr. 84 zeigen die erfindungsgemäßen Polypeptid- oder cDNA-Sequenzen aus Mensch oder Maus.

SEQ ID Nr. 59 bis SEQ ID Nr. 62 und SEQ ID Nr. 74 bis SEQ ID Nr. 79 zeigen DNA-Sequenzen von Oligonukleotiden, die für die Versuche der vorliegenden Erfindung verwendet wurden.

### Beispiele

### Beispiel 1: Anreicherung von wundrelevanter cDNA mittels subtraktiver Hybridisierung und Identifizierung von TTF-1 als wundrelevantes Gen

Aus intakter Haut und aus Wundgewebe (Verwundung am Rücken 1 Tag vor Gewebeentnahme durch Scherenschnitt) von BALB/c Mäusen wurde durch Standardmethoden (Chomczynski und Sacchi, 1987, Anal. Biochem. 162: 156-159, Chomczynski und Mackey, 1995, Anal. Biochem. 225: 163-164) Gesamt-RNA isoliert. Um Gewebe von Mäusen mit schlecht heilenden Wunden zu gewinnen, wurden BALB/c Mäuse vor der Verwundung mit Dexamethason (Injektion von 0,5 mg Dexamethason in isotonischer Salzlösung pro kg Körpergewicht zwei mal pro Tag für 5 Tage) behandelt. Die RNAs wurden dann mit Hilfe einer reversen Transkriptase in cDNA umgeschrieben. Die cDNA Synthese erfolgte mit dem "SMART PCR cDNA Synthesis Kit" der Firma Clontech Laboratories GmbH, Heidelberg, nach Anweisungen des entsprechenden Manuals.

Um diejenigen cDNAs zu identifizieren, die mit unterschiedlicher Häufigkeit in den cDNA Pools vorkamen, wurde eine subtraktive Hybridisierung (Diatchenko et al., 1996, Proc. Natl. Acad. Sci. U.S.A. 93: 6025-30) durchgeführt. Dies erfolgte mit dem "PCR-Select cDNA Subtraction Kit" der Firma Clontech Laboratories GmbH, Heidelberg, nach Anweisungen des entsprechenden Manuals, wobei die Abtrennung überschüssiger Oligonukleotide nach der cDNA Synthese über Agarose-Gelelekrophorese erfolgte. Es wurden vier für wundrelevante Gene angereicherte cDNA Pools angelegt, wobei ein Pool für cDNA Fragmente angereichert war, die im Wundgewebe im Vergleich zu intakter Haut stärker exprimiert sind ("wundspezifischer cDNA Pool"), ein Pool war angereichert an cDNA Fragmenten, die in intakter Haut im Vergleich zu Wundgewebe stärker exprimiert sind ("hautspezifischer cDNA Pool"), ein Pool war angereichert an cDNA Fragmenten, die in gut heilenden Wunde im Vergleich zu schlecht heilenden Wunden stärker exprimiert sind ("gut heilender cDNA Pool") und ein Pool war angereichert an cDNA Fragmenten, die in schlecht heilenden Wunden im Vergleich zu gut heilenden Wunden stärker exprimiert sind ("schlecht heilender cDNA Pool").

Um diejenigen Gene zu identifizieren, die in den wundheilungsrelevanten cDNA-Pools enthalten waren, wurde das Vorhandensein der entsprechenden cDNAs in den Pools im "Reverse Northern Blot" analysiert. Hier werden cDNA-Fragmente auf Membranen in Form von Arrays von vielen verschiedenen cDNAs fixiert, und mit einem komplexen Gemisch radioaktiv markierter cDNA hybridisiert (Sambrook et al., 1989, Molecular cloning: A Laboratory Manual, Cold Spring Harbor, Cold Spring Harbor Laboratory Press, New York, kapitel 9 Seite 9.47 bis9.58 und kapitel 10 Seite 10.38 bis 10.50; Anderson and Young: Quantitative filter hybridisation; in: Nucleic Acids Hybridisation, A Practical Approach, 1985, Eds. Hames and Higgins, IRL Press Ltd.; Oxford, Kapitel 4, Seite 73 bis 112). Beispielsweise wurden kommerziell erhältliche Membranen verwendet (Maus ATLAS Array, Clontech).

Zur Herstellung geeigneter Hybridisierungssonden wurden die subtrahierten cDNA Pools mit der Restriktionsendonuklease RsaI behandelt und über Agarosegelelektrophorese gereinigt (Sambrook et al., supra, Kapitel 6, Seite 6.1 bis 6.35), um die cDNA- Synthese- und Amplifikationsprimer (siehe Manual "PCR-Select cDNA Subtraction Kit", Clonetech) abzutrennen. Die cDNAs wurde dann mit der "random-hexamer priming" Methode (Feinberg und Vogelstein, 1983, Anal. Biochem. 132:6-13) radioaktiv markiert, um Hybridisierungssonden herzustellen.

Die Membran wurde für 30 min bei 65 °C in 25 ml Hybridisierungslösung vorinkubiert (25 mM Natriumphosphat, pH = 7,5, 125 mM NaCl, 7% SDS). Die Hybridisierungssonde wurde 10 min bei 100°C denaturiert, anschließend auf Eis abgekühlt, ca. 100 CPM ("counts per minute") pro ml zur Hybridisierungslösung gegeben und die Hybridisierung für 16 Stunden bei 65 °C im Hybridisierungsofen durchgeführt. Danach wurde die Membran zweimal für 10 min mit der Hybridisierungslösung ohne Sonde bei 65 °C gewaschen. Anschließend wurde die Membran mehrfach für jeweils 10 min in Waschlösung (2,5 mM Natriumphosphat, pH = 7,5, 12,5 mM NaCl, 0,7% SDS) bei 65°C gewaschen, bis in der abgegossenen Lösung keine Aktivität mehr nachgewiesen werden konnte. Die radioaktiven Signale wurden mit einem Phosphoimager (BioRad, Quantitiy One®) ausgewertet (Figur 1). Danach wurden diejenigen cDNAs ausgewählt, die mit den verschiedenen Sonden unterschiedliche Signalintensitäten ergaben. Dabei ergab sich an der Position von TTF-1 auf der Membran eine deutlich stärkere Signalintensität mit der Hybridisierungssonde des hautspezifischen cDNA Pools im Vergleich zum wundspezifischen cDNA Pool (Figur 1 A, B). Die Analyse des Experiments, bei dem parallel Hybridisierungen mit einem schlecht heilenden cDNA Pool und einem gut heilenden cDNA Pool durchgeführt wurden, ergab, daß die Hybridisierungssonde des schlecht heilenden cDNA Pools an der Position von TTF-1 signifikant stärker hybridisierte (Figur 1 C, D). Somit wurde in zwei unterschiedlichen Wundheilungszuständen eine differentielle Expression von TTF-1 beobachtet.

### Beispiel 2: Verifikation des Expressionsmusters von TTF-1 mittels "real time quantitative RTPCR"

Eine Verifikation der differentiellen Expression der vorangehend beschriebenen Nukleinsäuren als auch die Untersuchung weiterer Wundheilungszustände erfolgte über eine real-time RTPCR im ABI Prism 7700 Sequence Detection System (PE Applied Biosystems). Das Gerät war mit der ABI Prism 7200/7700 SDS-Software Version 1.6.3 (1998) ausgestattet. Der Nachweis von PCR Produkten erfolgte während der Amplifikation der cDNA mit Hilfe des Farbstoffs SYBR Green 1, dessen Fluoreszenz durch Bindung an doppelsträngige DNA stark erhöht wird (Karlsen et al. 1995, J. Virol. Methods. 55: 153-6; Wittwer et al., 1997, BioTechniques 22:130-8, Morrison et al., 1998, BioTechniques 24: 954-62). Basis für die Quantifizierung ist der PCR Zyklus ("threshold cycle", CT -Wert), der erreicht ist, wenn das Fluoreszenzsignal einen definierten Schwellenwert übersteigt. Die Auswertung erfolgt über die Δ-CT-Methode (User Bulletin #2, Relative Quantitation of Gene Expression, PE Applied Biosystems, 1997). Die Abundanzen der cDNAs wurden relativ zu einer endogenen Referenz (GAPDH) bestimmt. Die Ergebnisse sind in den Tabellen 1 und 2 dargestellt.

Um Gewebe von Mäusen mit schlecht heilenden Wunden zu gewinnen, wurden BALB/c Mäuse vor der Verwundung mit Dexamethason (Injektion von 0,5 mg Dexamethason in isotonischer Salzlösung pro kg Körpergewicht zwei mal pro Tag für 5 Tage) behandelt. Um Gewebe von jungen und alten Mäusen zu gewinnen, wurden 1-Tageswunden von 4 Wochen alten und 12 Monate alten BALB/c Mäusen verwendet. Um Wundgewebe von Mäusen mit Diabetes zu erhalten, wurden 1 Tageswunden von 10 Wochen alten C57BL/Ks*-db/db*/Ola Mäuse verwendet. Gesamt-RNA wurde wie oben beschrieben aus Haut und Wundgewebe gewonnen und 1 µg Gesamt-RNA wurde mit dem TaqMan Reverse Transcription Reagents Kit (Perkin Elmer) nach den Empfehlungen des Herstellers (SYBR Green PCR and RT-PCR Reagents-Protokol, Perkin Elmer Applied Biosystems, 1998) in einem Thermocycler (GeneAmp PCR-System 9700, PE) revers transkribiert. Die Primer für die Amplifikation der TTF-1 cDNA (*TTF-1*-Primer 1: CGAGCGCTACATTGTCGCT (SEQ ID Nr. 59), *TTF-1*-Primer 2: GTCTTAAATTTGCTTGTGCCCC (SEQ ID Nr. 60) und der Referenz (GAPDH-Primer1: ATCAACGGGAAGCCCATCA (SEQ ID Nr. 61), GAPDH-Primer2: GACATACTCAGCACCGGCCT (SEQ ID Nr. 62)) wurden anhand der vorangehend beschriebenen Nukleinsäure und der bekannten Sequenz von GAPDH mit der Primer-Express-Software für Macintosh PPC Version 1.0 (PE Applied Biosystems, P/N 402089, 1998) ausgewählt. Für die PCR wurde das SYBR Green PCR Core Reagents Kit (4304886, PE Applied Biosystems) verwendet. Die Konzentration der Primer in der PCR wurde zunächst im Bereich von 50 nM bis 600 nM optimiert und die Spezifität der PCR durch Analyse der Länge der amplifizierten Produkte in einer Agarose-Gel Elekrophorese geprüft. Anschließend wurde mittels einer Verdünnungsreihe die Effizienz der PCR-Systeme ermittelt (User Bulletin #2, Relative Quantitation of Gene Expression, PE Applied Biosystems, 1997). Dabei ergab sich, daß fiir beide cDNAs die Effizienz der Amplifikation bei 100% lag, d. h. bei jeder 1:2 Verdünnung der cDNA wurde ein Zyklus mehr benötigt, um den Fluoreszensschwellenwert zu überschreiten.

Für die Quantifizierung wurden je Ansatz cDNA aus 10 ng revers transkribierter Gesamt-RNA in einem Gesamtvolumen von 25 µl amplifiziert. Die Laufbedingungen für die PCR entsprachen den Angaben des Herstellers (PE Applied Biosystems, SYBR Green PCR and RT-PCR Reagents Protocol, 1998). Die CT-Werte wurden analysiert und die Abundanz von TTF-1 relativ zu GAPDH wurde berechnet. Dabei bestätigte sich zum Einen die Abnahme von *TTF-1* in Wunden im Vergleich zu intakter Haut von Kontrolltieren als auch von jungen Mäusen (Tabelle 1, vergleiche Figur 1 A, B). Zum Anderen wurde wiederum eine verstärkte Expression von TTF-1 in schlecht heilenden Wunden im Vergleich zu gut heilenden Wunden von Kontrolltieren beobachtet (Tabelle 1, vergleiche Figur 1 B, C). Die Untersuchung weiterer Wundheilungszustände ergab, daß TTF-1 in intakter Haut und in Wunden von alten Mäusen sowie in Wunden von Mäusen mit Diabetes im Vergleich zu Wunden von Kontrollmäusen nicht signifikant unterschiedlich exprimiert wird (Tabelle 1, Tabelle 2). Somit konnte die Regulation der Expression von TTF-1 in unterschiedlichen Wundheilungszuständen verifiziert werden.

### Beispiel 3: Identifizierung von MCP-2 als wundrelevantes Gen mittels vergleichendem Auszählen von Klonen in cDNA Bibliotheken durch Analyse von Restriktionsfragmentmustern

BALB/c Mäuse wurden mit Dexamethason (Injektion von 0,5 mg Dexamethason pro kg Körpergewicht zwei mal pro Tag für 5 Tage) behandelt und anschließend verwundet um Gewebe von Mäuse mit schlecht heilenden 1-Tageswunden zu gewinnen. Die Herstellung der cDNA aus der aus dem Gewebe isolierten Gesamt-RNA, sowie alle weiteren Schritte wurden wie in EP 0 965 642 beschrieben durchgeführt: die cDNA wurde gerichtet in einen geeigneten Vektor kloniert und die erhaltenen Plasmide in einen geeigneten *E*. *coli* Stamm transformiert. Je 100 *E*. *coli* Klone wurden gemischt und die Plasmid-DNA isoliert. Die DNA wurde in 3 Portionen aufgeteilt und anschließend mit der Restriktionsendonuklease BglI hydrolysiert und mit je einem Fluoreszenz-Farbstoff markiert. Jede Portion wurde anschließend auf 2 Portionen aufgeteilt und dann mit je einer der Restriktionsendonukleasen Bfal, DpnI, Rsal, DdeI, AluI und HinfI hydrolysiert. Die portionierten Nukleinsäuren wurden elektrophoretisch aufgetrennt und das Muster der aufgetrennten Nukleinsäuren analysiert. Die Restriktionsfragmentmuster der Klongemische wurde mit dem Muster von cDNA Einzelklonanalysen verglichen und die cDNAs somit identifiziert. Es wurden diejenigen Nukleinsäuren ausgewählt, deren Restriktionsfragmentmuster unterschiedlich häufig in cDNA-Pools schlecht heilender und gut heilender Wunden identifiziert wurde. Bei der Analyse von 37000 cDNAs konnte beispielsweise 3 mal das Muster von MCP-2 (DdeI: 196,23±0,3 Basenpaare; AluI: 67,51±0,5 Basenpaare; Hinfl: 349,88±0,7 Basenpaare; BfaI: 531,02±2,0 Basenpaare; DpnI: 245,02±0,3 Basenpaare; RsaI: 254,23±0,3 Basenpaare) im cDNA-Pool schlecht heilender Wunden identifiziert werden, während das Muster im cDNA-Pool gut heilender Wunden nicht beobachtet wurde.

### Beispiel 4: Analyse der Kinetik wundrelevanter Gene während der Wundheilung der Maus mittels "TaqMan Analyse"

Die Kinetik der Regulation der Expression wundrelevanter Gene während der normalen Wundheilung der Maus wurde mittels "TaqMan Analyse" im GeneAmp5700 von Applied Biosystems untersucht. Normal heilende Wundbiopsien von verschiedenen Zeitpunkten nach Verwundung sowie intakte Haut wurde aus 6 mit isotonischer Kochsalzlösung behandelten 10 Wochen alten BALB/c-Mäusen durch Scherenschnitt wie in Beispiel 1 beschrieben gewonnen. Die Isolierung der RNA erfolgte durch Homogenisieren der Biopsien in RNAclean Puffer (AGS, Heidelberg), dem 1/100 Volumenanteil 2-Mercaptoethanol zugesetzt worden war unter Verwendung eines Dispersers. Anschließend wurde die RNA durch zweimaliges Phenolisieren mittels mit Wasser gesättigtem saurem Phenol und in Gegenwart von 1-Brom-3-Chlor-Propan extrahiert. Anschließend wurden eine Isopropanol- und eine Ethanol-Fällung durchgeführt und die RNA mit 75% Ethanol gewaschen. Danach wurde ein DNase I-Verdau der RNA durchgeführt. Hierzu wurden 20 µg RNA (ad 50 µl mit DEPC-behandeltem Wasser) mit 5,7 µl Transkriptions-Puffer (Roche), 1 µl RNase-Inhibitor (Roche; 40 U/µl) und 1 µl DNase I (Roche; 10 U/µl) 20 min bei 37°C inkubiert. Dann wurde wiederum 1 µl DNase I zugegeben und weitere 20 min bei 37°C inkubiert. Anschließend wurde die RNA phenolisiert, Ethanol-gefällt und gewaschen. Alle oben aufgeführten Schritte wurden mit DEPC (Diethylpyrocarbonat)-behandelten Lösungen bzw. Flüssigkeiten durchgeführt soweit diese keine reaktiven Aminogruppen enthielten. Anschließend erfolgte die Herstellung von cDNA aus der extrahierten RNA. Dies erfolgte in Gegenwart von 1 x TaqMan RT-Puffer (Applied Biosystems), 5,5 mM MgCl₂ (Perkin Elmer), je 500 µM dNTPs (Perkin Elmer), 2,5 µM Random Hexamere (Perkin Elmer), 1,25 U/µl MultiScribe Reverse Transcriptase (50 U/µl Perkin Elmer), 0,4 U/µl RNase Inhibitor (20 U/µl, Perkin Elmer), 20 µl RNA (50 ng/µl) und DEPC-behandeltem Wasser (ad 100 µl Volumen). Nach Zugabe der RNA und guter Durchmischung wurde die Lösung auf 2 0,2 ml Gefäße verteilt (je 50 µl) und die reverse Transkription in einem Temperatur-Cycler durchgeführt (10 min bei 25°C; 30 min bei 48°C und 5 min bei 95°C). Die nachfolgende Quantifizierung der cDNA erfolgte mittels quantitativer PCR unter Verwendung des SYBR Green PCR Master Mixes (Perkin Elmer), wobei für jede zu bestimmende cDNA-Spezies eine Dreifachbestimmung (jeweils mit Target-Primern und GAPDH-Primern) durchgeführt wurde. Die Stammlösung für jedes Triplett enthielt bei 57 µl Gesamtvolumen 37,5 µl 2 x SYBR Master Mix, 0,75 µl AmpErase UNG (1 U/µl) und 18,75 µl DEPC-behandeltes Wasser. Pro Dreifachbestimmung wurden zu 57 µl Stammlösung je 1,5 µl Vorwärts- und Rüchwärts-Primer in einem zuvor optimierten Konzentrationsverhältnis hinzugefügt. Je 60 µl der Stammlösung/Primer-Mischung wurde mit 15 µl cDNA-Lösung (2 ng/µl) gemischt und auf 3 Reaktionsgefäße verteilt. Parallel dazu wurde eine Stammlösung mit Primern zur Bestimmung von GAPDH (SEQ ID Nr. 61 und SEQ ID Nr. 62) als Referenz hergestellt, mit weiteren 15 µl der gleichen cDNA-Lösung gemischt und auf 3 Reaktionsgefäße verteilt. Außerdem wurden um eine Standardkurve fiir die GAPDH-PCR zu erstellen verschiedene cDNA-Lösungen als Verdünnungsreihe hergestellt (4 ng/µl; 2 ng/µl; 1 ng/µl; 0,5 ng/µl und 0,25 ng/µl). Je 15 µl dieser cDNA-Löungen wurden mit 60 µl Stammlösung/Primer-Mischung zur Bestimmung von GAPDH gemischt und auf 3 Reaktionsgefäße verteilt. Ebenso wurde jeweils eine Standardkurve für die PCR der zu untersuchenden Gene erstellt; dabei wurden dieselben Verdünnungen, die auch für die GAPDH-Standardkurve zum Einsatz kamen, verwendet. Als Kontrolle diente ein PCR Ansatz ohne cDNA. Zu jeweils 60 µl Stammlösung/Primer-Mischung von jeweils Target und GAPDH wurden je 15 µl DEPC-Wasser gegeben, gemischt und jeweils auf 3 Reaktionsgefäße verteilt. Die Amplifikation der Ansätze wurde im GeneAmp 5700 durchgeführt (2 min bei 50°C; 10 min bei 95°C, gefolgt von 3 Zyklen mit 15 sek bei 96°C und 2 min bei 60°C; danach 37 Zyklen mit 15 sek bei 95°C und 1 min bei 60°C). Die Auswertung erfolgte durch die Bestimmung der relativen Abundanz jedes Target-Gens im Bezug auf die GAPDH-Referenz. Dazu wurde zuerst eine Standardkurve erstellt, indem die C_{T}-Werte der Verdünnungsreihe gegen den Logarithmus der cDNA-Menge im PCR-Ansatz (in ng umgeschriebener RNA) aufgetragen wurden und die Steigung (s) der Geraden ermittelt wurde. Die Effizienz (E) der PCR ergibt sich dann wie folgt: E = 10^{-1/s} - 1. Die relative Abundanz (X) der untersuchten cDNA-Spezies (Y) im Bezug auf GAPDH ist dann: X=(1+E_{GAPDH})^{C}_{T}^{(GAPDH)}/(1+E_{Y})^{C}_{T}^{(Y)}. Anschließend wurden die Zahlenwerte normiert, indem die Menge an cDNA aus intakter Haut der 10 Wochen alten BALB/c Kontrolltiere gleich 1 gesetzt wurde. Die relativen Änderungen der Target-Gen Expression in verschiedenen Wundheilungszuständen sind in Tabelle 6 zusammengestellt. So wird beispielsweise im Falle der Tyrosinkinase Fer deutlich, daß die Expression hauterkrankungsrelevanter Targets während der Wundheilung spezifisch reguliert wird. Bei diesem Target kommt es bereits eine Stunde nach Verwundung zu einer stark verminderten Expression an mRNA die während des gesamten Beobachtungszeitraums von 14 d anhält. Dies zeigt, daß die differentielle Regulation über den gesamten Zeitraum der Wundheilung essentiell für den normalen Verlauf der Wundheilung ist.

Eine ähnliche Kinetik, d.h. eine verminderte Expression über Tage hinweg nach Verwundung wurde bei einer Vielzahl der Gene festgestellt, beispielsweise für KIAA1247, Cystatin C, SW1136, SW1295, Baf57, TSC-22, Split hand foot deleted 1, Nicotinamid N-Methyl-Transferase, UBC9, tsg101, HMG-14, TAK1 und dem Golgi-4-Transmembrane spanning transporter. Dies zeigt, daß die differentielle Regulation dieser Gene während des gesamten Verlaufs der Wundheilung notwendig ist. Es wurden jedoch auch komplexe Kinetiken gemessen, beispielsweise bei der Analyse des Phospholipase Inhibitor, bei dem eine starke Hochregulation sowohl 1 Stunde als auch 7 und 14 Tage nach Verwundung gemessen wurde. Dies zeigt deutlich, daß die differentielle Expression des Phospholipase Inhibitors sowohl zu frühen als auch zu späten Phasen der Wundheilung essentiell für den Verlauf der Wundheilung ist und daß Störungen der Expression und/oder Aktivität des Phospholipase Inhibitors sowohl sofort nach Verwundung aber auch bei späten Phasen der Wundheilung zu Störungen des Wundheilungsprozesses rühren können.

### Beispiel 5: Differentielle Expression wundrelevanter Gene in humanen Wunden

Es sollte nun anhand der normal heilenden Wunde untersucht werden, ob die in Beispiel 4 verifizierte differentielle Regulation der Expression der als wundrelevant identifizierten Gene sich auch beim Menschen beobachten läßt.

Dazu wurden von 6 Patienten 4 mm Biopsien von intakter Haut wie oben beschrieben entnommen, sowie 6 mm Biopsien zu den Zeitpunkten T=1 h, 1 d, 5 d und 14 d. Die Biopsien pro Zeitpunkt wurden gepoolt und die cDNA wie oben beschrieben hergestellt. Danach wurde die Quantifizierung mittels TaqMan Analyse wie oben beschrieben, außer daß die Häufigkeit der zu bestimmenden Target Spezies relativ zu Cyclophilin (EMBL: Y00052) bestimmt wurde. Die dazu verwendeten Primer sind Cyclophilin-Primer 1: TCTTAACCAC CAGATCATTC CTTCT (Seq ID Nr. 78) und Cyclophilin-Primer 2: GGATACTGCG AGCAAATGGG (Seq ID Nr. 79). Die Auswertung des Experiments ist in Tabelle 7 gezeigt. Im Falle von CCR-1 (CCR-1 Primer 1: CCCAATGGGA ATTCACTCAC C (SEQ ID Nr. 76); CCR-1 Primer 2: GCTTCCACTC TCGTAGGCTT TC (SEQ ID Nr. 77)) konnte im Menschen ein starker Anstieg der Expression bis 24 h nach Verwundung gefolgt von einer lansamen Verringerung der CCR-1 Expression nachgewiesen werden. Dies ist konsistent mit der Kinetik der CCR-1 Expression in der Wundheilung der Maus (Tabelle 6). Im Falle des Golgi 4-Transmembrane spanning transporters konnte gezeigt werden, daß die differentielle Regulation der Expression während der Wundheilung sowohl in der Maus als auch im Menschen nachgewiesen werden kann. Somit konnte die Wundheilungs- und Hauterkrankungsrelevanz des Targets wiederum verifiziert werden. Auch bei den anderen untersuchten Genen konnte eine differentielle Regulation nach Verwundung verifiziert werden. Dabei wurden sowohl komplexe Kinetiken mit transienten Veränderungen gemessen werden, wie im Falle von Eps8, Phospholipase Inhibitor, TSC-22, Cathepsin C und HMG-14 als auch ein ständiger Anstieg der Expression über den geamten Beobachtungszeitraum, wie im Falle von KIAA1247 und Cystatin C. Dies beweist, daß die genaue Regulation der Expression und/oder Aktivität dieser Targets essentiell für den normalen Verlauf der Wundheilung sowohl im Menschen als auch in der Maus ist.

### Beispiel 6: Differentielle Expression wundrelevanter Gene in humanen Ulzera

Um zu zeigen, daß die als wundrelevant identifizierten Gene nicht nur bei normal verlaufender Wundheilung sondern auch bei einer Wundheilungsstörung differentiell reguliert ist, wurden Biopsien von Patienten mit chronischen venösen Ulzera (Ulcera venosa) gleichzeitig sowohl von intakter Haut als vom Wundgrund und Wundrand entnommen und auf Expression der Targetgene hin untersucht. Von jeder Gruppe (intakte Haut, Wundrand, Wundgrund) wurden die Biopsien von jeweils 6 Probanden gepoolt. Aus allen Biopsien wurde, wie im Beispiel 4 beschrieben, RNA isoliert, danach DNase I verdaut und anschließend in cDNA umgeschrieben. Die Quantifizierung wundheilungsrelevanter cDNAs erfolgte ebenfalls wie in Beispiel 5 beschrieben, wobei die Menge an Cyclophilin mRNA zur Berechnung der relativen Menge an Targetgen cDNA herangezogen wurde. Die Ergebnisse der Experimente sind in Tabelle 8 zusammengestellt. So wurde beispielsweise bei KIAA1247 eine Fehlregulation der Expression im Ulkus im Vergleich zur normal heilenden Wunde (Tabelle 7) festgestellt: während in der normal heilenden Wunde ein gleichmäßiger Anstieg der KIAA1247 zu beobachten war, wurde am Wundrand eine deutlich verminderte Expression festgestellt. Dies zeigt, daß die differentielle Expression von KIAA1247 nicht nur für die Wundheilung essentiell ist, sondern daß Fehlregulationen zu schweren Wundheilungsstörungen führen können. Das Experiment verdeutlicht, daß KIAA1247 zur Diagnose, Prävention und/oder Behandlung von Wundheilungsstörungen und/oder Hauterkrankungen verwendet werden kann.

### Beispiel 7: Differentielle Regulation erfindungsgemäß verwendbarer Gene in läsionaler und nicht-läsionaler Haut von Psoriasis Patienten im Vergleich zu intakter Haut gesunder Probanden

Es sollte nun anhand von Psoriasis Patienten verifiziert werden, daß erfindungsgemäß verwendbare Gene nicht nur bei der Wundheilung und Wundheilungsstörungen eine wichtige Rolle spielen sondern auch bei anderen Hauterkrankungen. Dazu wurden Psoriasis Patienten 4 mm Stanzbiopsien sowohl von läsionaler als auch nicht-läsionaler Haut wie in Beispiel 5 beschrieben entnommen. Als Kontrolle wurde gesunden Probanden Biopsien intakter Haut entnommen. Die Isolierung der mRNA aus den einzelnen Biopsien erfolgte durch Einbettung der Biopsien in tissue freezing medium (Jung), das Zerkleinern der Biopsie unter Verwendung eines Mikrotoms und die darauffolgende mRNA Isolierung mittels Dynabeads-Oligo dT (Dynal). Die zerkleinerten Biopsien werden zunächst in Lyse-Puffer suspendiert und dann mit dem Polytron homogenisiert. Um die genomische DNA zu zerkleinern wird die Lösung über Qia-Shredder Columns abzentrifugiert und zusätzlich in einer Spritze mit Kanüle mehrmals geschert. Die Dynabeads wurden gemäß den Angaben des Herstellers vorbehandelt und (250 µl der Stammsuspension) mit dem Lyse-Homogenisat gemischt, inkubiert und gewaschen (Endvolumen 250 µl). Die Suspension wurde nun in je eine Portion von 240 µl sowie von 10 µl (als Kontrolle) geteilt. Für die Erststrangsynthese wurden folgende Komponenten gemischt: 20 µl 10x TaqMan RT Puffer, 44 µl 25 mM MgCl₂, 40 µl dNTP-Mix (2,5 mM/dNTP), 87 µl DEPC-H₂O, 4 µl RNase Inhibitor (20 U/µl) und 5 µl MultiScribe Transcriptase (50 U/µl). 195 µl des Reaktionsmixes wurden nun zum 240 µl Ansatz und 20 µl zum Kontrollansatz gegeben, gemischt und 45 min bei 48°C inkubiert. Die Dynabeads wurden anschließend im Magnetständer pelletiert und der Überstand abgenommen. Anschließend wurden 20 µl Tris-HCI Puffer zugegeben und die Suspension 1 min bei 95°C inkubiert. Die Dynabeads wurden sofort im Magnetständer pelletiert und die mRNA im Überstand abgenommen. Die cDNA/Dynabeads wurden anschließend 3x mit TE Puffer gewaschen. Für die Zweitstrangsynthese wurden die cDNA/Dynabeads 2x in 1x EcoPol Puffer gewaschen und eine Lösung folgender Komponenten zugegeben: 23 µl 10x EcoPol Puffer; 4,6 µl dNTP Mix (25 mM/dNTP); 11,5 µl Random Hexamers; 118,7 µl DEPC-H₂O. Die Suspension wurde kurz mit Hilfe des Vortexers gemischt und anschließend 9,2 µl Klenow Fragment (5 U/µl) zugegeben. Zum Ansatz wurden 200 µl dieser Lösung zugegeben, zum Kontrollansatz 20 µl, und die Suspensionen wurden bei 37°C 1 h inkubiert. Die DNA wurde dann bei 94°C 1 min aufgeschmolzen und die Dynabeads im Magnetständer pelletiert. Der Überstand wurde in ein neues Reaktionsgefäß transferiert und das Enzym bei 75°C 10 min inaktiviert. Die im Überstand enthaltene sense DNA-Stränge wurden dann fiir die Taq Man Analyse eingesetzt.

Die TaqMan Analyse wurde wie in Beispiel 5 beschrieben durchgeführt, wobei die Menge an GAPDH (hGAPDH-Primer 1: CCTCCCCTCTTCAAGGGTCTA (SEQ ID Nr. 74); hGAPDH-Primer 2: AGGAGTAAGACCCCTGGACCA (SEQ ID Nr. 75) zur Berechnung der relativen Abundanz der jeweiligen mRNA Spezies in den einzelnen Biopsien herangezogen wurde. Da aus den Hautbiopsien von Psoriasis Patienten, insbesondere aus läsionaler Haut eine weitaus größere Gesamt mRNA Menge isolierbar ist als aus intakter Haut gesunder Probanden, ist eine Normalisierung auf gleiche mRNA Mengen notwendig, wobei die Menge an GAPDH mRNA als Housekeeping Gen als Marker für die Gesamt mRNA-Menge angenommen wurde.

Insgsamt wurden 4 Biopsien von intakter Haut gesunder Probanden analysiert, sowie jeweils 8 Biopsien von läsionaler und nichtläsionaler Haut von Psoriasispatienten. Die Abundanzen der einzelnen Gruppen (intakte Haut, läsionale Haut, nichtläsionale Haut) wurden dann auf die Gesamtzahl der Abundanzen der auf einer Mikrotiterplatte gemessenen cDNAs normiert. Diese Analysen wurden für Eps8 (EMBL: U12535), KIAA1247 (GB: AB033073; WO 99/63088) durchgeführt und MASPIN (EMBL: U04313); die Mittelwerte der Ergebnisse sind in Tabelle 9 zusammengestellt. Es wird deutlich, daß bei diesen erfindungsgemäß verwendbaren Genen eine deutliche und statistisch signifikante (p<0,05, gepaarter t-Test) verminderte Regulation in läsionaler Haut von 8 Psoriasispatienten gegenüber unauffälliger Haut der jeweils selben Patienten zu beobachten ist. Dies verdeutlicht, daß eine Fehlregulation dieser Gene zu Hauterkrankungen führen kann und daß daher die erfindungsgemäß verwendbaren Gene zur Prävention und/oder Behandlung und/oder Diagnose von Hauterkrankungen geeignet sind. Im Falle von Psoriasispatienten ist die Expression und/oder Aktivität von KIAA1247 und/oder Eps8 und/oder MASPIN zu modulieren, vorzugsweise zu aktivieren. Bevorzugt ist dabei die Modulation in der Haut, insbesondere der läsionalen Haut der Psoriasispatienten.

Am Beispiel KIAA1247 sollte nun der Zusammenhang zwischen Fehlregulation des Gens und Psoriasis Erkrankung nachgewiesen werden. Dabei wurde als Meß-parameter die Leitfähigkeit der Haut (ein Maß für die Feuchtigkeitder Haut) festgestellt und mit der KIAA1247 Expression des Gens in dieser Hautpartie verglichen. Die Leitfähigkeit der Haut wurde unter Verwendung eines Corneometers nach Angaben des Herstellers (Courage and Khazaka Electronics) bestimmt. Dabei wurde festgestellt, daß eine statistisch signifikante positive Korrelation (p=0,000659, Pearson Product Moment Correlation) zwischen Leitfähigkeit der jeweils untersuchten Biopsie und der KIAA1247-Expression zu beobachten ist: in sehr trockenen Biopsien von läsionaler Psoriasishaut mit geringer Leitfähigkeit wurde eine entsprechend geringe KIAA1247 mRNA Expression gemessen, während in feuchterer Haut, d.h. in unauffälliger Haut der Psoriasispatienten und in intakter Haut gesunder Probanden eine deutlich stärkere KIAA1247 Expression nachweisbar ist. Dies verifiziert die Relevanz der KIAA1247 Expression für die Pathogenese von Hauterkrankungen.

**Tabelle 1**

| **Verändert exprimierte Gene** | **Intakte Haut Kontrolltiere** | **Wunde Kontrolltiere** | **Intakte Haut Dexamethason** | **Wunde Dexamethason** |
|---|---|---|---|---|
| TTF-1 | 1,00 | 0,81 | 1,00 | 1,31 |
| CCR-1 | 1,00 | 31,84 | 0,45 | 76,74 |
| MASPIN | 1,00 | 0,76 | 1,00 | 1,03 |
| B-Raf | 1,00 | 0,77 | 0,77 | 0,71 |
| Prothymosin alpha | 1,00 | 0,97 | 0,91 | 1,90 |
| Eps8 | 1,00 | 0,70 | 0,60 | 0,20 |
| KIAA1247 | 1,00 | 0,30 | 0,90 | 0,20 |
| Cystatin C | 1,00 | 0,77 | 0,74 | 2,21 |
| SW1136 | 1,00 | 0,83 | 0,58 | 1,43 |
| SW1295 | 1,00 | 0,80 | 0,55 | 4,39 |
| BAF57 | 1,00 | 0,98 | 0,93 | 0,54 |
| EAT/MCL-1 | 1,00 | 1,99 | 0,79 | 2,55 |
| Phospholipase Inhibitor | 1,00 | 0,19 | 1,35 | 0,23 |
| TSC-22 | 1,00 | 0,60 | 0,56 | 2,31 |
| Split hand/foot deleted 1 | 1,00 | 1,89 | 0,98 | 10,53 |
| Gamma Sarcoglycan | 1,00 | 0,47 | 1,51 | 0,46 |
| Nicotinamid N-Methyltransferase | 1,00 | 0,93 | 1,00 | 2,15 |
| Golgi 4-Transmembrane spanning transporter | 1,00 | 0,57 | 0,28 | 2,00 |
| UBC9 | 1,00 | 0,95 | 1,06 | 1,91 |
| Cathepsin C | 1,00 | 1,68 | 1,58 | 1,09 |
| tsg101 | 1,00 | 0,94 | 1,03 | 2,06 |
| DAD-1 | 1,00 | 1,07 | 0,93 | 1,93 |
| HMG-14 | 1,00 | 0,61 | 0,43 | 2,11 |
| TAK1 | 1,00 | 0,64 | 0,90 | 0,46 |
| IL-5Ralpha | 1,00 | 5,89 | 0,84 | 1,12 |
| Fer | 1,00 | 0,72 | 0,46 | 0,33 |

| **Verändert exprimierte Gene** | **Intakte Haut Junge Mäuse** | **Wunde Junge Mäuse** | **Intakte Haut Alte Mäuse** | **Wunde Alte Mäuse** |
|---|---|---|---|---|
| TTF-1 | 1,87 | 0,78 | 0,84 | 0,65 |
| CCR-1 | 1,07 | 22,22 | 0,84 | 24,18 |
| MASPIN | 2,00 | 0,39 | 1,18 | 0,45 |
| B-Raf | 1,29 | 0,63 | 0,61 | 0,70 |
| Prothymosin alpha | 1,36 | 0,67 | 0,78 | 0,48 |
| Eps8 | 0,80 | 0,40 | 0,60 | 0,60 |
| KIAA1247 | 2,20 | 0,70 | 1,00 | 0,20 |
| Cystatin C | 0,76 | 0,37 | 0,91 | 0,26 |
| SW1136 | 1,05 | 0,33 | 0,44 | 0,26 |
| SW1295 | 0,73 | 0,50 | 0,70 | 0,35 |
| BAF57 | 1,82 | 0,54 | 0,70 | 0,92 |
| EAT/MCL-1 | 2,67 | 1,79 | 1,42 | 1,12 |
| Phospholipase Inhibitor | 6,03 | 0,11 | 0,59 | 0,23 |
| TSC-22 | 1,23 | 0,42 | 0,42 | 0,55 |
| Split hand/foot deleted 1 | 1,48 | 0,94 | 0,83 | 0,55 |
| Gamma Sarcoglycan | 0,43 | 0,48 | 0,44 | 0,14 |
| Nicotinamid N-Methyltransferase | 1,14 | 0,86 | 0,92 | 0,78 |
| Golgi 4-Transmembrane spanning transporter | 0,53 | 0,26 | 0,27 | 0,11 |
| UBC9 | 1,76 | 0,94 | 1,40 | 0,64 |
| Cathepsin C | 2,20 | 1,61 | 0,75 | 0,89 |
| tsg101 | 1,80 | 0,92 | 0,96 | 0,48 |
| DAD-1 | 1,32 | 1,04 | 0,87 | 0,61 |
| HMG-14 | 1,03 | 0,40 | 0,50 | 0,20 |
| TAK1 | 1,75 | 0,74 | 0,93 | 0,74 |
| IL-5Ralpha | 1,38 | 1,66 | 2,61 | 1,74 |
| Fer | 0,48 | 0,22 | 0,40 | 0,37 |

**Tabelle 2**

| **Verändert exprimierte Gene** | **Intakte Haut Kontroll Mäuse** | **Wunde Kontroll Mäuse** | **Intakte Haut Diabetes Mäuse** | **Wunde Diabetes Mäuse** |
|---|---|---|---|---|
| TTF-1 | 1,00 | 0,61 | 1,57 | 0,54 |
| CCR-1 | 1,00 | 23,51 | 2,26 | 20,66 |
| MASPIN | 1,00 | 0,30 | 1,54 | 0,38 |
| B-Raf | 1,00 | 1,25 | 1,66 | 1,05 |
| Prothymosin alpha | 1,00 | 0,58 | 1,26 | 0,83 |
| Eps8 | 1,00 | 2,50 | 0,70 | 1,40 |
| KIAA1247 | 1,00 | 0,30 | 1,50 | 0,50 |
| Cystatin C | 1,00 | 0,49 | 1,35 | 0,57 |
| SW1136 | 1,00 | 0,31 | 1,15 | 0,48 |
| SW1295 | 1,00 | 0,39 | 1,00 | 0,65 |
| BAF57 | 1,00 | 0,45 | 1,15 | 0,51 |
| EAT/MCL-1 | 1,00 | 1,36 | 1,54 | 1,56 |
| Phospholipase Inhibitor | 1,00 | 0,31 | 1,14 | 0,28 |
| TSC-22 | 1,00 | 0,43 | 1,70 | 0,60 |
| Split hand/foot deleted 1 | 1,00 | 0,41 | 0,35 | 0,79 |
| Gamma Sarcoglycan | 1,00 | 0,04 | 0,33 | 0,08 |
| Nicotinamid N-Methyltransferase | 1,00 | 0,77 | 3,26 | 1,88 |
| Golgi 4-Transmembrane spanning transporter | 1,00 | 0,22 | 0,35 | 0,40 |
| UBC9 | 1,00 | 0,63 | 1,66 | 0,63 |
| Cathepsin C | 1,00 | 0,89 | 0,58 | 0,34 |
| tsg101 | 1,00 | 0,64 | 1,53 | 0,53 |
| DAD-1 | 1,00 | 0,96 | 1,84 | 1,43 |
| HMG-14 | 1,00 | 0,31 | 1,56 | 0,58 |
| TAK1 | 1,00 | 0,35 | 1,30 | 0,46 |
| Fer | 1,00 | 0,27 | 1,35 | 0,31 |

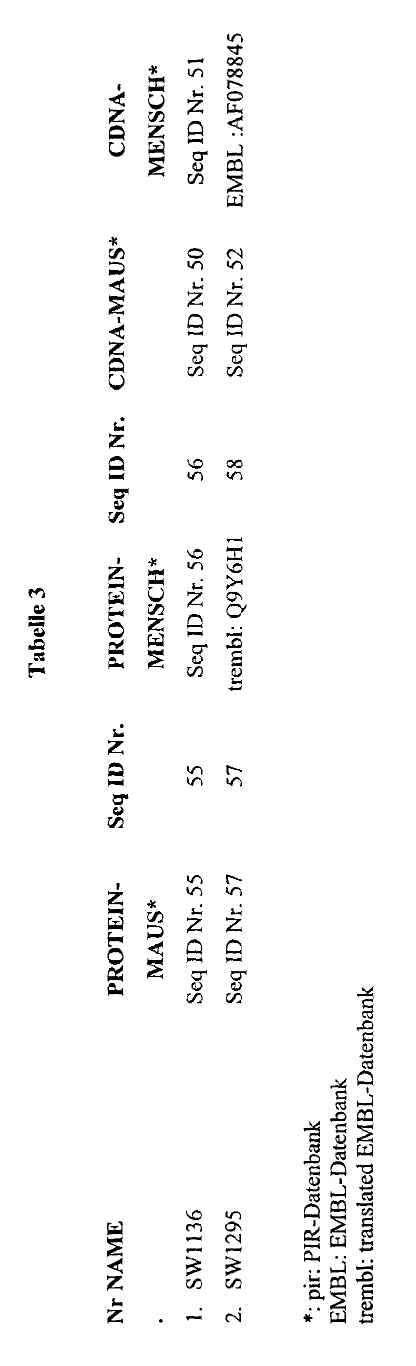

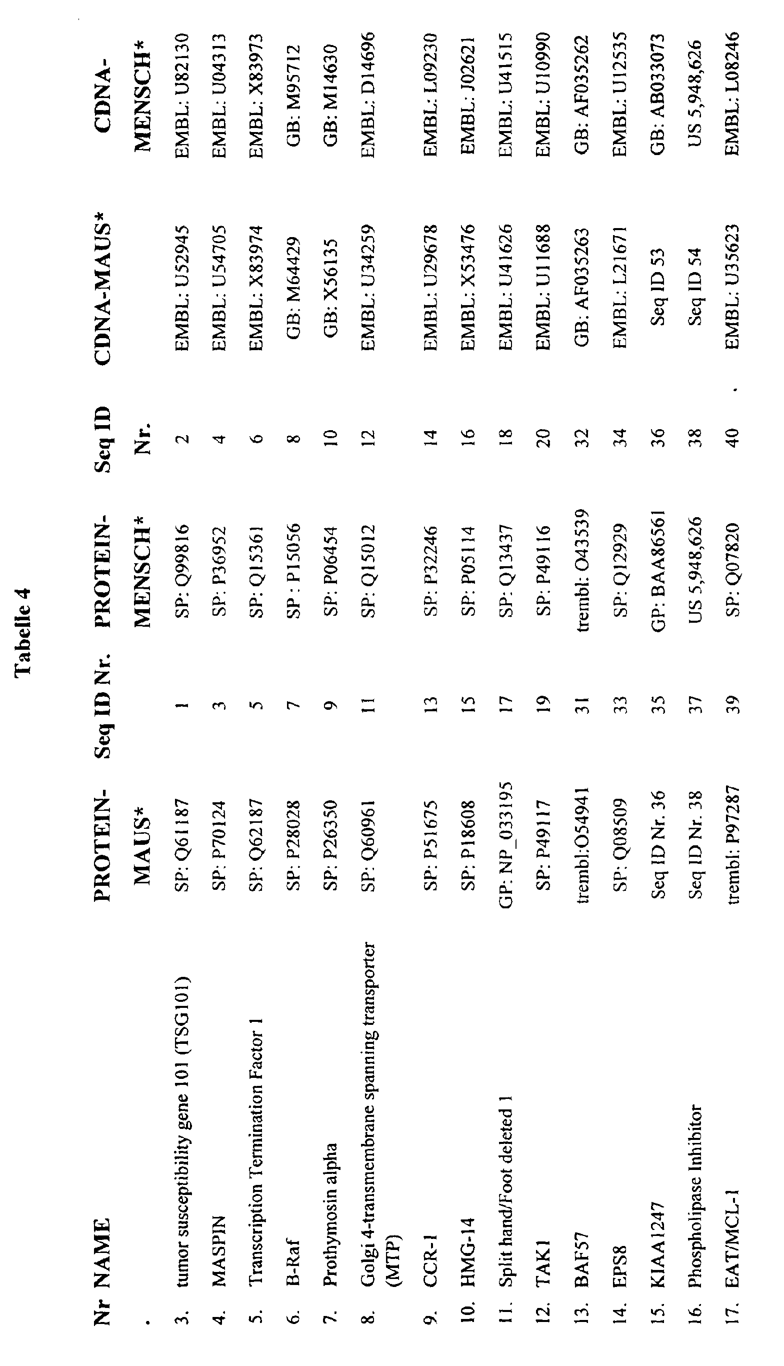

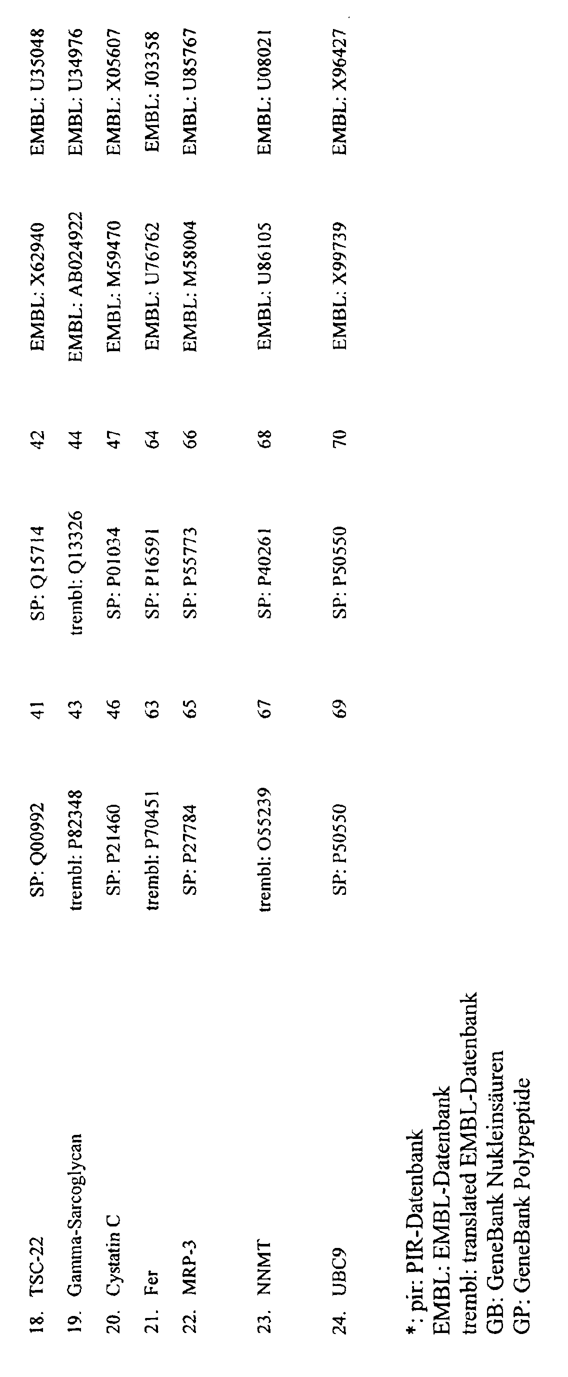

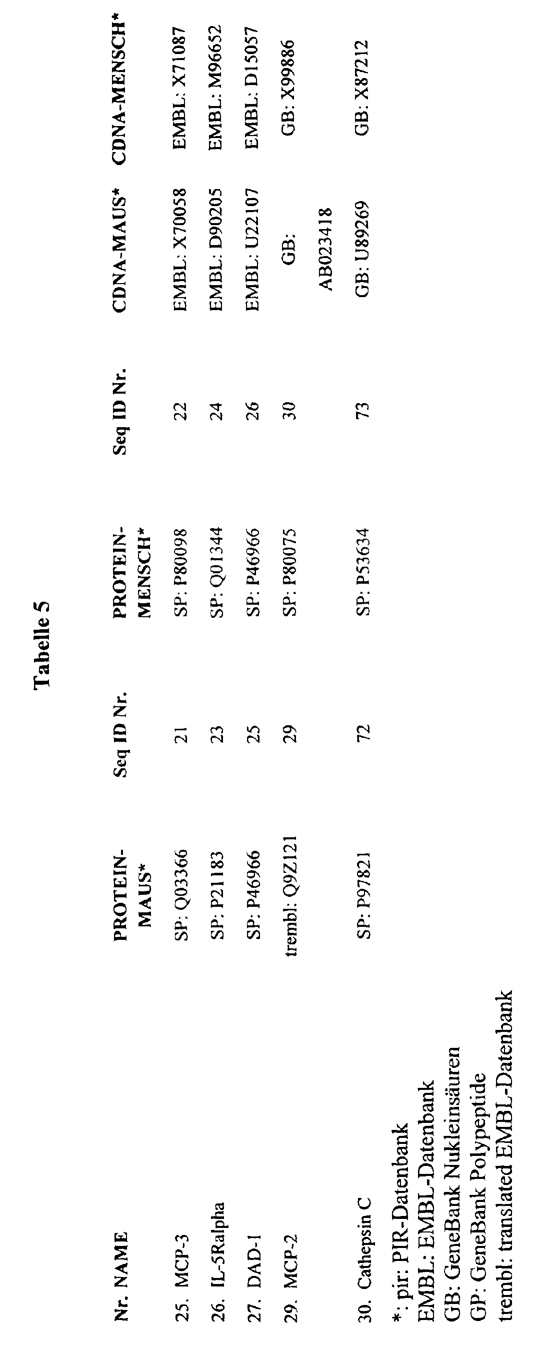

**Tabelle 6**

| **Verändert exprimierte Gene** | **Intakte Haut** | **Wunde 1 h** | **Wunde 7 h** | **Wunde 15 h** | **Wunde 24 h** | **Wunde 3 d** | **Wunde 5 d** | **Wunde 7 d** | **Wunde 14 d** |
|---|---|---|---|---|---|---|---|---|---|
| B-Raf | 1,00 | 1,30 | 0,79 | 0,89 | 0,87 | 0,94 | 1,21 | 0,72 | 0,93 |
| KIAA1247 | 1,00 | 0,67 | 0,53 | 0,30 | 0,31 | 0,44 | 0,67 | 1,11 | 0,97 |
| Cystatin C | 1,00 | 0,78 | 0,69 | 0,69 | 0,60 | 0,68 | 0,88 | 1,05 | 0,89 |
| SW1136 | 1,00 | 0,48 | 0,36 | 0,15 | 0,15 | 0,23 | 0,31 | 0,33 | 0,20 |
| SW1295 | 1,00 | 0,43 | 0,33 | 0,28 | 0,22 | 0,27 | 0,35 | 0,66 | 0,44 |
| BAF57 | 1,00 | 0,76 | 0,57 | 0,54 | 0,42 | 0,67 | 0,93 | 1,19 | 0,94 |
| EAT/MCL-1 | 1,00 | 0,58 | 0,81 | 1,08 | 0,75 | 0,85 | 1,16 | 0,66 | 0,66 |
| Phospholipase Inhibitor | 1,00 | 14,47 | 1,00 | 0,23 | 0,88 | 1,34 | 1,49 | 3,67 | 21,93 |
| TSC-22 | 1,00 | 0,68 | 0,66 | 0,43 | 0,40 | 0,43 | 0,70 | 0,88 | 0,95 |
| Split hand/foot deleted 1 | 1,00 | 0,57 | 0,40 | 0,42 | 0,43 | 0,53 | 0,67 | 0,62 | 0,66 |
| Nicotinamid N-Methyl-transferase | 1,00 | 0,36 | 0,60 | 0,46 | 0,43 | 0,71 | 0,43 | 0,80 | 0,62 |
| Golgi 4-Transmembrane spanning transporter | 1,00 | 0,67 | 0,69 | 0,34 | 0,26 | 0,33 | 0,46 | 0,50 | 0,48 |
| UBC9 | 1,00 | 0,72 | 0,68 | 0,49 | 0,51 | 0,81 | 0,87 | 0,95 | 1,18 |
| Cathepsin C | 1,00 | 0,83 | 0,29 | 0,26 | 1,54 | 0,81 | 1,29 | 1,07 | 3,88 |
| tsg101 | 1,00 | 0,71 | 0,51 | 0,45 | 0,33 | 0,62 | 0,53 | 0,66 | 0,83 |
| DAD-1 | 1,00 | 0,84 | 0,76 | 0,74 | 0,84 | 1,07 | 1,10 | 1,36 | 1,18 |
| HMG-14 | 1,00 | 0,55 | 0,31 | 0,29 | 0,33 | 0,46 | 0,49 | 0,45 | 0,62 |
| TAK1 | 1,00 | 0,49 | 0,62 | 0,28 | 0,39 | 0,54 | 0,64 | 0,71 | 0,65 |
| Fer | 1,00 | 0,17 | 0,15 | 0,09 | 0,13 | 0,17 | 0,22 | 0,25 | 0,08 |
| EPS8 | 1,00 | 0,60 | 1,10 | 1,00 | 0,70 | 0,70 | 1,00 | 0,70 | 0,90 |
| CCR-1 | 1,00 | 1,40 | 5,90 | 19,00 | 17,00 | 16,00 | 13,00 | 5,50 | 3,70 |
| MASPIN | 1,00 | 0,60 | 0,50 | 0,30 | 0,30 | 0,30 | 0,40 | 0,50 | 0,80 |
| TTF-1 | 1,00 | 0,80 | 0,60 | 0,50 | 0,40 | 0,50 | 0,60 | 0,60 | 0,90 |

**Tabelle 7**

| **Rel. cDNA Expression in humanen Biopsien bezüglich Cyclophilin** | **Zeitpunkt nach Verwundung** | | | | |
|---|---|---|---|---|---|
| | **Intakte Haut** | **Wunde 1 h** | **Wunde 24 h** | **Wunde 5 d** | **Wunde 14 d** |
| Eps8 | 1,00 | 1,41 | 0,74 | 1,3 | 1,29 |
| KIAA1247 | 1,00 | 0,92 | 1,22 | 1,02 | 1,58 |
| Phospholipase Inhibitor | 1,00 | 1,24 | 0,64 | 0,57 | 0,85 |
| Cystatin C | 1,00 | 0,86 | 1,27 | 1,79 | 2,33 |
| TSC-22 | 1,00 | 1,34 | 0,91 | 1,29 | 1,98 |
| Golgi 4-Transmembrane spanning transporter | 1,00 | 0,69 | 0,63 | 0,59 | 0,72 |
| Cathepsin C | 1,00 | 0,86 | 2,96 | 4,09 | 2,67 |
| HMG-14 | 1,00 | 1,87 | 0,56 | 0,98 | 0,87 |
| Nicotinamid N-Methyltransferase | 1,00 | 1,74 | 6,20 | 9,74 | 5,15 |
| UBC9 | 1,00 | 0,98 | 1,39 | 1,43 | 1,45 |
| CCR-1 | 1,00 | 1,28 | 10,56 | 7,78 | 5,66 |
| tsg101 | 1,00 | 1,09 | 1,07 | 1,24 | 1,27 |
| MASPIN | 1,00 | 1,62 | 0,38 | 1,11 | 0,61 |
| TTF-1 | 1,00 | 0,55 | 0,95 | 0,74 | 1,45 |
| B-Raf | 1,00 | 1,82 | 0,89 | 1,45 | 1,30 |
| DAD-1 | 1,00 | 0,66 | 1,47 | 1,61 | 1,46 |
| Fer | 1,00 | 8,07 | 0,83 | 3,35 | 5,06 |
| Split hand/foot deleted 1 | 1,00 | 0,98 | 0,79 | 1,31 | 1,20 |
| Gamma Sarcoglycan | 1,00 | 1,13 | 0,29 | 0,67 | 0,89 |
| TAK1 | 1,00 | 1,08 | 0,89 | 1,37 | 1,56 |

**Tabelle 8**

| **Erfindungsgemäß verwendbares Gen** | **Intakte Haut Ul-kus-Patienten** | **Wundrand Ulkus-Patienten** | **Wundgrund Ul-kus-Patienten** |
|---|---|---|---|
| Eps8 | 1,00 | 0,88 | 0,89 |
| KIAA1247 | 1,00 | 0,38 | 1,1 |
| Phospholipase Inhibitor (Variante SEQ ID Nr. 45) | 1,00 | 0,54 | 0,48 |
| Phospholipase Inhibitor (Variante SEQ ID Nr. 81) | 1,00 | 1,54 | 1,18 |
| Cystatin C | 1,00 | 0,66 | 0,52 |
| TSC-22 | 1,00 | 0,64 | 0,70 |
| Golgi 4-Transmembrane spanning transporter | 1,00 | 1,15 | 0,85 |
| Cathepsin C | 1,00 | 0,6 | 1,07 |
| HMG-14 | 1,00 | 1,64 | 0,87 |
| Nicotinamid N-Methyltransferase | 1,00 | 2,28 | 1,49 |
| UBC9 | 1,00 | 0,72 | 0,74 |
| CCR-1 | 1,00 | 6,00 | 16,80 |
| tsg101 | 1,00 | 0,54 | 0,50 |
| MASPIN | 1,00 | 0,29 | 0,04 |
| TTF-1 | 1,00 | 0,40 | 0,60 |
| B-Raf | 1,00 | 0,76 | 0,31 |
| DAD-1 | 1,00 | 0,97 | 0,65 |
| Fer | 1,00 | 0,80 | 0,26 |
| Split hand/foot deleted 1 | 1,00 | 0,19 | 0,16 |
| Gamma Sarcoglycan | 1,00 | 0,10 | 0,05 |
| TAK1 | 1,00 | 0,27 | 0,41 |

**Tabelle 9**

| **Erfindungsgemäß verwendbares Gen** | **Normierte Rel. Abundanzen** | | |
|---|---|---|---|
| | **Intakte gesunde Haut** | **Läsionale Haut Psoriasis Patienten** | **Nicht-läsionale Haut Psoriasis Patienten** |
| KIAA1247 | 9,69E-02 | 6,23E-02 | 1,39E-01 |
| Eps8 | 1,10E-01 | 5,36E-02 | 1,41E-01 |
| MASPIN | 7,55E-02 | 8,60E-02 | 1,26E-01 |
| | | 1,26E-01 | |

## Patentansprüche

1. Verwendung mindestens eines Polypeptids gemäß einer der SEQ ID Nr. 55 bis SEQ ID Nr. 58 oder einer funktionellen Variante davon und/oder dieses kodierende Nukleinsäure oder eine Variante davon, und/oder oder einer ein genanntes Polypeptid oder eine funktionelle Variante davon oder einer genannte Nukleinsäure oder eine Variante davon exprimierenden Zelle zur Herstellung eines Arzneimittels und/oder Diagnostikums zur Diagnose, Prävention und/oder Behandlung von Erkrankungen, bei der Wundheilung und/oder deren krankhaften Störungen, sowie ihre Verwendung zur Identifizierung von pharmakologisch aktiven Substanzen, gegebenenfalls zusammen oder kombiniert mit geeigneten Zusatz- und/oder Hilfsstoffen.

2. Verwendung mindestens eines Polypeptids gemäß einer der SEQ ID Nr. 1 bis SEQ ID Nr. 20 und SEQ ID Nr. 31 bis SEQ ID Nr. 48 und SEQ ID Nr. 63 bis SEQ ID Nr. 70 und SEQ ID Nr. 80 bis SEQ ID Nr. 82 oder einer funktionellen Variante davon und/oder dieses kodierende Nukleinsäure oder eine Variante davon, und/oder einer ein genanntes Polypeptid oder eine funktionelle Variante davon oder einer genannte Nukleinsäure oder eine Variante davon exprimierenden Zelle zur Herstellung eines Arzneimittels und/oder Diagnostikums zur Diagnose, Prävention und/oder Behandlung von Erkrankungen von Hautzellen, bei der Wundheilung und/oder deren krankhaften Störungen, sowie ihre Verwendung zur Identifizierung von pharmakologisch aktiven Substanzen, gegebenenfalls zusammen oder kombiniert mit geeigneten Zusatz- und/oder Hilfsstoffen.

3. Verwendung eines Polypeptids gemäß einer der SEQ ID Nr. 21 bis SEQ ID Nr. 26 und SEQ ID Nr. 29 bis SEQ ID Nr. 30 und SEQ ID Nr. 71 bis SEQ ID Nr. 73 oder einer funktionellen Variante davon und/oder dieses kodierende Nukleinsäure oder eine Variante davon, und/oder einer ein genanntes Polypeptid oder eine funktionelle Variante davon oder einer genannte Nukleinsäure oder eine Variante davon exprimierende Zelle zur Herstellung eines Arzneimittels und/oder Diagnostikums zur Diagnose, Prävention und/oder Behandlung bei der Wundheilung und/oder deren krankhaften Störungen, sowie ihre Verwendung zur Identifizierung von pharmakologisch aktiven Substanzen, gegebenenfalls zusammen oder kombiniert mit geeigneten Zusatz- und/oder Hilfsstoffen.

4. Verwendung nach mindestens einem der Ansprüche 1 bis 3, **dadurch gekennzeichnet, daß** das Polypeptid in Form eines Fusionsproteins eingesetzt wird.

5. Verwendung nach mindestens einem der Ansprüche 1 bis 3, **dadurch gekennzeichnet, daß** die Nukleinsäure in Form eines Expressionsvektors, eines knock-out Genkonstruktes und/oder eines gentherapeutisch wirksamen Vektors eingesetzt wird.

6. Verwendung nach mindestens einem der Ansprüche 1 bis 5, **dadurch gekennzeichnet, daß** es sich bei den genannten Zellen um autologe oder heterologe Zellen handelt.

7. Verwendung nach Anspruch 6, **dadurch gekennzeichnet, daß** es sich bei den Zellen um eine Hautzelle handelt.

8. Verwendung eines gegen ein Polypeptid nach mindestens einem der Ansprüche 1 bis 4 gerichteten Antikörpers oder Antikörperfragments, zur Herstellung eines Diagnostikums und/oder Arzneimittels zur Analyse, Diagnose, Behandlung und/oder Prävention von Erkrankungen von Hautzellen, von Wundheilung und/oder deren krankhaften Störungen, sowie seine Verwendung zur Identifizierung von pharmakologisch aktiven Substanzen, gegebenenfalls zusammen oder kombiniert mit geeigneten Zusatz- und/oder Hilfsstoffen.

9. Verwendung nach mindestens einem der Ansprüche 1 bis 3, **dadurch gekennzeichnet, daß** zur Identifizierung von pharmakologisch aktiven Substanzen mindestens ein Polypeptid nach mindestens einem der Ansprüche 1 bis 4, und/oder mindestens ein gegen ein genanntes Polypeptid gerichteter Antikörper oder ein Antikörperfragment an eine Festphase gebunden wird.

10. Verwendung mindestens eines Polypeptids nach mindestens einem der Ansprüche 1 bis 4 und/oder dieses kodierende Nukleinsäure, und/oder mindestens eines gegen genanntes Polypeptid gerichteten Antikörpers, zur Herstellung eines auf einem Trägermaterial fixierten Arrays, zur Analyse in Zusammenhang mit Erkrankungen von Hautzellen und/oder von Wundheilung und/oder deren krankhaften Störungen.
